# EUROPEAN PATENT APPLICATION

(11) **EP 4 477 228 A1**
(43) Date of publication of application: **18.12.2024**
(21) Application number: 23753226.2
(22) Date of filing: 10.02.2023
(51) Int. Cl.: A61K 38/00, A61K 38/20, A61K 39/395, A61K 45/06, A61P 35/00, A61K 39/00

(54) **PHARMACEUTICAL COMPOSITION FOR CANCER TREATMENT COMPRISING FUSION PROTEIN INCLUDING IL-12 AND ANTI-FAP ANTIBODY AND ANTICANCER AGENT**

(30) Priority: 11.02.2022 KR 20220018247
(71) Applicant: Kanaph Therapeutics Inc., Seoul 04348 (KR)
(72) Inventor: KIM, Dongsu, Seoul 04348 (KR); KIM, Donggeon, Seoul 04348 (KR); LEE, Hyunji, Seoul 04348 (KR); YOON, Yujin, Seoul 04348 (KR); LEE, Youngin, Seoul 04348 (KR); LEE, Dahea, Seoul 04348 (KR); CHANG, Jihoon, Seoul 04348 (KR); LEE, Byoung Chul, Seoul 04348 (KR)
(74) Representative: HGF
(86) International application number: PCT/KR2023/002030
(87) International publication number: WO 2023/153880

(57) **Abstract**

The present invention provides a combined therapeutic agent of: a fusion protein comprising IL-12 or a variant thereof and an antigen-binding site that specifically binds to FAP; and an anticancer agent. The bispecific antibody exhibits an anticancer effect by IL-12 and in particular, when the anti-FAP antibody is realized in one antibody, cancer can be efficiently treated by targeting FAP expressed highly specifically in a tumor and specifically localizing IL-12 to a tumor site. When the fusion protein is administered in combination with a known anticancer agent, there is a synergistic effect on anticancer treatment.

## Description

### Technical Field

The present invention relates to an anticancer pharmaceutical composition comprising a novel fusion protein comprising IL-12 or a variant thereof; and anti-FAP antibody, and an anticancer agent.

### Background Art

Interleukin-12 (IL-12) is a representative inflammatory cytokine and plays an essential role in effectively inducing a cellular immune response. IL-12 is a heterodimeric protein comprising 40 kDa (p40) and 35 kDa (p35) subunits linked by a disulfide bond and is produced by activated macrophages, monocytes, dendritic cells and activated B lymphocytes. IL-12 may enhance T-helper 1 cell immunity, increase cytotoxicity of cellular T-lymphocytes, and inhibit angiogenesis. In general, IL-12 activates IFN-γ production in T cells and NK cells.

Local expression of IL-12 renders tumor cells sensitive to T-cell mediated cytotoxicity, resulting in tumor growth inhibition and the establishment of humoral immunity. However, a disadvantage in the clinical application of IL-12 is that upon administration, cytokine-related toxicity may occur systemically. These clinical results show the limitations of IL-12 as a monotherapeutic agent for the treatment of cancer.

On the other hand, fibroblast activation protein alpha (FAP) is a gelatinase expressed on activated fibroblasts. FAP is known to be expressed over 90% in cancer-related fibroblasts of various human carcinomas, including prostate cancer and pancreatic cancer. Accordingly, recently, interest in developing an immunotherapy targeting FAP-expressing cells is rapidly increasing (Fang J. et.al., Mol. Ther. Oncolytics., 3:16007, 2016).

### Detailed Description of Invention

### Technical Problem

Accordingly, the present inventors have studied to develop a pharmaceutical composition having an effective anticancer effect. As a result, the present inventors have found the synergistic anticancer effect when a fusion protein comprising IL-12 and an anti-FAP antibody; and an anticancer agent are administered together, thereby completing the present invention.

### Solution to Problem

In one aspect of the present invention, there is provided a pharmaceutical composition for treating cancer comprising a fusion protein comprising a first monomer comprising IL-12 or a variant thereof; and a second monomer comprising an antigen binding site that specifically binds to FAP(Fibroblast activation protein alpha); and an anticancer agent.

In another aspect of the present invention, there is provided a kit for preventing or treating cancer comprising a fusion protein comprising a first monomer comprising IL-12 or a variant thereof; and a second monomer comprising an antigen binding site that specifically binds to FAP; and an anticancer agent as an active ingredient.

### Effects of Invention

A fusion protein comprising IL-12 and an antigen binding site that specifically binds to FAP may exhibit an anticancer effect by IL-12. In addition, it was found that when the anti-FAP antibody is implemented in one antibody, IL-12 is specifically accumulated in a tumor with high FAP expression, thereby reducing systemic toxicity and exhibiting a tumor-specific anticancer effect. That is, cancer may be efficiently treated by specifically targeting FAP expressed highly in a tumor, and specifically localizing IL-12 to the tumor site. In particular, when the fusion protein and an anticancer agent are administered together, there is a synergistic anticancer effect.

### Brief Description of Drawings

FIG. 1 illustrates a result obtained by identifying T1.01, T1.02, T1.03, T1.04, T1.05, T1.06 and T1.07 according to one embodiment with SDS-PAGE.
FIG. 2 illustrates a result obtained by identifying T1.08, T1.09, T1.10, T1.11, T1.12, T1.13, T1.14 and T1.15 according to one embodiment with SDS-PAGE.
FIG. 3 illustrates a result obtained by identifying T1.16, T1.17, T1.18, T1.19 and T1.21 according to one embodiment with SDS-PAGE.
FIG. 4 illustrates a result obtained by identifying T1.01m, T1.02m, T1.03m, T1.04m, T1.05m, T1.06m and T1.07m according to one embodiment with SDS-PAGE.
FIG. 5 illustrates a result obtained by identifying T1.08m, T1.09m, T1.10m, T1.11m, T1.12m, T1.13m and T1.15m according to one embodiment with SDS-PAGE.
FIG. 6 illustrates a result obtained by identifying T1.16m, T1.17m, T1.18m, T1.19m, T1.20m, T1.21m and T1.22m according to one embodiment with SDS-PAGE.
FIG. 7 illustrates a graph showing the binding affinities of T1.01 and T1.02 for recombinant human FAP (rhFAP) through surface plasmon resonance analysis method.
FIG. 8 illustrates a graph showing the binding affinity of T1.03 for recombinant human FAP through surface plasmon resonance analysis method.
FIG. 9 illustrates a drawing showing the binding degrees of T1.04 and T1.05 for recombinant human FAP through surface plasmon resonance analysis method.
FIG. 10 illustrates a drawing showing the binding degrees of T1.06 and T1.07 for recombinant human FAP through surface plasmon resonance analysis method.
FIG. 11 illustrates a drawing showing the binding degrees of T1.08 and T1.09 for recombinant human FAP through surface plasmon resonance analysis method.
FIG. 12 illustrates a drawing showing the binding degress of T1.10 and T1.11 for recombinant human FAP through surface plasmon resonance analysis method.
FIG. 13 illustrates a graph showing the binding affinities of T1.01m and T1.02m for recombinant mouse FAP through surface plasmon resonance analysis method.
FIG. 14 illustrates a graph showing the binding affinity of T1.03m for recombinant mouse FAP through surface plasmon resonance analysis method.
FIG. 15 illustrates a graph showing the binding affinities of T1.12, T1.01, T1.02 and T1.03 for HEK293-hFAP cells and HEK293 cells through flow cytometry.
FIG. 16 illustrates a graph showing the binding affinities of T1.12m, T1.01m, T1.02m and T1.03m for B16F10-mFAP cells and B16F10 cells through flow cytometry.
FIG. 17 illustrates a graph showing the binding affinities of T1.01, T1.02, T1.03 and T1.12 for IL-12 receptor beta 1 through enzyme-linked immunosorbent assay.
FIG. 18 illustrates a graph showing a result obtained by identifying the T1.01m, T1.02m, T1.03m and T1.12m and IL-12 activity through absorbance in HEKblue reporter cells.
FIG. 19 illustrates a graph showing the T1.01, T1.02, T1.03 and T1.12 and IL-12 activity through the signaling ability of human T cells.
FIG. 20 illustrates a graph showing the IL-12 activity of T1.01, T1.02 and T1.03 in the presence of low molecular weight heparin (LMWH) through the signaling ability of human T cells.
FIG. 21 illustrates a graph showing a result obtained by measuring the ability of human T cells to secrete cytokine IFN-γ by T1.12, T1.01, T1.02 and T1.03.
FIG. 22 illustrates a graph showing a result obtained by measuring the ability of human NK cells to secrete cytokine IFN-γ by T1.12, T1.01, T1.02 and T1.03.
FIG. 23 illustrates a graph showing the tumor growth inhibitory ability by T1.01m and T1.02m by measuring the change in tumor size of a mouse tumor model co-injected with mouse FAP-overexpressing fibroblast NIH-3T3 and colorectal cancer cell line CT26.
FIG. 24 illustrates a graph showing a result obtained by measuring the tumor size for each subject in order to identify the tumor growth inhibitory ability by T1.01m and T1.02m in a mouse tumor model co-injected with mouse FAP-overexpressing fibroblast NIH-3T3 and colorectal cancer cell line CT26.
FIG. 25 illustrates a graph showing the tumor growth inhibitory ability by administration of T1.01m and T1.02m for each concentration by measuring the change in tumor size for each subject in a mouse tumor model implanted mouse FAP-overexpressing colorectal cancer cell line CT26.
FIG. 26 illustrates a graph showing the change in CT26 tumor size after construction of a tumor rechallenge model by CT26 cells and 4T1 cells transplantation into mice in which complete response was achieved in a mouse tumor model co-injected with mouse FAP-overexpressing fibroblast NIH-3T3 and colorectal cancer cell line CT26.
FIG. 27 illustrates a graph showing the change in 4T1 tumor size after construction of a tumor rechallenge model by CT26 cells and 4T1 cells transplantation into mice in which complete response was achieved in a mouse tumor model co-injected with mouse FAP-overexpressing fibroblast NIH-3T3 and colorectal cancer cell line CT26.
FIG. 28 illustrates a graph showing the tumor growth inhibitory ability by administration of T1.01m, T1.02m and T1.03m at different concentrations for each subject by measuring the tumor size for each subject in a mouse model implanted with mouse FAP-overexpressing CT26 cells.
FIG. 29 illustrates a graph showing the tumor growth inhibitory ability for each subject by administration of T1.03m for each concentration by measuring the change in tumor size for each subject in a mouse model implanted with mouse FAP-overexpressing CT26 cells.
FIG. 30 illustrates a graph showing the tumor growth inhibitory ability by administration of T1.03m for each concentration by measuring the change in tumor size for each subject in a mouse model implanted with mouse FAP-overexpressing B16F10 cells.
FIG. 31 illustrates a graph showing the tumor growth inhibitory ability by administration of T1.03m for each concentration by measuring the change in tumor size for each subject in a mouse model co-injected with mouse FAP-overexpressing fibroblast NIH-3T3 and lung cancer cell line LLC1.
FIG. 32 illustrates a graph showing a result obtained by measuring the tumor size at the time point 10 days after treatment of T1.16m, T1.17m, T1.03m, or T1.16m and T1.17m simultaneous administration in a mouse model co-injected with mouse FAP-overexpressing fibroblast NIH-3T3 and lung cancer cell line LLC1.
FIG. 33a illustrates a graph showing a result obtained by measuring the tumor size at the time point 9 days after treatment of T1.03m and aPD-L1, respectively, or simultaneous administration in a mouse model co-injected with mouse FAP-overexpressing fibroblast NIH-3T3 and lung cancer cell line LLC1.
FIG. 33b illustrates a graph showing a result obtained by measuring the tumor size at the time point 9 days after treatment of T1.03m and aCTLA-4, respectively, or simultaneous administration in a mouse model co-injected with mouse FAP-overexpressing fibroblast NIH-3T3 and lung cancer cell line LLC1.
FIG. 33c illustrates a graph showing a result obtained by measuring the tumor size at the time point 9 days after treatment of T1.03m and aPD-1, respectively, or simultaneous administration in a mouse model co-injected with mouse FAP-overexpressing fibroblast NIH-3T3 and lung cancer cell line LLC1.
FIG. 34a illustrates a graph showing a result obtained by measuring the tumor size at the time point 16 days after treatment of T1.03m and aPD-L1, respectively, or simultaneous administration in a mouse model co-injected with mouse FAP-overexpressing fibroblast NIH-3T3 and colon cancer cell line MC38.
FIG. 34b illustrates a graph showing a result over time obtained by measuring the tumor size up to 16 days after treatment of T1.03m and aPD-L1, respectively, or simultaneous administration in a mouse model co-injected with mouse FAP-overexpressing fibroblast NIH-3T3 and colon cancer cell line MC38.
FIG. 35a illustrates a graph showing a result obtained by measuring the tumor size at the time point 11 days after treatment of T1.03m and aPD-L1, respectively, or simultaneous administration in a mouse model co-injected with mouse FAP-overexpressing fibroblast NIH-3T3 and colon cancer cell line MC38.
FIG. 35b illustrates a graph showing a result over time obtained by measuring the tumor size up to 11 days after treatment of T1.03m and aPD-L1, respectively, or simultaneous administration in a mouse model co-injected with mouse FAP-overexpressing fibroblast NIH-3T3 and colon cancer cell line MC38.
FIG. 36a illustrates a graph showing a result obtained by measuring the tumor size at the time point 10 days after treatment of T1.03m and aTIGIT, respectively, or simultaneous administration in a mouse model co-injected with mouse FAP-overexpressing fibroblast NIH-3T3 and colon cancer cell line MC38.
FIG. 36b illustrates a graph showing a result over time obtained by measuring the tumor size up to 10 days after treatment of T1.03m and aTIGIT, respectively, or simultaneous administration in a mouse model co-injected with mouse FAP-overexpressing fibroblast NIH-3T3 and colon cancer cell line MC38.
FIG. 37a illustrates a graph showing a result obtained by measuring the tumor size at the time point 10 days after treatment of T1.03m and aLAG-3, respectively, or simultaneous administration in a mouse model co-injected with mouse FAP-overexpressing fibroblast NIH-3T3 and colon cancer cell line MC38.
FIG. 37b illustrates a graph showing a result over time obtained by measuring the tumor size up to 10 days after treatment of T1.03m and aLAG-3, respectively, or simultaneous administration in a mouse model co-injected with mouse FAP-overexpressing fibroblast NIH-3T3 and colon cancer cell line MC38.
FIG. 38a illustrates a graph showing a result obtained by measuring the tumor size at the time point 10 days after treatment of T1.03m and aTIM-3, respectively, or simultaneous administration in a mouse model co-injected with mouse FAP-overexpressing fibroblast NIH-3T3 and colon cancer cell line MC38.
FIG. 38b illustrates a graph showing a result over time obtained by measuring the tumor size up to 10 days after treatment of T1.03m and aTIM-3, respectively, or simultaneous administration in a mouse model co-injected with mouse FAP-overexpressing fibroblast NIH-3T3 and colon cancer cell line MC38.
FIG. 39a illustrates a graph showing a result obtained by measuring the tumor size at the time point 11 days after treatment of T1.03m and Cyclophosphamide, respectively, or simultaneous administration in a mouse model co-injected with mouse FAP-overexpressing fibroblast NIH-3T3 and colon cancer cell line MC38.
FIG. 39b illustrates a graph showing a result over time obtained by measuring the tumor size up to 11 days after treatment of T1.03m and Cyclophosphamide, respectively, or simultaneous administration in a mouse model co-injected with mouse FAP-overexpressing fibroblast NIH-3T3 and colon cancer cell line MC38.
FIG. 40a illustrates a graph showing a result obtained by measuring the tumor size at the time point 11 days after treatment of T1.03m and Fluorouracil, respectively, or simultaneous administration in a mouse model co-injected with mouse FAP-overexpressing fibroblast NIH-3T3 and colon cancer cell line MC38.
FIG. 40b illustrates a graph showing a result over time obtained by measuring the tumor size up to 11 days after treatment of T1.03m and Fluorouracil, respectively, or simultaneous administration in a mouse model co-injected with mouse FAP-overexpressing fibroblast NIH-3T3 and colon cancer cell line MC38.
FIG. 41a illustrates a graph showing a result obtained by measuring the tumor size at the time point 11 days after treatment of T1.03m and Paclitaxel, respectively, or simultaneous administration in a mouse model co-injected with mouse FAP-overexpressing fibroblast NIH-3T3 and colon cancer cell line MC38.
FIG. 41b illustrates a graph showing a result over time obtained by measuring the tumor size up to 11 days after treatment of T1.03m and Paclitaxel, respectively, or simultaneous administration in a mouse model co-injected with mouse FAP-overexpressing fibroblast NIH-3T3 and colon cancer cell line MC38.
FIG. 42a illustrates a graph showing a result obtained by measuring the tumor size at the time point 11 days after treatment of T1.03m and Camptosar, respectively, or simultaneous administration in a mouse model co-injected with mouse FAP-overexpressing fibroblast NIH-3T3 and colon cancer cell line MC38.
FIG. 42b illustrates a graph showing a result over time obtained by measuring the tumor size up to 11 days after treatment of T1.03m and Camptosar, respectively, or simultaneous administration in a mouse model co-injected with mouse FAP-overexpressing fibroblast NIH-3T3 and colon cancer cell line MC38.

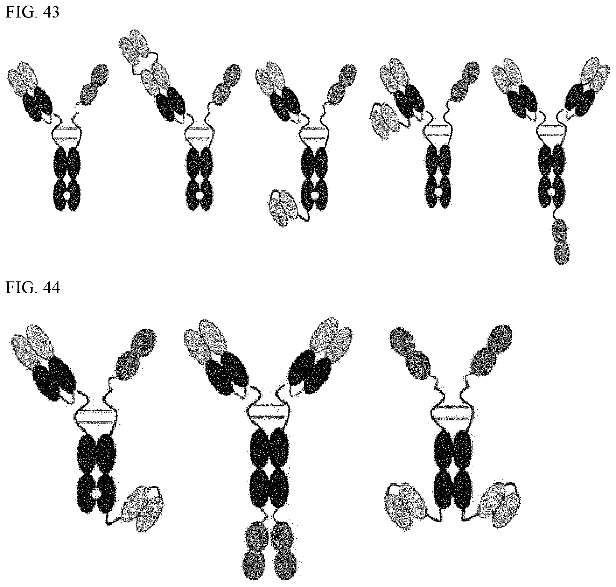

FIG. 43 illustrates a schematic diagram of an anti-FAP/IL-12 fusion protein (first from the left), an anti-FAP/IL-12 fusion protein having a dual variable domain (second), an anti-FAP/IL-12 dual antibody to which an FAP-specific single chain variable fragment (scFv) is bound (third and fourth), and an anti-FAP/IL-12 fusion protein in which IL-12 is bound to the C-terminus of Fc (fifth).
FIG. 44 illustrates a schematic diagram of an FAP-specific Fab/FAP-specific single chain variable fragment (scFv) and an IL-12 fusion protein (left), a fusion protein dimer comprising an FAP-specific Fab and IL-12 (middle), and an FAP-specific single chain variable fragment (scFv) and an IL-12 fusion protein (right).

### Mode for Carrying out the Invention

### Combination treatment comprising fusion protein comprising IL-12 and anti-FAP antibody and an anticancer agent

In one aspect of the present invention, there is provided a pharmaceutical composition for preventing or treating cancer comprising a first monomer a fusion protein comprising IL-12 or a variant thereof; and a second monomer comprising an antigen binding site that specifically binds to FAP (fibroblast activation protein alpha); and an anticancer agent.

The fusion protein exhibits an anticancer effect by IL-12, and not only exhibits a tumor-specific anticancer effect by accumulating IL-2 specifically in tumors with high FAP expression, but also reduces systemic toxicity, it can be used in combination with various anticancer treatments that have been used conventionally. Specifically, conventional treatments that can be used in combination may be selected from the group consisting of chemical anticancer agent for chemotherapy, oncolytic virus, immune checkpoint inhibitor, and combinations thereof.

### Anticancer agent

As used herein, the term "chemical anticancer agent" also referred to as an antineoplastic agent or cytotoxic agent. It is a general term for a drug that exhibit anticancer activity by directly affecting DNA and blocking replication, transcription, translation process of DNA, or interfering with the synthesis of nucleic acid precursors in metabolic pathways and inhibiting cell division. The antineoplastic agent not only acts on turner cells, but also on normal cells and exhibits cytotoxicity. A chemical anticancer agent may be used for maintenance therapy. In addition, as used herein, the term "maintenance therapy" refers to treating cancer with drugs after initial anti-cancer treatment, and refers to a treatment method performed to prevent or delay the recurrence of cancer.

The chemical anticancer agent may be any one selected from the group consisting of alkylating agent, microtubule inhibitor, anti metabolite, and topoisomerase inhibitor.

In one embodiment, the alkylating agent may be Cyclophosphamide, or Cisplatin. The microtubule inhibitor may be Paclitaxel. The anti-metabolite may be Fluorouracil or Gemcitabine. The topoisomerase inhibitor may be Camptosar or Adriamycin.

As used herein, the term "oncolytic virus therapy" is a treatment that kills cancer by inserting a specific gene that targets cancer cells into a proliferative and infectious virus. The oncolytic virus therapy may be Talimogene Laherparepvec (T-VEC).

As used herein, the term "immune checkpoint inhibitor" refers to a substance that inhibits the activity of an immune checkpoint protein that inhibits the differentiation, proliferation, activity of immune cells, and it is known to eliminate cancer cells by preventing cancer cells from excercising the function of evading the immune system.

In one embodiment, the immune checkpoint inhibitor may be any one selected from the group consisting of anti-CTLA-4 antibody, anti-PD-1 antibody, anti-PD-L1 antibody, anti-TIM3 antibody, anti-TIGIT antibody and anti-LAG3 antibody.

In one embodiment, the immune checkpoint inhibitor may be any one selected from the group consisting of Ipilimumab, Pembrolizumab, Nivolumab, Cemiplimab, Atezolizumab, Avelumab, and Durvalumab.

The fusion protein may be used in combination with an anticancer vaccine, and the like. In addition, anticancer agents are not only the above-mentioned anticancer agents, but also can be used with anticancer vaccines, and the like.

In addition, the anticancer agent may comprise one or more anticancer agents. Specifically, the fusion protein can be used together with two anticancer agents. For example, a chemical anti-cancer agent and an anti-cancer viruse; an anti-cancer viruse and an immune checkpoint inhibitor; and a chemical anticancer agent and an immune checkpoint inhibitor.

In addition, the fusion protein can be used together with three anticancer agents. An anticancer agent different from the above two anticancer agents may be further comprised and used. In one embodiment, the anticancer agent may be a chemical anticancer agent, an anticancer virus and an immune checkpoint inhibitor.

### Anticancer maintenance use of fusion protein

Another aspect of the present invention provides a composition for maintaining anticancer comprising a fusion protein comprising a first monomer comprising IL-12 or a variant thereof; and a second monomer comprising an antigen binding site that specifically binds to FAP, as an active ingredient.

The fusion protein is as described below.

As described above, "maintaining anticancer" refers to treating cancer after initial anticancer treatment. In particular, it is a treatment method that improves the effects of cancer treatment by preventing or delaying the recurrence of cancer.

At this time, at least one additional anticancer agent may be comprised for maintenance therapy. At this time, the anticancer agent is as described above.

### Kit comprising fusion protein and anticancer agent

Another aspect of the present invention provides a kit for preventing or treating cancer comprising a fusion protein comprising a first monomer comprising IL-12 or a variant thereof; and a second monomer comprising an antigen binding site that specifically binds to FAP; and an anticancer agent as an active ingredient.

### Fusion protein comprising IL-12 and FAP antibodody

The fusion protein comprising IL-12 or a variant thereof and an antigen binding site that specifically binds to FAP may comprise an immunoglobulin Fc region. At this time, IL-12 or the variant thereof may be a variant having low heparin binding ability.

In one embodiment, the fusion protein may be a fusion protein comprising a first monomer comprising IL-12 or a variant thereof; and a second monomer comprising an antigen binding site that specifically binds to FAP.

In another embodiment, the fusion protein may be a dimer of a fusion protein comprising IL-12 or a variant thereof and an antigen binding site that specifically binds to FAP.

### IL-12 or variant thereof

As used herein, the term "IL-12" is a heterodimeric cytokine composed of the p35 and p40 subunits encoded by two separate genes, IL-12A and IL-12B, respectively. IL-12 is produced by antigen-presenting cells such as macrophages and binds to receptors on the cell surface of activated T cells and NK cells. IL-12 promotes the proliferation of T cells and NK cells, and enhances the cytotoxic effects of T cells, NK cells and macrophages. In addition, IL-12 induces the productions of IFN-γ, TNF-α and GM-CSF, and acts to induce activation of Th1 cells. On the other hand, IL-12 binds to the IL-12 receptor, a heterodimeric receptor formed by IL-12Rβ1 and IL-12Rβ2.

As used herein, the term "variant" of IL-12 includes an amino acid sequence having a function similar to or identical to that of a wild-type IL-12 protein. Specifically, the amino acid sequence of the p35 and p40 subunits constituting IL-12 may have a substitution, an insertion or a deletion as compared to the wild-type.

The IL-12 or the variant thereof may include IL-12A (p35) or a variant thereof, and IL-12B (p40) or a variant thereof.

In addition, the IL-12 or the variant thereof may include a structure in which IL-12A or a variant thereof; and IL-12B or a variant thereof are bound by a peptide linker.

In one embodiment, the IL-12 or the variant thereof may be a fusion protein comprising the following structural formula (I) or (II):

N'-Y-[linker (1)]o-Z-C' (I)

N'-Z-[linker (1)]o-Y-C' (II)

in the structural formulas (I) and (II),
N' may be the N-terminus of the fusion protein,
C' may be the C-terminus of the fusion protein,
Y may be the IL-12A or the variant thereof,
Z may be the IL-12B or the variant thereof,
the linker (1) may be a peptide linker, and
o may be 0 or 1.

One embodiment of the IL-12 or the variant thereof may be a structure in which IL-12B or a variant thereof, a peptide linker, and IL-12A or a variant thereof are bound sequentially from the N-terminus. In addition, one embodiment of the IL-12 or the variant thereof may be a structure in which IL-12A or a variant thereof, a peptide linker, and IL-12B or a variant thereof are bound sequentially from the N-terminus.

As used herein, the term "IL-12A" is a 35 kDa subunit (p35) of IL-12, which binds to the 40 kDa subunit (p40) of IL-12 through a disulfide bond to generate an active cytokine.

The amino acid sequence of IL-12A may be one described in GenBank accession No. AAK84425 (for the amino acid sequence of mouse p35, see GenBank accession No. AAA39292). In addition, the IL-12A (p35) gene is a sequence encoding the IL-12A subunit, and may be a nucleotide sequence corresponding to a coding sequence (CDS) among the sequences described in GenBank accession No. AF404773 (for the mouse sequence, see GenBank accession No. M86672).

As used herein, the term "variant" of IL-12A includes an amino acid sequence having a function similar to or identical to that of a wild-type IL-12A protein. Specifically, it means that the amino acid sequence of IL-12A has a substitution, an insertion or a deletion as compared to the wild-type. Specifically, the IL-12A variant may be a fragment of IL-12A, and may have an amino acid sequence in which the 1^{st} to 22^{nd} amino acids of SEQ ID NO: 122 are deleted. More specifically, the variant of IL-12A may have the amino acid sequence of SEQ ID NO: 75.

In addition, IL-12A used in one embodiment of the present invention may be the amino acid sequence of SEQ ID NO: 75 as a human IL-12A. In one embodiment, a mouse IL-12A may be the amino acid sequence of SEQ ID NO: 87.

As used herein, the term "IL-12B" refers to a 40 kDa subunit (p40) of IL-12, which binds to a 35 kDa subunit (p35) of IL-12 through a disulfide bond to form IL-12, and also binds to the subunit p19 of IL-23 to form IL-23. On the other hand, IL-12B is also called natural killer cell stimulating factor 2.

The amino acid sequence of "IL-12B" may be one described in GenBank accession No. AAD56386 (for the amino acid sequence of mouse p40, see GenBank accession No. AAA39296). In addition, the IL-12B (p40) gene is a sequence encoding the IL-12B subunit, and may be a nucleotide sequence corresponding to a coding sequence (CDS) among the sequences described in GenBank accession No. AF180563 (for the mouse sequence, see GenBank accession No. M86671).

In addition, IL-12B used in one embodiment of the present invention may be a human IL-12B, and specifically, IL-12B may be the amino acid sequence of SEQ ID NO: 74. In one embodiment, a mouse IL-12B may have the amino acid sequence of SEQ ID NO: 86.

As used herein, the term "variant" of IL-12B includes an amino acid sequence having a function similar to or identical to that of a wild-type IL-12B protein. Specifically, it means that the amino acid sequence of IL-12B has a substitution, an insertion or a deletion as compared to the wild-type.

Specifically, the IL-12B variant may be one in which the 1^{st} to 22^{nd} amino acids of SEQ ID NO: 121 are deleted. More specifically, the IL-12B variant may be one in which 1 to 8 amino acids are substituted in the sequence of SEQ ID NO: 74. In addition, the IL-12B variant may be one in which the 1^{st} to 22^{nd} amino acids of SEQ ID NO: 123 are deleted. More specifically, the IL-12B variant may be one in which at most 1 to 8 amino acids are substituted in the sequence of SEQ ID NO: 86.

The variant of the IL-12B (p40) may be one in which an amino acid involved in heparin binding of IL-12 is substituted.

In one embodiment, the variant of the IL-12B (p40) may be a form in which lysine (K) binding to heparin in IL-12B is substituted with another amino acid. Specifically, it may be a form in which the 258^{th}, 260^{th}, 263^{rd} and 264^{th} lysines of SEQ ID NO: 74 are substituted with other amino acids. Specifically, the variant of the IL-12B (p40) may include an amino acid sequence obtained by at least one substitution selected from the group consisting of K258A, K260A, K263A, and K264A in the amino acid sequence of SEQ ID NO: 74. Specifically, the variant of the IL-12B (p40) may include an amino acid sequence obtained by a substitution of K258A and K263A in the amino acid sequence of SEQ ID NO: 74, or may further include a mutation of K260A and/or K264A in the amino acid sequence of SEQ ID NO: 74.

In one embodiment, the variant of the IL-12B (p40) may be a form in which arginine (R) or lysine (K) binding to heparin in IL-12B is substituted with another amino acid. Specifically, it may be a form in which the 254^{th} arginine and/or the 255^{th}, 256^{th} and 260^{th} lysines of SEQ ID NO: 86 are substituted with other amino acids. Specifically, the variant of the IL-12B (p40) may include an amino acid sequence obtained by at least one substitution selected from the group consisting of R254A, K255A, K256A and K260A in the amino acid sequence of SEQ ID NO: 86. Specifically, it may include mutations of R254A, K255A, K256A and K260A in the amino acid sequence of SEQ ID NO: 86.

In one embodiment, the variant of the IL-12B (p40) may include the amino acid sequence of SEQ ID NO: 77, SEQ ID NO: 79, SEQ ID NO: 89 or SEQ ID NO: 91.

In one embodiment, the human IL-12B variant may consist of the following structural formula A:

[Structural formula A] X₁-L-X₂

wherein, X₁ is the amino acid sequence of SEQ ID NO: 125,
X₂ is the amino acid sequence of SEQ ID NO: 126, and
L is an amino acid sequence consisting of V-A₁-A₂-Q-A₃-K*-A₄-A₅-A₆-A₇-K*-A₈.
A₁ is R or Q, A₂ is V, A or I, A₃ is G or R, A₄ is S, N or K*, A₅ is K*, N or E, A₆ is R or K, A₇ is E, M or T, and A₈ is K* or E.

At least one amino acid residue marked with "*" may be substituted with a non-polar amino acid residue selected from the group consisting of A, G, I, L, M, F, P and V, but is not limited thereto.

In one embodiment, the variant of the IL-12B (p40) may include a substitution of amino acids involved in heparin binding to reduce the cytokine-associated toxicity of IL-12 while maintaining the killing effect on cancer cells.

### FAP and antigen binding site that specifically binds to FAP

As used herein, the term "FAP (fibroblast activation protein)" refers to a fibroblast activation protein, which is a protein expressed on the cell surface and presented in the environment of tumor cells of various tumor types or in tumor cells of various tumor types. It is known that FAP is selectively overexpressed on the surface of cancer-associated fibroblasts (CAF) in the tumor microenvironment.

In addition, FAP is a homodimer containing two N-glycosylated subunits having a C-terminal extracellular domain in which the enzyme catalytic domain is located, and the glycosylated form of FAP has both post-prolyl dipeptidyl peptidase and gelatinase activities.

On the other hand, FAP is a prolyl endopeptidase, which is about 170 kDa membrane-bound gelatinase. The prolyl endopeptidase FAP may recognize and cleave a specific amino acid sequence. Accordingly, FAP is also referred to as FAPα, seprase, or circulating antiplasmin-cleaving enzyme.

The amino acid sequence of the FAP may be one described in GenBank accession No. AAC51668, and human FAP was first identified in cultured fibroblasts through the use of monoclonal antibody (mAb) F19 (see International Patent Application Publication No. WO 93/05804). FAP is expressed in many cancers and is utilized as a promising antigenic target for imaging, diagnosis and treatment of various carcinomas due to its limited expression in normal tissues.

The FAP includes any variant, isoform and species homologues of human FAP that is expressed either naturally by a cell or on a cell transfected with the FAP gene. The antibody of the present invention may be bound to FAP present on the membrane or the cell surface of cancer cells (tumor cells or cells of the tumor stroma) and may cause ADCC or other effector-mediated killing of cancer cells. In addition, it may be used to block the enzymatic activity of FAP (for example, serine peptidase, gelatinase, collagenase activity), FAP-mediated ECM degradation, and FAP-mediated cell invasion or migration.

As used herein, the term "specifically binding" refers to binding that is measurably different from a non-specific interaction. Specifically binding may be determined by competing with a control molecule similar to a target that does not have binding activity.

As used herein, the term "antigen binding site (epitope)" refers to a determinant that interacts with a specific antigen binding site in a variable region of the antibody. The antigen binding site is a group of molecules, such as amino acids or sugar side chains, which usually have specific structural characteristics as well as specific charge characteristics. The antigen binding site that specifically binds to FAP may be a generic term for molecules capable of antigen-antibody binding specifically to FAP.

In addition, the antibody or fragment thereof may be used in any form as long as it contains an antigen binding domain capable of specifically binding to FAP.

The antigen binding site may include other amino acids not directly involved in binding, or amino acids whose effects are blocked by amino acid residues of the antigen binding site.

Specifically, the antigen binding site may be an antibody that specifically binds to FAP or a fragment thereof.

As used herein, the term "antibody" refers to a molecule containing an antigen binding site, and an immunologically active fragment of an immunoglobulin molecule containing an antigen binding site. The immunoglobulin molecule may be an immunoglobulin molecule of IgG, IgE, IgM, IgD, IgA, IgY or a subclass thereof. Antibodies include synthetic antibodies, monoclonal antibodies, single domain antibodies, single chain antibodies, recombinantly produced antibodies, multispecific antibodies (including bispecific antibodies), human antibodies, humanized antibodies, chimeric antibodies, intrabodies, scFv (including for example monospecific and bispecific, etc.), Fab fragment, F(ab') fragment, disulfide-linked Fv (sdFv), anti-idiotypic (anti-Id) antibodies, and an antigen binding fragment of the antibodies, but are not limited thereto.

As used herein, the term "antibody fragment" may be a portion of an antibody, such as F(ab')₂, F(ab)₂, Fab', Fab, Fv, scFv. Regardless of structure, an antibody fragment binds to the same antigen recognized by the intact antibody.

In one embodiment, the antigen binding site that specifically binds to FAP may comprise a heavy chain variable region comprising HCDR1 of SEQ ID NO: 96, HCDR2 of SEQ ID NO: 97 and HCDR3 of SEQ ID NO: 98; and a light chain variable region comprising LCDR1 of SEQ ID NO: 99, LCDR2 of SEQ ID NO: 100 and LCDR3 of SEQ ID NO: 101. Then, in one embodiment, the antigen binding site that specifically binds to FAP may have a heavy chain variable region of SEQ ID NO: 102 and a light chain variable region of SEQ ID NO: 103.

In addition, in one embodiment, the antigen binding site that specifically binds to FAP may comprise a heavy chain variable region comprising HCDR1 of SEQ ID NO: 104, HCDR2 of SEQ ID NO: 105 and HCDR3 of SEQ ID NO: 106; and a light chain variable region comprising LCDR1 of SEQ ID NO: 107, LCDR2 of SEQ ID NO: 108 and LCDR3 of SEQ ID NO: 109. Then, in one embodiment, the antigen binding site that specifically binds to FAP may have a heavy chain variable region of SEQ ID NO: 110 and a light chain variable region of SEQ ID NO: 111.

In one embodiment, the antigen binding site that specifically binds to FAP may be scFv. Then, the scFv may comprise the above-described heavy chain and light chain CDRs. In one embodiment, the scFv that specifically binds to FAP may have the amino acid sequence of SEQ ID NO: 72 or SEQ ID NO: 84.

In addition, the antigen binding site that specifically binds to FAP may include a known anti-FAP antibody or a fragment thereof. The anti-FAP antibody or fragment thereof may refer to an antibody known to those skilled in the art without limitation.

The anti-FAP antibody or fragment thereof may include at least one variable region selected from the group consisting of NG-641, AMG-506/MP-0310, 28H1 of RG-7827, 4B9 of RG-7827, OMTX-705, 3F2, 4G8, 3D9, 4B3, 19G1, 20G8, 5B8, 5F1, 14B3, 16F1, 16F8, O3C9, 29B11, O2D7 and 23C10.

In another example, as the antibody, an anti-FAP antibody or a fragment thereof disclosed in Korean Patent Application Publication No. KR 20200037826 A, US Patent Application Publication No. US 2020-0385488 A1, US Patent No. US 3,924,445 B2, US Patent No. US 9,011,847 B2 or US Patent Application Publication No. US 2017-007716 A1 may be used.

In one embodiment, the anti-FAP antibody may comprise a variable region of NG-641. Specifically, the antibody may comprise a heavy chain variable region comprising HCDR1 of SEQ ID NO: 127, HCDR2 of SEQ ID NO: 128 and HCDR3 of SEQ ID NO: 129, and a light chain variable region comprising LCDR1 of SEQ ID NO: 130, LCDR2 of SEQ ID NO: 131 and LCDR3 of SEQ ID NO: 132. In addition, the anti-FAP antibody may comprise a heavy chain variable region of SEQ ID NO: 248, and a light chain variable region of SEQ ID NO: 249.

In one embodiment, the anti-FAP antibody may comprise a variable region of 28H1. Specifically, the antibody may comprise a heavy chain variable region comprising HCDR1 of SEQ ID NO: 133, HCDR2 of SEQ ID NO: 134 and HCDR3 of SEQ ID NO: 135, and a light chain variable region comprising LCDR1 of SEQ ID NO: 136, LCDR2 of SEQ ID NO: 137 and LCDR3 of SEQ ID NO: 138. In addition, the anti-FAP antibody may comprise a heavy chain variable region of SEQ ID NO: 250 and a light chain variable region of SEQ ID NO: 251.

In one embodiment, the anti-FAP antibody may comprise a variable region of 4B9. Specifically, the antibody may comprise a heavy chain variable region comprising HCDR1 of SEQ ID NO: 139, HCDR2 of SEQ ID NO: 140 and HCDR3 of SEQ ID NO: 141, and a light chain variable region comprising LCDR1 of SEQ ID NO: 142, LCDR2 of SEQ ID NO: 143 and LCDR3 of SEQ ID NO: 144. In addition, the anti-FAP antibody may comprise a heavy chain variable region of SEQ ID NO: 252 and a light chain variable region of SEQ ID NO: 253.

In one embodiment, the anti-FAP antibody may comprise a variable region of OMTX-705. Specifically, the antibody may comprise a heavy chain variable region comprising HCDR1 of SEQ ID NO: 145, HCDR2 of SEQ ID NO: 146 and HCDR3 of SEQ ID NO: 147, and a light chain variable region comprising LCDR1 of SEQ ID NO: 148, LCDR2 of SEQ ID NO: 149 and LCDR3 of SEQ ID NO: 150. In addition, the anti-FAP antibody may comprise a heavy chain variable region of SEQ ID NO: 254 and a light chain variable region of SEQ ID NO: 255.

In one embodiment, the anti-FAP antibody may comprise a variable region of 3F2. Specifically, the antibody may comprise a heavy chain variable region comprising HCDR1 of SEQ ID NO: 151, HCDR2 of SEQ ID NO: 152 and HCDR3 of SEQ ID NO: 153, and a light chain variable region comprising LCDR1 of SEQ ID NO: 154, LCDR2 of SEQ ID NO: 155 and LCDR3 of SEQ ID NO: 156. In addition, the anti-FAP antibody may comprise a heavy chain variable region of SEQ ID NO: 256 and a light chain variable region of SEQ ID NO: 257.

In one embodiment, the anti-FAP antibody may comprise a variable region of 4G8. Specifically, the antibody may comprise a heavy chain variable region comprising HCDR1 of SEQ ID NO: 157, HCDR2 of SEQ ID NO: 158 and HCDR3 of SEQ ID NO: 159, and a light chain variable region comprising LCDR1 of SEQ ID NO: 160, LCDR2 of SEQ ID NO: 161 and LCDR3 of SEQ ID NO: 162. In addition, the anti-FAP antibody may comprise a heavy chain variable region of SEQ ID NO: 258 and a light chain variable region of SEQ ID NO: 259.

In one embodiment, the anti-FAP antibody may comprise a variable region of 3D9. Specifically, the antibody may comprise a heavy chain variable region comprising HCDR1 of SEQ ID NO: 163, HCDR2 of SEQ ID NO: 164 and HCDR3 of SEQ ID NO: 165, and a light chain variable region comprising LCDR1 of SEQ ID NO: 166, LCDR2 of SEQ ID NO: 167 and LCDR3 of SEQ ID NO: 168. In addition, the anti-FAP antibody may comprise a heavy chain variable region of SEQ ID NO: 260 and a light chain variable region of SEQ ID NO: 261.

In one embodiment, the anti-FAP antibody may comprise a variable region of 4B3. Specifically, the antibody may comprise a heavy chain variable region comprising HCDR1 of SEQ ID NO: 169, HCDR2 of SEQ ID NO: 170 and HCDR3 of SEQ ID NO: 171, and a light chain variable region comprising LCDR1 of SEQ ID NO: 172, LCDR2 of SEQ ID NO: 173 and LCDR3 of SEQ ID NO: 174. In addition, the anti-FAP antibody may comprise a heavy chain variable region of SEQ ID NO: 262 and a light chain variable region of SEQ ID NO: 263.

In one embodiment, the anti-FAP antibody may comprise a variable region of 19G1. Specifically, the antibody may comprise a heavy chain variable region comprising HCDR1 of SEQ ID NO: 175, HCDR2 of SEQ ID NO: 176 and HCDR3 of SEQ ID NO: 177, and a light chain variable region comprising LCDR1 of SEQ ID NO: 178, LCDR2 of SEQ ID NO: 179 and LCDR3 of SEQ ID NO: 180. In addition, the anti-FAP antibody may comprise a heavy chain variable region of SEQ ID NO: 264 and a light chain variable region of SEQ ID NO: 265.

In one embodiment, the anti-FAP antibody may comprise a variable region of 20G8. Specifically, the antibody may comprise a heavy chain variable region comprising HCDR1 of SEQ ID NO: 181, HCDR2 of SEQ ID NO: 182 and HCDR3 of SEQ ID NO: 183, and a light chain variable region comprising LCDR1 of SEQ ID NO: 184, LCDR2 of SEQ ID NO: 185 and LCDR3 of SEQ ID NO: 186. In addition, the anti-FAP antibody may comprise a heavy chain variable region of SEQ ID NO: 266 and a light chain variable region of SEQ ID NO: 267.

In one embodiment, the anti-FAP antibody may comprise a variable region of 5B8. Specifically, the antibody may comprise a heavy chain variable region comprising HCDR1 of SEQ ID NO: 187, HCDR2 of SEQ ID NO: 188 and HCDR3 of SEQ ID NO: 189, and a light chain variable region comprising LCDR1 of SEQ ID NO: 190, LCDR2 of SEQ ID NO: 191 and LCDR3 of SEQ ID NO: 192. In addition, the anti-FAP antibody may comprise a heavy chain variable region of SEQ ID NO: 268 and a light chain variable region of SEQ ID NO: 269.

In one embodiment, the anti-FAP antibody may comprise a variable region of 5F1. Specifically, the antibody may comprise a heavy chain variable region comprising HCDR1 of SEQ ID NO: 193, HCDR2 of SEQ ID NO: 194 and HCDR3 of SEQ ID NO: 195, and a light chain variable region comprising LCDR1 of SEQ ID NO: 196, LCDR2 of SEQ ID NO: 197 and LCDR3 of SEQ ID NO: 198. In addition, the anti-FAP antibody may comprise a heavy chain variable region of SEQ ID NO: 270 and a light chain variable region of SEQ ID NO: 271.

In one embodiment, the anti-FAP antibody may comprise a variable region of 14B3. Specifically, the antibody may comprise a heavy chain variable region comprising HCDR1 of SEQ ID NO: 199, HCDR2 of SEQ ID NO: 200 and HCDR3 of SEQ ID NO: 201, and a light chain variable region comprising LCDR1 of SEQ ID NO: 202, LCDR2 of SEQ ID NO: 203 and LCDR3 of SEQ ID NO: 204. In addition, the anti-FAP antibody may comprise a heavy chain variable region of SEQ ID NO: 272 and a light chain variable region of SEQ ID NO: 273.

In one embodiment, the anti-FAP antibody may comprise a variable region of 16F1. Specifically, the antibody may comprise a heavy chain variable region comprising HCDR1 of SEQ ID NO: 205, HCDR2 of SEQ ID NO: 206 and HCDR3 of SEQ ID NO: 207, and a light chain variable region comprising LCDR1 of SEQ ID NO: 208, LCDR2 of SEQ ID NO: 209 and LCDR3 of SEQ ID NO: 210. In addition, the anti-FAP antibody may comprise a heavy chain variable region of SEQ ID NO: 274 and a light chain variable region of SEQ ID NO: 275.

In one embodiment, the anti-FAP antibody may comprise a variable region of 16F8. Specifically, the antibody may comprise a heavy chain variable region comprising HCDR1 of SEQ ID NO: 211, HCDR2 of SEQ ID NO: 212 and HCDR3 of SEQ ID NO: 213, and a light chain variable region comprising LCDR1 of SEQ ID NO: 214, LCDR2 of SEQ ID NO: 215 and LCDR3 of SEQ ID NO: 216. In addition, the anti-FAP antibody may comprise a heavy chain variable region of SEQ ID NO: 276 and a light chain variable region of SEQ ID NO: 277.

In one embodiment, the anti-FAP antibody may comprise a variable region of O3C9. Specifically, the antibody may comprise a heavy chain variable region comprising HCDR1 of SEQ ID NO: 217, HCDR2 of SEQ ID NO: 218 and HCDR3 of SEQ ID NO: 219, and a light chain variable region comprising LCDR1 of SEQ ID NO: 220, LCDR2 of SEQ ID NO: 221 and LCDR3 of SEQ ID NO: 222. In addition, the anti-FAP antibody may comprise a heavy chain variable region of SEQ ID NO: 278 and a light chain variable region of SEQ ID NO: 279.

In one embodiment, the anti-FAP antibody may comprise a variable region of 22A3. Specifically, the antibody may comprise a heavy chain variable region comprising HCDR1 of SEQ ID NO: 223, HCDR2 of SEQ ID NO: 224 and HCDR3 of SEQ ID NO: 225, and a light chain variable region comprising LCDR1 of SEQ ID NO: 226, LCDR2 of SEQ ID NO: 227 and LCDR3 of SEQ ID NO: 228. In addition, the anti-FAP antibody may comprise a heavy chain variable region of SEQ ID NO: 280 and a light chain variable region of SEQ ID NO: 281.

In one embodiment, the anti-FAP antibody may comprise a variable region of 29B11. Specifically, the antibody may comprise a heavy chain variable region comprising HCDR1 of SEQ ID NO: 229, HCDR2 of SEQ ID NO: 230 and HCDR3 of SEQ ID NO: 231, and a light chain variable region comprising LCDR1 of SEQ ID NO: 232, LCDR2 of SEQ ID NO: 233 and LCDR3 of SEQ ID NO: 234. In addition, the anti-FAP antibody may comprise a heavy chain variable region of SEQ ID NO: 282 and a light chain variable region of SEQ ID NO: 283.

In one embodiment, the anti-FAP antibody may comprise a variable region of O2D7. Specifically, the antibody may comprise a heavy chain variable region comprising HCDR1 of SEQ ID NO: 235, HCDR2 of SEQ ID NO: 236 and HCDR3 of SEQ ID NO: 237, and a light chain variable region comprising LCDR1 of SEQ ID NO: 238, LCDR2 of SEQ ID NO: 239 and LCDR3 of SEQ ID NO: 240. In addition, the anti-FAP antibody may comprise a heavy chain variable region of SEQ ID NO: 284 and a light chain variable region of SEQ ID NO: 285.

In one embodiment, the anti-FAP antibody may comprise a variable region of 23C10. Specifically, the antibody may comprise a heavy chain variable region comprising HCDR1 of SEQ ID NO: 241, HCDR2 of SEQ ID NO: 242 and HCDR3 of SEQ ID NO: 243, and a light chain variable region comprising LCDR1 of SEQ ID NO: 244, LCDR2 of SEQ ID NO: 245 and LCDR3 of SEQ ID NO: 246. In addition, the anti-FAP antibody may comprise a heavy chain variable region of SEQ ID NO: 286 and a light chain variable region of SEQ ID NO: 287.

In one embodiment, the anti-FAP antibody may be AMG-506/MP-0310. Specifically, the antibody may include the amino acid sequence of SEQ ID NO: 247.

The anti-FAP antibody specifically binds to FAP present on cancer cells, and any antibody may be used as long as it may induce the death of cancer cells.

### Structure of first monomer

The first monomer comprises IL-12 or a variant thereof.

The first monomer may further comprise an antigen binding site that specifically binds to FAP.

In one embodiment, the first monomer may comprise IL-12 or a variant thereof; and an Fc region fragment or a variant thereof.

In one embodiment, the first monomer may comprise IL-12 or a variant thereof, an Fc region fragment or a variant thereof, and an antigen binding site that specifically binds to FAP.

The first monomer may include the following structural formula (III) or (IV):

N'-X-[linker (2)]p-Fc region fragment or variant thereof-[linker (3)]q-(T)r-C' (III)

N'-(T)r-[linker (2)]q-Fc region fragment or variant thereof-[linker (3)]p-X-C' (IV)

in the structural formulas (III) and (IV),
N' may be the N-terminus of the fusion protein,
C' may be the C-terminus of the fusion protein,
X may be the structural formula (I) or (II),
T may be the antigen binding site that specifically binds to FAP,
the linkers (2) and (3) may be peptide linkers, and
p, q and r may be each independently 0 or 1.

In one embodiment, when r is 0, the first monomer may be in a form of a fusion protein in which IL-12 or a variant thereof and an Fc region fragment or a variant thereof are linked by a peptide linker.

Then, the first monomer may include the amino acid sequence of SEQ ID NO: 3, SEQ ID NO: 4, SEQ ID NO: 5, SEQ ID NO: 15, SEQ ID NO: 20, SEQ ID NO: 21, SEQ ID NO: 22 or SEQ ID NO: 32.

Then, in the structural formula (III), when r is 1, the first monomer may be in a form in which a monomer of an anti-FAP antibody is bound to IL-12 or a variant thereof.

Then, in the structural formula (III), when r is 1, the first monomer may include the amino acid sequence of SEQ ID NO: 292, SEQ ID NO: 293, SEQ ID NO: 296, SEQ ID NO: 297, SEQ ID NO: 298, SEQ ID NO: 299, SEQ ID NO: 302 or SEQ ID NO: 303.

Then, in the structural formula (IV), when r is 1, the first monomer may include the amino acid sequence of SEQ ID NO: 294, SEQ ID NO: 295, SEQ ID NO: 300 or SEQ ID NO: 301.

In one embodiment, when r is 1, the structural formula (III) may include the following structural formulas (III') and (III"):

N'-X-[linker (2)]p-Fc region fragment or variant thereof-[linker (3)]q-(T')-C' (III')

N'-(T")-C' (III")

in the structural formulas (III') and (III"),
T' is a heavy chain region of an antibody that specifically binds to FAP, comprising a variable region and a CH1 region, or a light chain region of the antibody;
T" is a light chain region of an antibody that specifically binds to FAP, or a heavy chain region of the antibody, comprising a variable region and a CH1 region;
wherein, T' and T" bind to each other to form a variable region of the antibody, wherein the variable region specifically binds to FAP;
the linkers (2) to (3) are peptide linkers,
p and q are each independently 0 or 1, and
N', X and C' are as defined above.

In one embodiment, when r is 1, the structural formula (IV) may include the following structural formulas (IV') and (IV"):

N'-(T')-[linker (2)]q-Fc region fragment or variant thereof-[linker (3)]p-X-C' (IV')

; and

N'-(T")-C' (IV")

in the structural formulas (IV') and (IV"),
T' is a heavy chain region of an antibody that specifically binds to FAP, comprising a variable region and a CH1 region, or a light chain region of the antibody;
T" is a light chain region of an antibody that specifically binds to FAP, or a heavy chain region of the antibody, comprising a variable region and a CH1 region;
wherein, T' and T" bind to each other to form a variable region of the antibody, wherein the variable region specifically binds to FAP;
the linkers (2) to (3) are peptide linker,
p and q are each independently 0 or 1, and
N', X and C' are as defined above.

Then, the first monomer may include the amino acid sequence of SEQ ID NO: 10 and SEQ ID NO: 2; SEQ ID NO: 11 and SEQ ID NO: 2; SEQ ID NO: 27 and SEQ ID NO: 19; SEQ ID NO: 28 and SEQ ID NO: 19; SEQ ID NO: 294 and SEQ ID NO: 2; SEQ ID NO: 295 and SEQ ID NO: 2; SEQ ID NO: 300 and SEQ ID NO: 19; or SEQ ID NO: 301 and SEQ ID NO: 19.

### Structure of second monomer

The second monomer may further comprise an antigen binding site that specifically binds to FAP.

In one embodiment, the second monomer may comprise a first FAP binding site; and a second FAP binding site.

The antigen binding site that specifically binds to FAP may be Fab, scFv, Fv, or a fragment thereof. Then, the first FAP binding site may be Fab, and the second FAP binding site may be Fv or scFv.

The second FAP binding site may be bound to the N-terminus or C-terminus of the second monomer. Specifically, the second monomer may be additionally bound to the C-terminus of the heavy chain, the C-terminus of the light chain, or the C-terminus of the variable region.

The second monomer may include the following structural formula (V):

N'-(R)s-[linker (4)]t-Q-[linker (5)]u-Fc region fragment or variant thereof-[linker (6)]v-(W)a-C' (V)

in the structural formula (V),
N' may be the N-terminus of the fusion protein,
C' may be the C-terminus of the fusion protein,
R and Q may be an antigen binding site that specifically binds to FAP,
W may be scFv that specifically binds to FAP; or the IL-12 of structural formula (I) or (II) or the variant thereof,
the linkers (4), (5) and (6) may be each peptide linkers, and
s, t, u, v and a may be each independently 0 or 1.

In one embodiment, the structural formula (V) may include at least one amino acid sequence selected from the group consisting of SEQ ID NO: 1, SEQ ID NO: 2, SEQ ID NO: 6, SEQ ID NO: 7, SEQ ID NO: 8, SEQ ID NO: 9, SEQ ID NO: 14, SEQ ID NO: 18, SEQ ID NO: 19, SEQ ID NO: 23, SEQ ID NO: 24, SEQ ID NO: 25, SEQ ID NO: 26, SEQ ID NO: 31, SEQ ID NO: 294, SEQ ID NO: 295, SEQ ID NO: 300 and SEQ ID NO: 301.

In one embodiment, the structural formula (V) may include the following structural formulas (V') and (V"):

N'-(R')s-[linker (4)]t-Q'-[linker (5)]u-Fc region fragment or variant thereof-[linker (6)]p-(W)a-C' (V')

N'-(R")s-[linker (4)]t-Q"-[linker (7)]x-(W)b-C' (V")

in the structural formulas (V') and (V"),
R' may be a heavy chain region of an antibody that specifically binds to FAP, comprising a variable region and a CH1 region, or a light chain region of the antibody;
R" may be a light chain region of an antibody that specifically binds to FAP, or a heavy chain region of the antibody, comprising a variable region and a CH1 region;
wherein R' and R" may bind to each other to form a variable region of the antibody, wherein the variable region specifically binds to FAP;
Q' may be a heavy chain region of an antibody that specifically binds to FAP, comprising a variable region and a CH1 region, or a light chain region of the antibody;
Q" may be a light chain region of an antibody that specifically binds to FAP, or a heavy chain region of the antibody, comprising a variable region and a CH1 region;
wherein Q' and Q" may bind to each other to form a variable region of the antibody, wherein the variable region specifically binds to FAP,
W may be scFv that specifically binds to FAP; or the IL-12 of structural formula (I) or (II) or the variant thereof,
the linkers (4), (5), (6) and (7) may be each peptide linkers, and
s, t, u, x, a and b may be each independently 0 or 1.

In one embodiment, the structural formula (V') may be SEQ ID NO: 1, SEQ ID NO: 6, SEQ ID NO: 8, SEQ ID NO: 14, SEQ ID NO: 18, SEQ ID NO: 23, SEQ ID NO: 25, SEQ ID NO: 31, SEQ ID NO: 294, SEQ ID NO: 295, SEQ ID NO: 300 or SEQ ID NO: 301.

In one embodiment, the structural formula (V") may be SEQ ID NO: 2, SEQ ID NO: 7, SEQ ID NO: 9, SEQ ID NO: 19, SEQ ID NO: 24 or SEQ ID NO: 26.

In one embodiment, the second monomer may include a heavy chain consisting of the amino acid sequence of SEQ ID NO: 102 and a light chain consisting of the amino acid sequence of SEQ ID NO: 103; or a heavy chain consisting of the amino acid sequence of SEQ ID NO: 110 and a light chain consisting of the amino acid sequence of SEQ ID NO: 111.

In addition, when W is scFv, W may have the amino acid sequence of SEQ ID NO: 72 or SEQ ID NO: 84.

### Peptide linker

As used herein, the term "peptide linker" refers to a peptide used to provide a physicochemical distance or to connect between domains in a fusion protein. The linker may comprise a hinge region of an immunoglobulin.

The peptide linkers (1) to (7) refer to peptide linkers composed of amino acids. Specifically, the peptide linker (2) or (5) may consist of 5 to 80 consecutive amino acids, 7 to 70 consecutive amino acids, or 10 to 60 consecutive amino acids, or 12 to 50 amino acids. The peptide linker may include (G₄S)n (wherein, n is an integer from 1 to 10). Then, in (G₄S)n, n may be each 1, 2, 3, 4, 5, 6, 7, 8, 9 or 10.

In one embodiment, the peptide linker (1) may be a peptide linker consisting of the amino acid sequence of SEQ ID NO: 93, SEQ ID NO: 94 or SEQ ID NO: 95.

The peptide linker (2) or (5) may consist of 5 to 80 consecutive amino acids, 7 to 70 consecutive amino acids, or 10 to 60 consecutive amino acids, or 12 to 50 amino acids. In one embodiment, the peptide linker (2) may consist of 30 amino acids. In addition, the peptide linker (2) may comprise at least one cysteine. Specifically, it may comprise one, two or three cysteines. In addition, the peptide linker (2) may be derived from a hinge of an immunoglobulin, and may further include (G₄S)n (wherein, n is an integer from 1 to 10). Specifically, the peptide linker (2) may comprise a hinge region consisting of an amino acid sequence of any one of SEQ ID NO: 112, SEQ ID NO: 113, SEQ ID NO: 114, SEQ ID NO: 115, SEQ ID NO: 116, SEQ ID NO: 117, and SEQ ID NO: 119.

In addition, the peptide linkers (3), (4), (6) and (7) may consist of 1 to 30 consecutive amino acids, 5 to 20 consecutive amino acids, or 10 to 15 consecutive amino acids. The peptide linker may include (G₄S)n (wherein, n is an integer from 1 to 10). Then, in (G₄S)n, n may be each 1, 2, 3, 4, 5, 6, 7, 8, 9 or 10.

In addition, specifically, the peptide linker (2), (3) or (5) may include SEQ ID NO: 93 or SEQ ID NO: 94.

In one embodiment of the present invention, the peptide linker (2) or (5) may be a peptide linker comprising the amino acid sequence of SEQ ID NO: 118 or 120.

### Fc region or fragment thereof

Then, the above-described immunoglobulin fragment may be an Fc region of an immunoglobulin. The Fc region of an immunoglobulin may be a wild-type Fc domain as well as an Fc domain variant. Then, the Fc region may be an Fc region of IgG, IgA, IgE, IgD or IgM. Specifically, it may be derived from IgG1 or IgG2a.

As used herein, the term "Fc domain variant" may refer to a form which is different from the wild-type Fc domain in terms of glycosylation pattern, has a high glycosylation as compared with the wild-type Fc domain, has a low glycosylation as compared with the wild-type Fc domain, or has a deglycosylated form. In addition, an aglycosylated Fc domain is included therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD or IgM. In addition, the Fc domain variant may be in a form in which some amino acids of the Fc domain are substituted with other amino acids.

The "amino acid" introduced by the substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenylalanine (F), proline (P), serine (S), threonine (T), tryptophan (W), tyrosine (Y) and valine (V).

In addition, the Fc region may comprise a knob structure or a hole structure.

As used herein, the term "knob-into-hole" is a design strategy for manufacturing an antibody that specifically binds to different regions, such as a bispecific antibody, a multispecific antibody, or a heterodimeric antibody. The knob-into-hole is also called knob-in-hole. In general, this technique involves introducing a knob at the interface of a first polypeptide (for example, a first CH3 domain of a first antibody heavy chain) and a corresponding hole at the interface of a second polypeptide (for example, a second CH3 domain of a second antibody heavy chain), so that the knob may be positioned within the hole to promote heterodimer formation and hinder homodimer formation.

A 'knob' is constructed by replacing small amino acid side chains from the interface of a first polypeptide (for example, a first CH3 domain of a first antibody heavy chain) with larger side chains (for example, arginine, phenylalanine, tyrosine or tryptophan). A complementary 'hole' of the same or similar size in the knob is produced by replacing large amino acid side chains from the interface of a second polypeptide (for example, a second CH3 domain of a second antibody heavy chain) with smaller side chains (for example, alanine, serine, valine, or threonine). The knob and hole may be produced by altering a nucleic acid encoding a polypeptide, for example, by site-specific mutagenesis or by peptide synthesis.

In one embodiment, the knob structure of the IgG1 may be SEQ ID NO: 13 or 288, and the hole structure may be SEQ ID NO: 12 or 289. The knob structure or the hole structure may be a form in which the 146^{th}, 148^{th} and 187^{th} amino acids are substituted with other amino acids. Specifically, in one embodiment of the present invention, the knob structure or the hole structure may be a form in which one or more selected from the group consisting of T146W, T146S, L148A and Y187V are substituted. Specifically, the knob structure may be a form in which it is substituted with T146W, and the hole structure may be a form in which it is substituted with T146S, L148A and Y187V.

On the other hand, in one embodiment of the present invention, the knob structure of the IgG2a may be SEQ ID NO: 30 or 290, and the hole structure may be SEQ ID NO: 29 or 291. The knob structure or the hole structure may be a form in which the 152^{nd}, 154^{th} and 193^{rd} amino acids of the amino acid sequence of SEQ ID NO: 305 are substituted with other amino acids. Specifically, in one embodiment of the present invention, the knob structure or the hole structure may be a form in which the amino acid sequence of SEQ ID NO: 305 is substituted with T152W or T152S, M154A and Y193V. Specifically, the knob structure may be a form in which it is substituted with T152W, and the hole structure may be a form in which it is substituted with T152S, M154A and Y193V.

In one embodiment, the Fc domain variant may include a DANG mutation or an NG mutation. Then, a "DANG mutation" refers to the D265A/N297G mutation for removing an effector function in human IgG1 or mouse IgG2a.

The effector functions mediated by the Fc region in an IgG molecule include C1q binding, complement dependent cytotoxicity, Fc receptor binding, antibody dependent cell mediated cytotoxicity (ADCC), phagocytosis, downregulation of cell surface receptors (for example, B cell receptor, BCR) and the like. In general, these effector functions require the binding of the Fc region with a binding domain (for example, an antibody variable domain).

The effector function may be changed by the substitution of the amino acid sequence of the non-mutated Fc region, and the Fc region in which the effector function is changed may be designed for example, by modifying C1q binding and/or FcR binding, thereby changing CDC activity and/or ADCC activity. That is, the 'DANG mutation' means that the effector function mediated by the Fc region is removed from the IgG molecule so that an unwanted effector fuction does not occur during antibody production.

In one embodiment, the DANG mutation may be a form in which the amino acid in human IgG1 of SEQ ID NO: 289 is substituted with D45A/N77G. In addition, it may be a form in which the amino acid in mouse IgG2a of SEQ ID NO: 291 is substituted with D51A/N83G.

### Structure of fusion protein

The fusion protein may be an antibody. Specifically, the antibody may be a heterodimeric antibody including a knob-in-hole structure.

The fusion protein may include structural formula (III) and structural formula (V); or structural formula (IV) and structural formula (V).

More specifically, one embodiment of the fusion protein may comprise a first monomer of (i) or (ii) below; and a second monomer of (iii), (iv), (v) or (vi) below:

### Examples of a first monomer:

(i) when r is 0, structural formula (III)
(ii) when r is 1, structural formula (IV)

### Examples of a second monomer:

(iii) when s, a and b are 0, structural formula (V') and structural formula (V")
(iv) when s is 1 and a and b are 0, structural formula (V') and structural formula (V")
(v) when s and b are 0 and a is 1, structural formula (V') and structural formula (V")
(vi) when b is 1 and s and a are 0, structural formula (V') and structural formula (V").

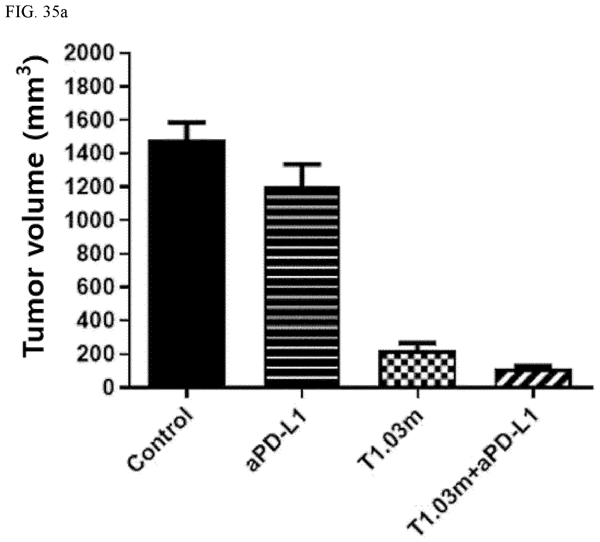

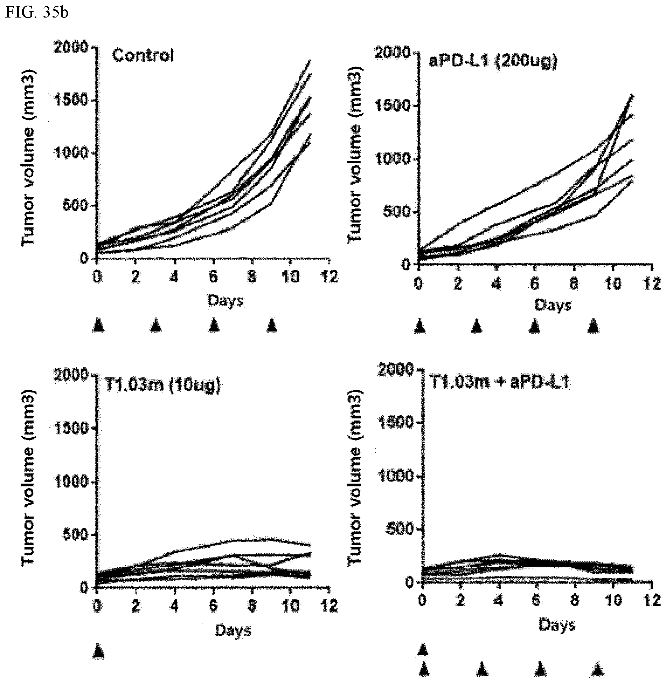

In one embodiment, the fusion protein may comprise a first monomer comprising (i) above; and a second monomer comprising (iii) above (first from the left in FIG. 35). Then, the fusion protein may include at least one amino acid sequence selected from the group consisting of SEQ ID NO: 1, SEQ ID NO: 2, SEQ ID NO: 3, SEQ ID NO: 4, SEQ ID NO: 5, SEQ ID NO: 18, SEQ ID NO: 19, SEQ ID NO: 20, SEQ ID NO: 21 and SEQ ID NO: 22. Specifically, the fusion protein may include SEQ ID NO: 1, SEQ ID NO: 2 and SEQ ID NO: 3; SEQ ID NO: 1, SEQ ID NO: 2 and SEQ ID NO: 4; SEQ ID NO: 1, SEQ ID NO: 2 and SEQ ID NO: 5; SEQ ID NO: 18, SEQ ID NO: 19 and SEQ ID NO: 20; SEQ ID NO: 18, SEQ ID NO: 19 and SEQ ID NO: 21; or SEQ ID NO: 18, SEQ ID NO: 19 and SEQ ID NO: 22.

In one embodiment, the fusion protein may comprise a first monomer comprising (i) above; and a second monomer comprising (iv) above (second from the left in FIG. 35). Then, the fusion protein may include at least one amino acid sequence selected from the group consisting of SEQ ID NO: 3, SEQ ID NO: 5, SEQ ID NO: 6, SEQ ID NO: 7, SEQ ID NO: 20, SEQ ID NO: 22, SEQ ID NO: 23 and SEQ ID NO: 24. Specifically, the fusion protein may include SEQ ID NO: 3, SEQ ID NO: 6 and SEQ ID NO: 7; SEQ ID NO: 5, SEQ ID NO: 6 and SEQ ID NO: 7; SEQ ID NO: 20, SEQ ID NO: 23 and SEQ ID NO: 24; or SEQ ID NO: 22, SEQ ID NO: 23 and SEQ ID NO: 24.

In one embodiment, the fusion protein may comprise a first monomer comprising (i) above; and a second monomer comprising (v) above (third from the left in FIG. 35). Then, the fusion protein may include at least one amino acid sequence selected from the group consisting of SEQ ID NO: 2, SEQ ID NO: 3, SEQ ID NO: 5, SEQ ID NO: 8, SEQ ID NO: 19, SEQ ID NO: 20, SEQ ID NO: 22 and SEQ ID NO: 25. Specifically, the fusion protein may include SEQ ID NO: 2, SEQ ID NO: 3 and SEQ ID NO: 8; SEQ ID NO: 2, SEQ ID NO: 5 and SEQ ID NO: 8; SEQ ID NO: 19, SEQ ID NO: 20 and SEQ ID NO: 25; or SEQ ID NO: 19, SEQ ID NO: 22 and SEQ ID NO: 25.

In one embodiment, the fusion protein may comprise a first monomer comprising (i) above; and a second monomer comprising (vi) above (fourth from the left in FIG. 35). Then, the fusion protein may include at least one amino acid sequence selected from the group consisting of SEQ ID NO: 1, SEQ ID NO: 3, SEQ ID NO: 5, SEQ ID NO: 9, SEQ ID NO: 18, SEQ ID NO: 20, SEQ ID NO: 22 and SEQ ID NO: 26. Specifically, the fusion protein may include SEQ ID NO: 1, SEQ ID NO: 3 and SEQ ID NO: 9; SEQ ID NO: 1, SEQ ID NO: 5 and SEQ ID NO: 9; SEQ ID NO: 18, SEQ ID NO: 20 and SEQ ID NO: 26; or SEQ ID NO: 18, SEQ ID NO: 22 and SEQ ID NO: 26.

In one embodiment, the fusion protein may comprise a first monomer comprising (ii) above; and a second monomer comprising (iii) above (fifth from the left in FIG. 35). Then, the fusion protein may include at least one amino acid sequence selected from the group consisting of SEQ ID NO: 1, SEQ ID NO: 2, SEQ ID NO: 10, SEQ ID NO: 11, SEQ ID NO: 18, SEQ ID NO: 19, SEQ ID NO: 27 and SEQ ID NO: 28. Specifically, the fusion protein may include SEQ ID NO: 1, SEQ ID NO: 2 and SEQ ID NO: 10; SEQ ID NO: 1, SEQ ID NO: 2 and SEQ ID NO: 11; SEQ ID NO: 18, SEQ ID NO: 19 and SEQ ID NO: 27; or SEQ ID NO: 18, SEQ ID NO: 19 and SEQ ID NO: 28.

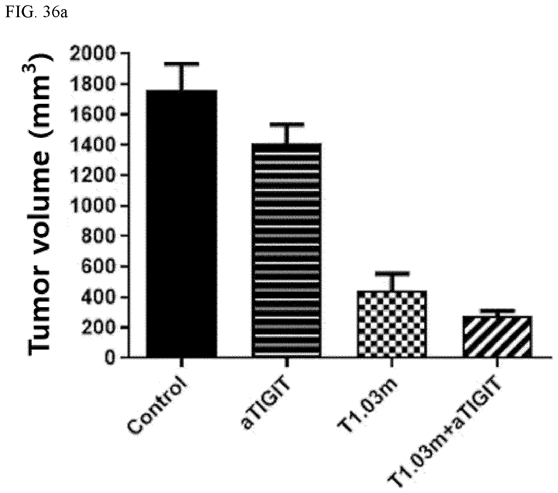

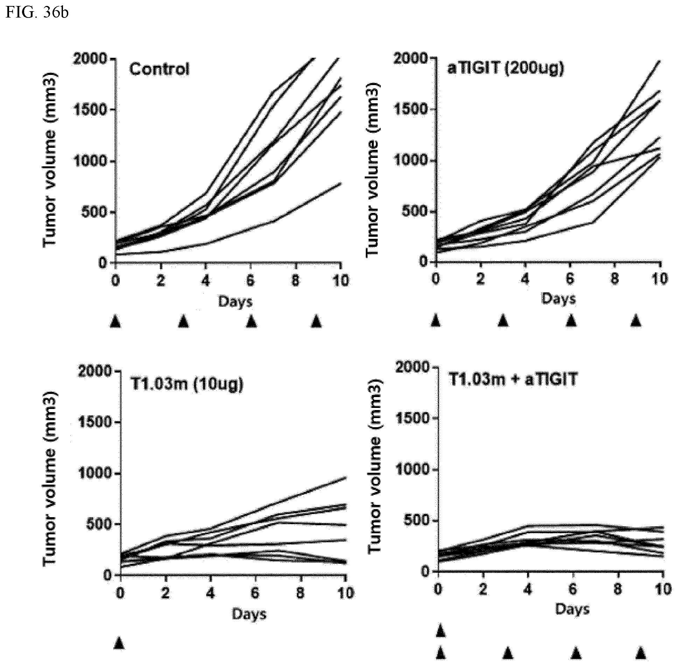

In addition, in one embodiment, a fusion protein dimer comprising IL-12 or a variant thereof and an antigen binding site that specifically binds to FAP may be a fusion protein dimer comprising the monomer of the structural formula (III) and the monomer of the structural formula (V) (left in FIG. 36). Then, in the structural formula (III), r is 1, and in the structural formula (V), s, a and t are 0. Then, the structural formula (V) may include (V') and (V"). Then, in the (V') and (V"), s, a, t and b are 0. In addition, the Fc region of the dimer has a knob-in-hole structure, through which different fusion proteins may be bound to each other.

Specifically, the fusion protein may include at least one amino acid sequence selected from the group consisting of SEQ ID NO: 1, SEQ ID NO: 2, SEQ ID NO: 18, SEQ ID NO: 19, SEQ ID NO: 292, SEQ ID NO: 293, SEQ ID NO: 298 and SEQ ID NO: 299. Specifically, the fusion protein may include SEQ ID NO: 1, SEQ ID NO: 2 and SEQ ID NO: 292; SEQ ID NO: 1, SEQ ID NO: 2 and SEQ ID NO: 293; SEQ ID NO: 18, SEQ ID NO: 19 and SEQ ID NO: 298; or SEQ ID NO: 18, SEQ ID NO: 19 and SEQ ID NO: 299.

In addition, a fusion protein dimer comprising IL-12 or a variant thereof and an antigen binding site that specifically binds to FAP may be a fusion protein dimer including a structure of the structural (V) (middle in FIG. 36). Then, the fusion protein dimer may include (V') and (V"). Then, in the structural formula (V), s and t are 0, and a is 1. In addition, in the (V') and (V"), s, t and b are 0, and a is 1. In addition, the fusion protein dimer may be a fusion protein dimer including a structure of the structural formula (IV). Then, in the structural formula (IV), r is 1.

Specifically, the fusion protein may include at least one amino acid sequence selected from the group consisting of SEQ ID NO: 2, SEQ ID NO: 19, SEQ ID NO: 294, SEQ ID NO: 295, SEQ ID NO: 300 and SEQ ID NO: 301. Specifically, the fusion protein may include SEQ ID NO: 2 and SEQ ID NO: 294; SEQ ID NO: 2 and SEQ ID NO: 295; SEQ ID NO: 19 and SEQ ID NO: 300; or SEQ ID NO: 19 and SEQ ID NO: 301.

In one embodiment, a fusion protein dimer comprising IL-12 or a variant thereof and an antigen binding site that specifically binds to FAP may be a fusion protein dimer including a structure of the structural formula (III) (right in FIG. 36). Then, in the structural formula (III), r is 1.

Specifically, the fusion protein may include SEQ ID NO: 296, SEQ ID NO: 297, SEQ ID NO: 302 or SEQ ID NO: 303.

The multispecific fusion protein herein may be in a chemically modified form. In one embodiment, the fusion protein may be chemically modified by glycosylation, acetylation, PEGylation, phosphorylation, amidation, derivatization with known protecting/blocking groups, proteolytic cleavage and/or binding to cellular ligands or other proteins. Many of these chemical modifications may be performed by known techniques.

### Polynucleotide encoding fusion protein

In another aspect of the present invention, there is provided a polynucleotide encoding the first monomer or a polynucleotide encoding the second monomer.

In one embodiment, a polypeptide of the first monomer may comprise an amino acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 3, SEQ ID NO: 4, SEQ ID NO: 5, SEQ ID NO: 10, SEQ ID NO: 11, SEQ ID NO: 15, SEQ ID NO: 20, SEQ ID NO: 21, SEQ ID NO: 22, SEQ ID NO: 27, SEQ ID NO: 28, SEQ ID NO: 32, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, or SEQ ID NO: 303.

In one embodiment, a polynucleotide encoding the first monomer may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 38, SEQ ID NO: 39, SEQ ID NO: 40, SEQ ID NO: 45, SEQ ID NO: 46, SEQ ID NO: 50, SEQ ID NO: 55, SEQ ID NO: 56, SEQ ID NO: 57, SEQ ID NO: 62, SEQ ID NO: 63, or SEQ ID NO: 67.

In one embodiment, a polypeptide of the second monomer may comprise an amino acid sequence having about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 1, SEQ ID NO: 2, SEQ ID NO: 6, SEQ ID NO: 7, SEQ ID NO: 8, SEQ ID NO: 9, SEQ ID NO: 14, SEQ ID NO: 18, SEQ ID NO: 19, SEQ ID NO: 23, SEQ ID NO: 24, SEQ ID NO: 25, SEQ ID NO: 26, SEQ ID NO: 31, SEQ ID NO: 294, SEQ ID NO: 295, SEQ ID NO: 300 or SEQ ID NO: 301.

In one embodiment, a polynucleotide encoding the second monomer may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 36, SEQ ID NO: 37, SEQ ID NO: 41, SEQ ID NO: 42, SEQ ID NO: 43, SEQ ID NO: 44, SEQ ID NO: 49, SEQ ID NO: 53, SEQ ID NO: 54, SEQ ID NO: 58, SEQ ID NO: 59, SEQ ID NO: 60, SEQ ID NO: 61, SEQ ID NO: 66, SEQ ID NO: 306.

The polynucleotide may further comprise a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates transportation of a protein across the endoplasmic reticulum (ER) membrane.

Signal sequences are well known in the art for their characteristics. Such signal sequences typically contain 16 to 30 amino acid residues, and may contain more or fewer amino acid residues than such amino acid residues. A typical signal peptide is composed of three regions, that is, a basic N-terminal region, a central hydrophobic region, and a more polar C-terminal region. The central hydrophobic region contains 4 to 12 hydrophobic residues that cause the signal sequence to be immobilized during transportation of an immature polypeptide through the membrane lipid bilayer.

After initiation, signal sequences are cleaved in the lumen of ER by cellular enzymes, commonly known as signal peptidases. Then, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used. In addition, a wild-type signal sequence may be used, or a signal sequence that has been substituted with a codon having high expression frequency in a host cell may be used.

### Use of pharmaceutical composition comprising fusion protein and anticancer agent

A composition comprising a fusion protein comprising IL-12 or a variant thereof and an antigen binding site that specifically binds to FAP; and an anticancer agent as an active ingredient of the present invention may be used for treating or preventing cancer, and/or may increase the efficacy of the treatment.

Accordingly, the present invention can provide a use of the fusion protein for treating cancer. In addition, the use may be for treating cancer in combination with the anticancer agent. Furthermore, the present invention provides a use of the fusion protein for preparing a medicament for treating cancer in combination with an anticancer agent.

The fusion protein and fusion protein dimer are as described above.

Then, the cancer may be any one selected from the group consisting of gastric cancer, liver cancer, lung cancer, colorectal cancer, breast cancer, prostate cancer, skin cancer, bone cancer, multiple myeloma, glioma, ovarian cancer, pancreatic cancer, cervical cancer, thyroid cancer, laryngeal cancer, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer and lymphoma.

A preferred dose of the pharmaceutical composition varies depending on the patient's condition and body weight, severity of disease, form of drug, route and duration of administration and may be appropriately selected by those skilled in the art. In the pharmaceutical composition for treating or preventing tumor of the present invention, the active ingredient may be contained in any amount (effective amount) depending on application, dosage form, blending purpose, and the like, as long as the active ingredient may exhibit an activity of treating tumor or, in particular, a therapeutic effect on cancer. A conventional effective amount thereof will be determined within a range of 0.001% to 20.0% by weight, based on the total weight of the composition. Then, the term "effective amount" refers to an amount of an active ingredient capable of inducing an effect of improving or treating the condition of a disease, and in particular, inducing an effect of improving or treating the condition of cancer. Such an effective amount may be experimentally determined within the scope of common knowledge of those skilled in the art.

As used herein, the term "treatment" may be used to mean both therapeutic and prophylactic treatment. Then, prevention may be used to mean that a pathological condition or disease of a subject is alleviated or mitigated. In one embodiment, the term "treatment" includes both application or any form of administration for treating a disease in a mammal, including a human. In addition, the term includes inhibiting or slowing down the progression of a disease; and includes meanings of restoring or repairing impaired or lost function so that a disease is partially or completely alleviated; stimulating inefficient processes; or alleviating a serious disease.

As used herein, the term "efficacy" can be determined by one or more parameters such as survival over a certain period of time such as 1 year, 5 years, or 10 years, or disease-free survival. Further, the parameter may comprise suppression of the size of at least one tumor in an individual.

Pharmacokinetic parameters such as bioavailability and underlying parameters such as clearance rate may also affect efficacy. Therefore, "enhanced efficacy" (for example, improvement in efficacy) may be due to enhanced pharmacokinetic parameters and enhanced efficacy, which may be measured by comparing parameters such as clearance rate and treatment or improvement of tumor in test animals or human subjects.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause side effects. A level of the effective amount may be determined depending on factors including the patient's health condition, type and severity of disease, activity of drug, the patient's sensitivity to drug, mode of administration, time of administration, route of administration and excretion rate, duration of treatment, formulation or simultaneously used drugs, and other factors well known in the medical field. In one embodiment, the therapeutically effective amount refers to an amount of drug effective to treat cancer.

Then, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffering agent, dispersing agent) may also be contained in the pharmaceutical composition.

Specifically, by including a pharmaceutically acceptable carrier in addition to the active ingredient, the pharmaceutical composition may be prepared into a parenteral formulation depending on route of administration using conventional methods known in the art. Then, the term "pharmaceutically acceptable" means that the carrier does not have more toxicity than the subject to be applied (prescribed) may adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may be preferably used. Formulation of pharmaceutical compositions is known in the art, and reference may specifically be made to Remington's Pharmaceutical Sciences (19th ed., 1995) and the like. This document is considered part of the present specification.

A preferred dose of the pharmaceutical composition may range from 0.01 µg/kg to 10 g/kg, or 0.01 mg/kg to 1 g/kg, per day, depending on the patient's condition, body weight, sex, age, severity of the patient, and route of administration. The dose may be administered once a day or may be divided into several times a day. Such a dose should not be construed as limiting the scope of the present invention in any aspect.

Subjects to which the pharmaceutical composition may be applied (prescribed) are mammals and humans, with humans being particularly preferred. In addition to the active ingredient, the pharmaceutical composition of the present application may further contain any compound or natural extract, which is known to have a therapeutic effect on tumor.

### Combination treatment method comprising fusion protein and anticancer agent

In yet another aspect of the present invention, there is provided a method for treating or preventing cancer, comprising administering, to a subject, a fusion protein comprising a first monomer comprising IL-12 or a variant thereof; and a second monomer comprising an antigen binding site that specifically binds to FAP or a dimer thereof; and an anticancer agent.

Then, the subject may be a subject suffering from cancer. In addition, the subject may be a mammal, preferably a human. The fusion protein or dimer thereof, an anticancer agnet are as described above.

Route of administration, dose, and frequency of administration of the fusion protein or fusion protein dimer may vary depending on the patient's condition and the presence or absence of side effects, and thus the fusion protein or fusion protein dimer may be administered to a subject in various ways and amounts. The optimal administration method, dose, and frequency of administration may be selected in an appropriate range by those skilled in the art. In addition, the fusion protein or fusion protein dimer may be administered in combination with other drugs or physiologically active substances whose therapeutic effect is known with respect to a disease to be treated(for example, anticancer agent), or may be formulated in the form of combination preparations with other drugs.

Hereinafter, the present invention will be described in more detail by way of the following examples. However, the following examples are only for illustrating the present invention, and the scope of the present invention is not limited thereto.

### Preparation Example 1. Overview of anti-FAP/IL-12 IgG1 DANG, human fusion protein

**[Table 1]**

| PROTEIN | Format | Description | Corresponding sequence |
|---|---|---|---|
| T1.01 | K&H | anti-hu FAP/hu IL-12, hu IgG1 DANG | seq1, seq2, seq3 |
| T1.02 | K&H | anti-hu FAP/hu IL-12 mut1, hu IgG1 DANG | seq1, seq2, seq4 |
| T1.03 | K&H | anti-hu FAP/hu IL-12 mut2, hu IgG1 DANG | seq1, seq2, seq5 |
| T1.04 | K&H | anti-hu FAP/hu IL-12, hu IgG1 DANG (2+1) | seq3, seq6, seq7 |
| T1.05 | K&H | anti-hu FAP/hu IL-12 mut2, hu IgG1 DANG (2+1) | seq5, seq6, seq7 |
| T1.06 | K&H | anti-hu FAP/hu IL-12, hu IgG1 DANG (2+1, HC scFv) | seq2, seq3, seq8 |
| T1.07 | K&H | anti-hu FAP/hu IL-12 mut2, hu IgG1 DANG (2+1, HC scFv) | seq2, seq5, seq8 |
| T1.08 | K&H | anti-hu FAP/hu IL-12, hu IgG1 DANG (2+1, LC scFv) | seq1, seq3, seq9 |
| T1.09 | K&H | anti-hu FAP/hu IL-12 mut2, hu IgG1 DANG (2+1, LC scFv) | seq1, seq5, seq9 |
| T1.10 | K&H | anti-hu FAP/anti-hu FAP-hu IL-12, hu IgG1 DANG (2+1) | seq1, seq2, seq10 |
| T1.11 | K&H | anti-hu FAP/anti-hu FAP-hu IL-12 mut2, hu IgG1 DANG (2+1) | seq1, seq2, seq11 |
| T1.12 | K&H | anti-hu FAP/null, hu IgG1 DANG | seq1, seq2, seq12 |
| T1.13 | K&H | null/hu IL-12, hu IgG1 DANG | seq3, seq13 |
| T1.14 | K&H | null/hu IL-12 mut1, hu IgG1 DANG | seq4, seq13 |
| T1.15 | K&H | null/hu IL-12 mut2, hu IgG1 DANG | seq5, seq 13 |
| T1.16 | Mab | anti-hu FAP, hu IgG1 DANG | seq2, seq14 |
| T1.17 | Fc-fusion | hu IL-12 mut2, hu IgG1 DANG | seq 15 |
| T1.18 | K&H | anti-hu CD20/hu IL-12, hu IgG1 DANG | seq3, seq16, seq17 |
| T1.19 | K&H | anti-hu CD20/hu IL-12 mut1, hu IgG1 DANG | seq4, seq16, seq17 |
| T1.20 | K&H | anti-hu CD20/hu IL-12 mut2, hu IgG1 DANG | seq5, seq16, seq17 |
| T1.21 | K&H | anti-hu CD20/null, hu IgG1 DANG | seq12, seq16, seq17 |
| T1.23 | K&H | anti-hu FAP/hu IL-12-anti-hu FAP scFv, hu IgG1 DANG (2+1) | seq1, seq2, seq292 |
| T1.24 | K&H | anti-hu FAP/hu IL-12 mut2-anti-hu FAP scFv, hu IgG1 DANG (2+1) | seq1, seq2, seq293 |
| T1.25 | Fc-fusion | anti-hu FAP-hu IL-12, hu IgG1 DANG | seq2, seq294 |
| T1.26 | Fc-fusion | anti-hu FAP-hu IL-12 mut2, hu IgG1 DANG | seq2, seq295 |
| T1.27 | Fc-fusion | hu IL-12-anti-hu FAP scFv, hu IgG1 DANG | Seq296 |
| T1.28 | Fc-fusion | hu IL-12 mut2-anti-hu FAP scFv, hu IgG1 DANG | Seq297 |

[seq1] is composed of a human anti-FAP heavy chain variable region sequence and a human IgG1 Fc in which an effector function is removed through DANG mutations (D265A, N297G), and a knob structure is formed by a T366W mutation.

[seq2] is composed of a human anti-FAP light chain sequence.

[seq3] is composed of a human IL-12 p40 (beta) region sequence, and a linker GGGGSGGGGSGGGGS, a human interleukin 12 p35 (alpha) region sequence, a linker GGGGSGGGGS, and a human IgG1 Fc in which an effector function is removed through DANG mutations (D265A, N297G), and a hole structure is formed by T366S, L368A and Y407V mutations.

[seq4] is composed of a sequence in which lysine (K) at the 280^{th} and 285^{th} amino acids involved in heparin binding in a human IL-12 p40 (beta) region is mutated to alanine (A), a linker GGGGSGGGGSGGGGS, a human IL-12 p35 (alpha) region sequence, a linker GGGGSGGGGS, and a human IgG1 Fc hole DANG.

[seq5] is composed of a sequence in which lysine (K) at the 280^{th}, 282^{nd}, 285^{th} and 286^{th} amino acids involved in heparin binding in a human IL-12 p40 (beta) region is mutated to alanine (A), a linker GGGGSGGGGSGGGGS, a human IL-12 p35 (alpha) region sequence, a linker GGGGSGGGGS, and a human IgG1 Fc hole DANG.

[seq6] is composed of a human anti-FAP heavy chain variable region sequence, a linker GGGGSGGGGSGGGGS, a human anti-FAP heavy chain variable region sequence, and a human IgG1 Fc knob DANG.

[seq7] is composed of a human anti-FAP light chain variable region sequence, a linker GGGGSGGGGSGGGGS, and a human anti-FAP light chain sequence.

[seq8] is composed of a human anti-FAP heavy chain variable region sequence, a human IgG1 Fc knob DANG, a linker GGGGSGGGGSGGGGS, a human anti-FAP heavy chain variable region sequence, a linker GGGGSGGGGSGGGGSGGGGS, and a human anti-FAP light chain variable region sequence.

[seq9] is composed of a human anti-FAP light chain sequence, a linker GGGGSGGGGSGGGGS, a human anti-FAP heavy chain variable region sequence, a linker GGGGSGGGGSGGGGSGGGGS, and a human anti-FAP light chain variable region sequence.

[seq10] is composed of a human anti-FAP heavy chain sequence, a human IgG1 Fc hole DANG, a linker GGGGSGGGGSGGGGS, a p40 (beta) region sequence of human IL-12, a linker GGGGSGGGGSGGGGS, and a human IL-12 p35 (alpha) region sequence.

[seq11] is composed of a human anti-FAP heavy chain sequence, a human IgG1 Fc hole DANG, a linker GGGGSGGGGSGGGGS, a p40 (beta) region sequence of human IL-12 containing mutations (four) of amino acids involved in heparin binding, a linker GGGGSGGGGSGGGGS, and a human IL-12 p35 (alpha) region sequence.

[seq12] is composed of only a human IgG1 Fc hole DANG.

[seq13] is composed of only a human IgG1 Fc knob DANG.

[seq14] is composed of a human anti-FAP heavy chain variable region sequence and a human IgG1 Fc DANG.

[seq15] is composed of a p40 (beta) region sequence of human IL-12 containing mutations (four) of amino acids involved in heparin binding, a linker GGGGSGGGGSGGGGS, a human IL-12 p35 (alpha) region sequence, a linker GGGGSGGGGS, and a human IgG1 Fc DANG.

[seq16] is composed of a heavy chain sequence of a human anti-CD20 antibody and a human IgG1 Fc knob DANG.

[seq17] is a light chain sequence of a human anti-CD20 antibody.

[seq292] is composed of a human IL-12 p40 (beta) region sequence, a linker GGGGSGGGGSGGGGS, a human IL-12 p35 (alpha) region sequence, a linker GGGGSGGGGS and a human IgG1 Fc hole DANG, a linker GGGGSGGGGSGGGGS, a human anti-FAP heavy chain variable region sequence, a linker GGGGSGGGGSGGGGSGGGGS and a human anti-FAP light chain variable region sequence.

[seq293] is composed of a human IL-12 p40 (beta) region sequence containing mutations (four) of amino acids involved in heparin binding, a linker GGGGSGGGGSGGGGS, a human IL-12 p35 (alpha) region sequence, a linker GGGGSGGGGS and a human IgG1 Fc hole DANG, a linker GGGGSGGGGSGGGGS, a human anti-FAP heavy chain variable region sequence, a linker GGGGSGGGGSGGGGSGGGGS, and a human anti-FAP light chain variable region sequence.

[seq294] is composed of a human anti-FAP heavy chain sequence, a human IgG1 Fc DANG, a linker GGGGSGGGGSGGGGS, a p40 (beta) region sequence of human IL-12, a linker GGGGSGGGGSGGGGS, and a human IL-12 p35 (alpha) region sequence.

[seq295] is composed of a human anti-FAP heavy chain sequence, a human IgG1 Fc DANG, a linker GGGGSGGGGSGGGGS, a p40 (beta) region sequence of human IL-12 containing mutations (four) of amino acids involved in heparin binding, a linker GGGGSGGGGSGGGGS, and a human IL-12 p35 (alpha) region sequence.

[seq296] is composed of a human IL-12 p40 (beta) region sequence, a linker GGGGSGGGGSGGGGS, a human IL-12 p35 (alpha) region sequence, a linker GGGGSGGGGS and a human IgG1 Fc DANG, a linker GGGGSGGGGSGGGGS, a human anti-FAP heavy chain variable region sequence, a linker GGGGSGGGGSGGGGSGGGGS, and a human anti-FAP light chain variable region sequence.

[seq297] is composed of a human IL-12 p40 (beta) region sequence containing mutations (four) of amino acids involved in heparin binding, a linker GGGGSGGGGSGGGGS, a human IL-12 p35 (alpha) region sequence, a linker GGGGSGGGGS and a human IgG1 Fc DANG, a linker GGGGSGGGGSGGGGS, a human anti-FAP heavy chain variable region sequence, a linker GGGGSGGGGSGGGGSGGGGS, and a human anti-FAP light chain variable region sequence.

T1.01 (seq1, seq2, seq3) is a bispecific antibody in which a human anti-FAP sequence targeting human FAP and a human IL-12 sequence form a knob-in-hole structure, and an effector function is removed.

T1.02 (seq1, seq2, seq4) is a bispecific antibody in which a human anti-FAP sequence targeting human FAP and a human IL-12 sequence containing mutations (two) of amino acids involved in heparin binding form a knob-in-hole structure, and an effector function is removed.

T1.03 (seq1, seq2, seq5) is a bispecific antibody in which a human anti-FAP sequence targeting human FAP and a human IL-12 sequence containing mutations (four) of amino acids involved in heparin binding form a knob-in-hole structure, and an effector function is removed.

T1.04 (seq3, seq6, seq7) is a bispecific antibody in which a human anti-FAP sequence in a dual variable domain immunoglobulin (DVD-Ig) form and a human IL-12 sequence form a knob-in-hole structure, and an effector function is removed.

T1.05 (seq5, seq6, seq7) is a bispecific antibody in which a human anti-FAP sequence in a DVD-Ig form and a human IL-12 sequence containing mutations (four) of amino acids involved in heparin binding form a knob-in-hole structure, and an effector function is removed.

T1.06 (seq2, seq3, seq8) is a bispecific antibody in which a human anti-FAP sequence in which a single chain variable fragment (scFv of a human anti-FAP sequence targeting human FAP is linked to the C-terminus of the heavy chain and a human IL-12 sequence form a knob-in-hole structure, and an effector function is removed.

T1.07 (seq2, seq5, seq8) is a bispecific antibody in which a human anti-FAP sequence in which a scFv of a human anti-FAP sequence targeting human FAP is linked to the C-terminus of the heavy chain and a human IL-12 sequence containing mutations (four) of amino acids involved in heparin binding form a knob-in-hole structure, and an effector function is removed.

T1.08 (seq1, seq3, seq9) is a bispecific antibody in which a human anti-FAP sequence in which a scFv of a human anti-FAP sequence targeting human FAP is linked to the C-terminus of the light chain and a human IL-12 sequence form a knob-in-hole structure, and an effector function is removed.

T1.09 (seq1, seq5, seq9) is a bispecific antibody in which a human anti-FAP sequence in which a scFv of a human anti-FAP sequence targeting human FAP is linked to the C-terminus of the light chain and a human IL-12 sequence containing mutations (four) of amino acids involved in heparin binding form a knob-in-hole structure, and an effector function is removed.

T1.10 (seq1, seq2, seq10) is an antibody in which a human anti-FAP sequence targeting human FAP and a human anti-FAP sequence in which a human IL-12 sequence is linked to the C-terminus of the heavy chain form a knob-in-hole structure.

T1.11 (seq1, seq2, seq11) is an antibody in which a human anti-FAP sequence targeting human FAP and a human anti-FAP sequence in which a human IL-12 sequence containing mutations (four) of amino acids involved in heparin binding is linked to the C-terminus of the heavy chain form a knob-in-hole structure.

T1.12 (seq1, seq2, seq12) is an antibody in which an effector function of a knob-in-hole structure having only a human anti-FAP sequence targeting human FAP is removed.

T1.13 (seq3, seq13) is an antibody in which an effector function of a knob-in-hole structure having only a human IL-12 sequence is removed.

T1.14 (seq4, seq13) is an antibody in which an effector function of a knob-in-hole structure having only a human IL-12 sequence containing mutations (two) of amino acids involved in heparin binding is removed.

T1.15 (seq5, seq13) is an antibody in which an effector function of a knob-in-hole structure having only a human IL-12 sequence containing mutations (four) of amino acids involved in heparin binding is removed.

T1.16 (seq2, seq14) is a human anti-FAP antibody targeting human FAP.

T1.17 (seq15) is an Fc-fusion protein having only a human IL-12 sequence containing mutations (four) of amino acids involved in heparin binding.

T1.18 (seq3, seq16, seq17) is a bispecific antibody in which a human anti-CD20 sequence targeting human CD20 and a human IL-12 sequence form a knob-in-hole structure, and an effector function is removed.

T1.19 (seq4, seq16, seq17) is a bispecific antibody in which a human anti-CD20 sequence targeting human CD20 and a human IL-12 sequence containing mutations (two) of amino acids involved in heparin binding form a knob-in-hole structure, and an effector function is removed.

T1.20 (seq5, seq16, seq17) is a bispecific antibody in which a human anti-CD20 sequence targeting human CD20 and a human IL-12 sequence containing mutations (four) of amino acids involved in heparin binding form a knob-in-hole structure, and an effector function is removed.

T1.21 (seq12, seq16, seq17) is an antibody in which an effector function of a knob-in-hole structure having only a human anti-CD20 sequence targeting human CD20 is removed.

T1.23 (seq1, seq2, seq292) is a bispecific antibody in which a human anti-FAP sequence and a human IL-12 sequence in which a scFv of a human anti-FAP sequence is linked to the C-terminus form a knob-in-hole structure, and an effector function is removed.

T1.24 (seq1, seq2, seq293) is a bispecific antibody in which a human anti-FAP heavy chain sequence and a human IL-12 sequence containing mutations (four) of amino acids involved in heparin binding in which a scFv of a human anti-FAP sequence is linked to the C-terminus form a knob-in-hole structure, and an effector function is removed.

T1.25 (seq2, seq294) is an Fc-fusion protein dimer containing a human anti-FAP sequence in which a human IL-12 sequence is linked to the C-terminus of the heavy chain.

T1.26 (seq2, seq295) is an Fc-fusion protein dimer containing a human anti-FAP sequence in which a human IL-12 sequence containing mutations (four) of amino acids involved in heparin binding is linked to the C-terminus of the heavy chain.

T1.27 (seq296) is an Fc-fusion protein dimer consisting of a human IL-12 sequence in which a single chain fragment variable (scFv) of a human anti-FAP sequence is linked to the C-terminus.

T1.28 (seq297) is an Fc-fusion protein dimer composed of a human IL-12 sequence containing mutations (four) of amino acids involved in heparin binding in which a scFv of a human anti-FAP sequence is linked to the C-terminus.
[seq1] anti-hu FAP HC hu IgG1 Fc knob DANG QVQLVQSGAEVKKPGASVKVSCKTSRYTFTEYTIHWVRQAPGQRLEWIGGINP 
NNGIPNYNQKFKGRVTITVDTSASTAYMELSSLRSEDTAVYYCARRRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLWCLVKGFYPSDIAVEWESNGQPENNYKTTPPVLD
SDGSFFLYSKLTVDKSRWQQGNVFSCSVMHEALHNHYTQKSLSLSPGK
[seq2] anti-hu FAP LC 
DIVMTQSPDSLAVSLGERATINCKSSQSLLYSRNQKNYLAWYQQKPGQPPKLLI
FWASTRESGVPDRFSGSGFGTDFTLTISSLQAEDVAVYYCQQYFSYPLTFGQGTKVEIKR
TVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
[seq3] hu scIL-12-hu IgG1 Fc hole DANG 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGKSKREKKDRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLSCAVKGFYPSDIAVEWESNGQPENNYKTTPP
VLDSDGSFFLVSKLTVDKSRWQQGNVFSCSVMHEALHNHYTQKSLSLSPGK
[seq4] hu scIL-12 mut1-hu IgG1 Fc hole DANG 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGASKREAKDRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLSCAVKGFYPSDIAVEWESNGQPENNYKTTPP
 VLDSDGSFFLVSKLTVDKSRWQQGNVFSCSVMHEALHNHYTQKSLSLSPGK
[seq5] hu scIL-12 mut2-hu IgG1 Fc hole DANG 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGASAREAADRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLSCAVKGFYPSDIAVEWESNGQPENNYKTTPP
VLDSDGSFFLVSKLTVDKSRWQQGNVFSCSVMHEALHNHYTQKSLSLSPGK
[seq6] (anti-hu FAP VH)2-hu IgG1 Fc knob DANG 
QVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRRIAYGYDEGHAM
DYWGQGTLVTVSSGGGGSGGGGSGGGGSQVQLVQSGAEVKKPGASVKVSCKTSRYTF
TEYTIHWVRQAPGQRLEWIGGINPNNGIPNYNQKFKGRVTITVDTSASTAYMELSSLRSE
DTAVYYCARRRIAYGYDEGHAMDYWGQGTLVTVSSASTKGPSVFPLAPSSKSTSGGTA
ALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVT
CVVVAVSHEDPEVKFNWYVDGVEVHNAKTKPREEQYGSTYRVVSVLTVLHQDWLNG
KEYKCKVSNKALPAPIEKTISKAKGQPREPQVYTLPPSRDELTKNQVSLWCLVKGFYPS
DIAVEWESNGQPENNYKTTPPVLDSDGSFFLYSKLTVDKSRWQQGNVFSCSVMHEALH
NHYTQKSLSLSPGK
[seq7] (anti-hu FAP VL)2 
DIVMTQSPDSLAVSLGERATINCKSSQSLLYSRNQKNYLAWYQQKPGQPPKLLI
FWASTRESGVPDRFSGSGFGTDFTLTISSLQAEDVAVYYCQQYFSYPLTFGQGTKVEIKG
GGGSGGGGSGGGGSDIVMTQSPDSLAVSLGERATINCKSSQSLLYSRNQKNYLAWYQQ
KPGQPPKLLIFWASTRESGVPDRFSGSGFGTDFTLTISSLQAEDVAVYYCQQYFSYPLTF
GQGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC
[seq8] anti-hu FAP HC hu IgG1 Fc knob DANG-anti-hu FAP scFv 
QVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
 
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLWCLVKGFYPSDIAVEWESNGQPENNYKTTPPVLD
SDGSFFLYSKLTVDKSRWQQGNVFSCSVMHEALHNHYTQKSLSLSPGKGGGGSGGGGS
GGGGSQVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRRIAYGYDEGHAM
DYWGQGTLVTVSSGGGGSGGGGSGGGGSGGGGSDIVMTQSPDSLAVSLGERATINCKS
SQSLLYSRNQKNYLAWYQQKPGQPPKLLIFWASTRESGVPDRFSGSGFGTDFTLTISSLQ
AEDVAVYYCQQYFSYPLTFGQGTKVEIK
[seq9] anti-hu FAP LC-anti-hu FAP scFv 
DIVMTQSPDSLAVSLGERATINCKSSQSLLYSRNQKNYLAWYQQKPGQPPKLLI
FWASTRESGVPDRFSGSGFGTDFTLTISSLQAEDVAVYYCQQYFSYPLTFGQGTKVEIKR
TVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGECGGGGSGGGGSGG
GGSQVQLVQSGAEVKKPGASVKVSCKTSRYTFTEYTIHWVRQAPGQRLEWIGGINPNN
GIPNYNQKFKGRVTITVDTSASTAYMELSSLRSEDTAVYYCARRRIAYGYDEGHAMDY
WGQGTLVTVSSGGGGSGGGGSGGGGSGGGGSDIVMTQSPDSLAVSLGERATINCKSSQ
SLLYSRNQKNYLAWYQQKPGQPPKLLIFWASTRESGVPDRFSGSGFGTDFTLTISSLQAE
DVAVYYCQQYFSYPLTFGQGTKVEIK
[seq10] anti-hu FAP HC hu IgG1 Fc hole DANG-hu scIL-12 
QVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLSCAVKGFYPSDIAVEWESNGQPENNYKTTPPVLD
SDGSFFLVSKLTVDKSRWQQGNVFSCSVMHEALHNHYTQKSLSLSPGGGGGSGGGGSG
GGGSIWELKKDVYVVELDWYPDAPGEMVVLTCDTPEEDGITWTLDQSSEVLGSGKTLT
IQVKEFGDAGQYTCHKGGEVLSHSLLLLHKKEDGIWSTDILKDQKEPKNKTFLRCEAKN
YSGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVEVS
WEYPDTWSTPHSYFSLTFCVQVQGKSKREKKDRVFTDKTSATVICRKNASISVRAQDRY
YSSSWSEWASVPCSGGGGSGGGGSGGGGSRNLPVATPDPGMFPCLHHSQNLLRAVSNM
LQKARQTLEFYPCTSEEIDHEDITKDKTSTVEACLPLELTKNESCLNSRETSFITNGSCLAS
RKTSFMMALCLSSIYEDLKMYQVEFKTMNAKLLMDPKRQIFLDQNMLAVIDELMQAL
NFNSETVPQKSSLEEPDFYKTKIKLCILLHAFRIRAVTIDRVMSYLNAS
[seq11] anti-hu FAP HC hu IgG1 Fc hole DANG-hu scIL-12 mut2 QVQLVQSGAEVKKPGASVKVSCKTSRYTFTEYTIHWVRQAPGQRLEWIGGINP 
NNGIPNYNQKFKGRVTITVDTSASTAYMELSSLRSEDTAVYYCARRRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLSCAVKGFYPSDIAVEWESNGQPENNYKTTPPVLD
SDGSFFLVSKLTVDKSRWQQGNVFSCSVMHEALHNHYTQKSLSLSPGGGGGSGGGGSG
GGGSIWELKKDVYVVELDWYPDAPGEMVVLTCDTPEEDGITWTLDQSSEVLGSGKTLT
IQVKEFGDAGQYTCHKGGEVLSHSLLLLHKKEDGIWSTDILKDQKEPKNKTFLRCEAKN
YSGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVEVS
WEYPDTWSTPHSYFSLTFCVQVQGASAREAADRVFTDKTSATVICRKNASISVRAQDRY
YSSSWSEWASVPCSGGGGSGGGGSGGGGSRNLPVATPDPGMFPCLHHSQNLLRAVSNM
LQKARQTLEFYPCTSEEIDHEDITKDKTSTVEACLPLELTKNESCLNSRETSFITNGSCLAS
RKTSFMMALCLSSIYEDLKMYQVEFKTMNAKLLMDPKRQIFLDQNMLAVIDELMQAL
NFNSETVPQKSSLEEPDFYKTKIKLCILLHAFRIRAVTIDRVMSYLNAS
[seq12] hu IgG1 Fc hole DANG 
DKTHTCPPCPAPELLGGPSVFLFPPKPKDTLMISRTPEVTCVVVAVSHEDPEVKF
NWYVDGVEVHNAKTKPREEQYGSTYRVVSVLTVLHQDWLNGKEYKCKVSNKALPAPI
EKTISKAKGQPREPQVYTLPPSRDELTKNQVSLSCAVKGFYPSDIAVEWESNGQPENNY
KTTPPVLDSDGSFFLVSKLTVDKSRWQQGNVFSCSVMHEALHNHYTQKSLSLSPGK
[seq13] hu IgG1 Fc knob DANG 
DKTHTCPPCPAPELLGGPSVFLFPPKPKDTLMISRTPEVTCVVVAVSHEDPEVKF
NWYVDGVEVHNAKTKPREEQYGSTYRVVSVLTVLHQDWLNGKEYKCKVSNKALPAPI
EKTISKAKGQPREPQVYTLPPSRDELTKNQVSLWCLVKGFYPSDIAVEWESNGQPENNY
KTTPPVLDSDGSFFLYSKLTVDKSRWQQGNVFSCSVMHEALHNHYTQKSLSLSPGK
[seq14] anti-hu FAP HC hu IgG1 Fc DANG 
QVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
[seq15] hu scIL-12 mut2-hu IgG1 Fc DANG 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
 
QEDSACPAAEESLPIEVMVDAVHKLKYENYTSSFFIRDIIKPDPPKNLQLKPLKNSRQVE
VSWEYPDTWSTPHSYFSLTFCVQVQGASAREAADRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK
[seq16] anti-hu CD20 HC hu IgG1 Fc knob DANG 
QVQLQQPGAELVKPGASVKMSCKASGYTFTSYNMHWVKQTPGRGLEWIGAIY
PGNGDTSYNQKFKGKATLTADKSSSTAYMQLSSLTSEDSAVYYCARSTYYGGDWYFN
VWGAGTTVTVSAASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVAVSHEDPEVKFNWYVDGVE
VHNAKTKPREEQYGSTYRVVSVLTVLHQDWLNGKEYKCKVSNKALPAPIEKTISKAKG
QPREPQVYTLPPSRDELTKNQVSLWCLVKGFYPSDIAVEWESNGQPENNYKTTPPVLDS
DGSFFLYSKLTVDKSRWQQGNVFSCSVMHEALHNHYTQKSLSLSPGK
[seq17] anti-hu CD20 LC 
QIVLSQSPAILSASPGEKVTMTCRASSSVSYIHWFQQKPGSSPKPWIYATSNLASG
VPVRFSGSGSGTSYSLTISRVEAEDAATYYCQQWTSNPPTFGGGTKLEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
[seq292] hu scIL-12-hu IgG1 Fc hole DANG-anti-hu FAP scFv 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGKSKREKKDRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLSCAVKGFYPSDIAVEWESNGQPENNYKTTPP
VLDSDGSFFLVSKLTVDKSRWQQGNVFSCSVMHEALHNHYTQKSLSLSPGGGGGSGGG
GSGGGGSQVQLVQSGAEVKKPGASVKVSCKTSRYTFTEYTIHWVRQAPGQRLEWIGGI
 
NPNNGIPNYNQKFKGRVTITVDTSASTAYMELSSLRSEDTAVYYCARRRIAYGYDEGHA
MDYWGQGTLVTVSSGGGGSGGGGSGGGGSGGGGSDIVMTQSPDSLAVSLGERATINC
KSSQSLLYSRNQKNYLAWYQQKPGQPPKLLIFWASTRESGVPDRFSGSGFGTDFTLTISS
LQAEDVAVYYCQQYFSYPLTFGQGTKVEIK
[seq293] hu scIL-12 mut2-hu IgG1 Fc hole DANG-anti-hu FAP scFv 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGASAREAADRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLSCAVKGFYPSDIAVEWESNGQPENNYKTTPP
VLDSDGSFFLVSKLTVDKSRWQQGNVFSCSVMHEALHNHYTQKSLSLSPGGGGGSGGG
GSGGGGSQVQLVQSGAEVKKPGASVKVSCKTSRYTFTEYTIHWVRQAPGQRLEWIGGI
NPNNGIPNYNQKFKGRVTITVDTSASTAYMELSSLRSEDTAVYYCARRRIAYGYDEGHA
MDYWGQGTLVTVSSGGGGSGGGGSGGGGSGGGGSDIVMTQSPDSLAVSLGERATINC
KSSQSLLYSRNQKNYLAWYQQKPGQPPKLLIFWASTRESGVPDRFSGSGFGTDFTLTISS
LQAEDVAVYYCQQYFSYPLTFGQGTKVEIK
[seq294] anti-hu FAP HC hu IgG1 Fc DANG-hu scIL-12 
QVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGGGGGSGGGGSG
GGGSIWELKKDVYVVELDWYPDAPGEMVVLTCDTPEEDGITWTLDQSSEVLGSGKTLT
IQVKEFGDAGQYTCHKGGEVLSHSLLLLHKKEDGIWSTDILKDQKEPKNKTFLRCEAKN
YSGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVEVS
WEYPDTWSTPHSYFSLTFCVQVQGKSKREKKDRVFTDKTSATVICRKNASISVRAQDRY
YSSSWSEWASVPCSGGGGSGGGGSGGGGSRNLPVATPDPGMFPCLHHSQNLLRAVSNM
LQKARQTLEFYPCTSEEIDHEDITKDKTSTVEACLPLELTKNESCLNSRETSFITNGSCLAS
 
RKTSFMMALCLSSIYEDLKMYQVEFKTMNAKLLMDPKRQIFLDQNMLAVIDELMQAL
NFNSETVPQKSSLEEPDFYKTKIKLCILLHAFRIRAVTIDRVMSYLNAS
[seq295] anti-hu FAP HC hu IgG1 Fc DANG-hu scIL-12 mut2 
QVQLVQSGAEVKKPGASVKVSCKTSRYTFTEYTIHWVRQAPGQRLEWIGGINP
NNGIPNYNQKFKGRVTITVDTSASTAYMELSSLRSEDTAVYYCARRRIAYGYDEGHAM
DYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVAVSHEDPEVKFNWYVDGV
EVHNAKTKPREEQYGSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGGGGGSGGGGSG
GGGSIWELKKDVYVVELDWYPDAPGEMVVLTCDTPEEDGITWTLDQSSEVLGSGKTLT
IQVKEFGDAGQYTCHKGGEVLSHSLLLLHKKEDGIWSTDILKDQKEPKNKTFLRCEAKN
YSGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVEVS
WEYPDTWSTPHSYFSLTFCVQVQGASAREAADRVFTDKTSATVICRKNASISVRAQDRY
YSSSWSEWASVPCSGGGGSGGGGSGGGGSRNLPVATPDPGMFPCLHHSQNLLRAVSNM
LQKARQTLEFYPCTSEEIDHEDITKDKTSTVEACLPLELTKNESCLNSRETSFITNGSCLAS
RKTSFMMALCLSSIYEDLKMYQVEFKTMNAKLLMDPKRQIFLDQNMLAVIDELMQAL
NFNSETVPQKSSLEEPDFYKTKIKLCILLHAFRIRAVTIDRVMSYLNAS
[seq296] hu scIL-12-hu IgG1 Fc DANG-anti-hu FAP scFv 
IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGKSKREKKDRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGGGGGSGGG
GSGGGGSQVQLVQSGAEVKKPGASVKVSCKTSRYTFTEYTIHWVRQAPGQRLEWIGGI
NPNNGIPNYNQKFKGRVTITVDTSASTAYMELSSLRSEDTAVYYCARRRIAYGYDEGHA
MDYWGQGTLVTVSSGGGGSGGGGSGGGGSGGGGSDIVMTQSPDSLAVSLGERATINC
KSSQSLLYSRNQKNYLAWYQQKPGQPPKLLIFWASTRESGVPDRFSGSGFGTDFTLTISS
LQAEDVAVYYCQQYFSYPLTFGQGTKVEIK
[seq297] hu scIL-12 mut2-hu IgG1 Fc DANG-anti-hu FAP scFv

IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVEC
QEDSACPAAEESLPIEVMVDAVHKLKYENYTSSFFIRDIIKPDPPKNLQLKPLKNSRQVE
VSWEYPDTWSTPHSYFSLTFCVQVQGASAREAADRVFTDKTSATVICRKNASISVRAQD
RYYSSSWSEWASVPCSGGGGSGGGGSGGGGSRNLPVATPDPGMFPCLHHSQNLLRAVS
NMLQKARQTLEFYPCTSEEIDHEDITKDKTSTVEACLPLELTKNESCLNSRETSFITNGSC
LASRKTSFMMALCLSSIYEDLKMYQVEFKTMNAKLLMDPKRQIFLDQNMLAVIDELMQ
ALNFNSETVPQKSSLEEPDFYKTKIKLCILLHAFRIRAVTIDRVMSYLNASGGGGSGGGG
SDKTHTCPPCPAPELLGGPSVFLFPPKPKDTLMISRTPEVTCVVVAVSHEDPEVKFNWYV
DGVEVHNAKTKPREEQYGSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGGGGGSGGG
GSGGGGSQVQLVQSGAEVKKPGASVKVSCKTSRYTFTEYTIHWVRQAPGQRLEWIGGI
NPNNGIPNYNQKFKGRVTITVDTSASTAYMELSSLRSEDTAVYYCARRRIAYGYDEGHA
MDYWGQGTLVTVSSGGGGSGGGGSGGGGSGGGGSDIVMTQSPDSLAVSLGERATINC
KSSQSLLYSRNQKNYLAWYQQKPGQPPKLLIFWASTRESGVPDRFSGSGFGTDFTLTISS
LQAEDVAVYYCQQYFSYPLTFGQGTKVEIK

### Preparation Example 2. Overview of anti-FAP/IL-12 IgG2a DANG, mouse fusion protein

**[Table 2]**

| Code | Format | Description | Corresponding sequence |
|---|---|---|---|
| T1.01m | K&H | anti-mu FAP/mu IL-12, mu IgG2a DANG | seq18, seq19, seq20 |
| T1.02m | K&H | anti-mu FAP/mu IL-12 mut1, mu IgG2a DANG | seq18, seq19, seq21 |
| T1.03m | K&H | anti-mu FAP/mu IL-12 mut2, mu IgG2a DANG | seq18, seq19, seq22 |
| T1.04m | K&H | anti-mu FAP/mu IL-12, mu IgG2a DANG (2+1) | seq20, seq23, seq24 |
| T1.05m | K&H | anti-mu FAP/mu IL-12 mut2, mu IgG2a DANG (2+1) | seq22, seq23, seq24 |
| T1.06m | K&H | anti-mu FAP/mu IL-12, mu IgG2a DANG (2+1, HC scFv) | seq19, seq20, seq25 |
| T1.07m | K&H | anti-mu FAP/mu IL-12 mut2, mu IgG2a DANG (2+1, HC scFv) | seq19, seq22, seq25 |
| T1.08m | K&H | anti-mu FAP/mu IL-12, mu IgG2a DANG (2+1, LC scFv) | seq18, seq20, seq26 |
| T1.09m | K&H | anti-mu FAP/mu IL-12 mut2, mu IgG2a DANG (2+1, LC scFv) | seq18, seq22, seq26 |
| T 1. 10m | K&H | anti-mu FAP/anti-mu FAP-mu IL-12, mu IgG2a DANG (2+1) | seq18, seq19, seq27 |
| T1.11m | K&H | anti-mu FAP/anti-mu FAP-mu IL-12 mut2, mu IgG2a DANG (2+1) | seq18, seq19, seq28 |
| T1.12m | K&H | anti-mu FAP/null, mu IgG2a DANG | seq18, seq19, seq29 |
| T1.13m | K&H | null/mu IL-12, mu IgG2a DANG | seq20, seq30 |
| T1.14m | K&H | null/mu IL-12 mut1, mu IgG2a DANG | seq21, seq30 |
| T1.15m | K&H | null/mu IL-12 mut2, mu IgG2a DANG | seq22, seq30 |
| T1.16m | Mab | anti-mu FAP, mu IgG2a DANG | seq19, seq31 |
| T1.17m | Fc-fusion | mu IL-12 mut2, mu IgG2a DANG | seq32 |
| T1.18m | K&H | anti-mu CD20/mu IL-12, mu IgG2a DANG | seq20, seq33, seq34 |
| T1.19m | K&H | anti-mu CD20/mu IL-12 mut1, mu IgG2a DANG | seq21, seq33, seq34 |
| T1.20m | K&H | anti-mu CD20/mu IL-12 mut2, mu IgG2a DANG | seq22, seq33, seq34 |
| T1.21m | K&H | anti-mu CD20/null, mu IgG2a DANG | seq29, seq33, seq34 |
| T1.22m | Fc fusion | mu IgG2a Fc DANG | seq35 |
| T1.23m | K&H | anti-mu FAP/mu IL-12-anti-mu FAP scFv, mu IgG2a DANG (2+1) | seq18, seq19, seq298 |
| T1.24m | K&H | anti-mu FAP/mu IL-12 mut2-anti-mu FAP scFv, mu IgG2a DANG (2+1) | seq18, seq19, seq299 |
| T1.25m | Fc-fusion | anti-mu FAP-mu IL-12, mu IgG2a DANG | seq19, seq300 |
| T1.26m | Fc-fusion | anti-mu FAP-mu IL-12 mut2, mu IgG2a DANG | seq19, seq301 |
| T1.27m | Fc-fusion | mu IL-12-anti-mu FAP scFv, mu IgG2a DANG | Seq302 |
| T1.28m | Fc-fusion | mu IL-12 mut2-anti-mu FAP scFv, mu IgG2a DANG | Seq303 |

[seq18] is composed of a mouse anti-FAP heavy chain variable region sequence and a mouse IgG2a Fc in which an effector function is removed through DANG mutations (D265A, N297G), and a knob structure is formed by a T321W mutation.

[seq19] is composed of a mouse anti-FAP light chain sequence.

[seq20] is composed of a mouse IL-12 p40 (beta) region sequence, a linker GGGGSGGGGSGGGGS, a mouse IL-12 p35 (alpha) region sequence, a linker GGGGSGGGGS, and a mouse IgG2a Fc in which an effector function is removed through DANG mutations and a hole structure is formed by T321S, M323A, Y362V mutations.

[seq21] is composed of a sequence in which lysine (K) at the 277^{th} and 282^{nd} amino acids involved in heparin binding in a mouse IL-12 p40 (beta) region is mutated to alanine (A), a linker GGGGSGGGGSGGGGS, a mouse IL-12 p35 (alpha) region sequence, a linker GGGGSGGGGS, and a mouse IgG2a Fc hole DANG.

[seq22] is composed of a sequence in which arginine (R) at the 276^{th} amino acid and lysine (K) at the 277^{th}, 278^{th} and 282^{nd} amino acids involved in heparin binding in a mouse IL-12 p40 (beta) region is mutated to alanine (A), a linker GGGGSGGGGSGGGGS, a mouse IL-12 p35 (alpha) region sequence, a linker GGGGSGGGGS, and a mouse IgG2a Fc hole DANG.

[seq23] is composed of a mouse anti-FAP heavy chain variable region sequence, a linker GGGGSGGGGSGGGGS, a mouse anti-FAP heavy chain variable region sequence, and a mouse IgG2a Fc knob DANG.

[seq24] is composed of a mouse anti-FAP light chain variable region sequence, a linker GGGGSGGGGSGGGGS, and a mouse anti-FAP light chain sequence.

[seq25] is composed of a mouse anti-FAP heavy chain variable region sequence, a mouse IgG2a Fc knob DANG, a linker GGGGSGGGGSGGGGS, a mouse anti-FAP heavy chain variable region sequence, a linker GGGGSGGGGSGGGGSGGGGS, and a mouse anti-FAP light chain variable region sequence.

[seq26] is composed of a mouse anti-FAP light chain sequence, a linker GGGGSGGGGSGGGGS, a mouse anti-FAP heavy chain variable region sequence, a linker GGGGSGGGGSGGGGSGGGGS, and a mouse anti-FAP light chain variable region sequence.

[seq27] is composed of a mouse anti-FAP heavy chain sequence, a mouse IgG2a Fc hole DANG, a linker GGGGSGGGGSGGGGS, a p40 (beta) region sequence of mouse IL-12, a linker GGGGSGGGGSGGGGS, and a mouse IL-12 p35 (alpha) region sequence.

[seq28] is composed of a mouse anti-FAP heavy chain sequence, a mouse IgG2a Fc hole DANG, a linker GGGGSGGGGSGGGGS, a p40 (beta) region sequence of mouse IL-12 containing mutations (four) of amino acids involved in heparin binding, a linker GGGGSGGGGSGGGGS, and a mouse IL-12 p35 (alpha) region sequence.

[seq29] is composed of only a mouse IgG2a Fc hole DANG.

[seq30] is composed of only a mouse IgG2a Fc knob DANG.

[seq3 1] is composed of a mouse anti-FAP heavy chain variable region and a mouse IgG2a Fc DANG.

[seq32] is composed of a p40 (beta) region sequence of mouse IL-12 containing mutations (four) of amino acids involved in heparin binding, a linker GGGGSGGGGSGGGGS, a mouse IL-12 p35 (alpha) region sequence, a linker GGGGSGGGGS, and a mouse IgG2a Fc DANG.

[seq33] is composed of a heavy chain variable region sequence of a mouse anti-CD20 antibody 18B12 and a mouse IgG2a knob DANG.

[seq34] is composed of a light chain sequence of a mouse anti-CD20 antibody 18B12.

[seq35] is composed of only a mouse IgG2a Fc DANG.

[seq298] is composed of a mouse IL-12 p40 (beta) region sequence, a linker GGGGSGGGGSGGGGS, a mouse IL-12 p35 (alpha) region sequence, a linker GGGGSGGGGS and a mouse IgG2a Fc hole DANG, a linker GGGGSGGGGSGGGGS, a mouse anti-FAP heavy chain variable region sequence, a linker GGGGSGGGGSGGGGSGGGGS, and a mouse anti-FAP light chain variable region sequence.

[seq299] is composed of a mouse IL-12 p40 (beta) region sequence containing mutations (four) of amino acids involved in heparin binding, a linker GGGGSGGGGSGGGGS, a mouse IL-12 p35 (alpha) region sequence, a linker GGGGSGGGGS and a mouse IgG2a Fc hole DANG, a linker GGGGSGGGGSGGGGS, a mouse anti-FAP heavy chain variable region sequence, a linker GGGGSGGGGSGGGGSGGGGS, and a mouse anti-FAP light chain variable region sequence.

[seq300] is composed of a mouse anti-FAP heavy chain sequence, a mouse IgG2a Fc DANG, a linker GGGGSGGGGSGGGGS, a p40 (beta) region sequence of a mouse IL-12, a linker GGGGSGGGGSGGGGS, and a mouse IL-12 p35 (alpha) region sequence.

[seq301] is composed of a mouse anti-FAP heavy chain sequence, a mouse IgG1 Fc DANG, a linker GGGGSGGGGSGGGGS, a p40 (beta) region sequence of mouse IL-12 containing mutations (four) of amino acids involved in heparin binding, a linker GGGGSGGGGSGGGGS, and a mouse IL-12 p35 (alpha) region sequence.

[seq302] is composed of a mouse IL-12 p40 (beta) region sequence, a linker GGGGSGGGGSGGGGS, a mouse IL-12 p35 (alpha) region sequence, a linker GGGGSGGGGS and a mouse IgG2a Fc DANG, a linker GGGGSGGGGSGGGGS, a mouse anti-FAP heavy chain variable region sequence, a linker GGGGSGGGGSGGGGSGGGGS, and a mouse anti-FAP light chain variable region sequence.

[seq303] is composed of a mouse IL-12 p40 (beta) region sequence containing mutations (four) of amino acids involved in heparin binding, a linker GGGGSGGGGSGGGGS, a mouse IL-12 p35 (alpha) region sequence, a linker GGGGSGGGGS and a mouse IgG2a Fc DANG, a linker GGGGSGGGGSGGGGS, a mouse anti-FAP heavy chain variable region sequence, a linker GGGGSGGGGSGGGGSGGGGS, and a mouse anti-FAP light chain variable region sequence.

T1.01m (seq18, seq19, seq20) is a bispecific antibody in which a mouse anti-FAP sequence targeting mouse FAP and a mouse IL-12 sequence form a knob-in-hole structure, and an effector function is removed.

T1.02m (seq18, seq19, seq21) is a bispecific antibody in which a mouse anti-FAP sequence targeting mouse FAP and a mouse IL-12 sequence containing mutations (two) of amino acids involved in heparin binding form a knob-in-hole structure, and an effector function is removed.

T1.03m (seq18, seq19, seq22) is a bispecific antibody in which a mouse anti-FAP sequence targeting mouse FAP and a mouse IL-12 sequence containing mutations (four) of amino acids involved in heparin binding form a knob-in-hole structure, and an effector function is removed.

T1.04m (seq20, seq23, seq24) is a bispecific antibody in which a mouse anti-FAP sequence targeting mouse FAP in a dual variable domain immunoglobulin (DVD-Ig) form and a mouse IL-12 sequence form a knob-in-hole structure, and an effector function is removed.

T1.05m (seq22, seq23, seq24) is a bispecific antibody in which a mouse anti-FAP sequence targeting mouse FAP in a DVD-Ig form and a mouse IL-12 sequence containing mutations (four) of amino acids involved in heparin binding form a knob-in-hole structure, and an effector function is removed.

T1.06m (seq19, seq20, seq25) is a bispecific antibody in which a mouse anti-FAP sequence in which a single chain variable fragment (scFv) of a mouse anti-FAP sequence targeting mouse FAP is included to the C-terminus of the heavy chain and a mouse IL-12 sequence form a knob-in-hole structure, and an effector function is removed.

T1.07m (seq19, seq22, seq25) is a bispecific antibody in which a mouse anti-FAP sequence in which a scFv of a mouse anti-FAP sequence targeting mouse FAP is included to the C-terminus of the heavy chain and a mouse IL-12 sequence containing mutations (four) of amino acids involved in heparin binding form a knob-in-hole structure, and an effector function is removed.

T1.08m (seq18, seq20, seq26) is a bispecific antibody in which a mouse anti-FAP sequence in which a scFv of a mouse anti-FAP sequence targeting mouse FAP is included to the C-terminus of the light chain and a mouse IL-12 sequence form a knob-in-hole structure, and an effector function is removed.

T1.09m (seq18, seq22, seq26) is a bispecific antibody in which a mouse anti-FAP sequence in which a scFv of a mouse anti-FAP sequence targeting mouse FAP is included to the C-terminus of the light chain and a mouse IL-12 sequence containing mutations (four) of amino acids involved in heparin binding form a knob-in-hole structure, and an effector function is removed.

T1.10m (seq18, seq19, seq27) is an antibody in which a mouse anti-FAP sequence targeting mouse FAP and a mouse anti-FAP sequence in which a mouse IL-12 sequence is linked to the C-terminus of the heavy chain form a knob-in-hole structure.

T1.11m (seq18, seq19, seq28) is an antibody in which a mouse anti-FAP sequence targeting mouse FAP and a mouse anti-FAP sequence in which a mouse IL-12 sequence containing mutations (four) of amino acids involved in heparin binding is linked to the C-terminus of the heavy chain form a knob-in-hole structure.

T1.12m (seq18, seq19, seq29) is an antibody in which an effector function of a knob-in-hole structure having only a mouse anti-FAP sequence targeting mouse FAP is removed.

T1.13m (seq20, seq30) is an antibody in which an effector function of a knob-in-hole structure having only a mouse IL-12 sequence is removed.

T1.14m (seq21, seq30) is an antibody in which an effector function of a knob-in-hole structure having only a mouse IL-12 sequence containing mutations (two) of amino acids involved in heparin binding is removed.

T1.15m (seq22, seq30) is an antibody in which an effector function of a knob-in-hole structure having only a mouse IL-12 sequence containing mutations (four) of amino acids involved in heparin binding is removed.

T1.16m (seq19, seq31) is a mouse anti-FAP antibody targeting mouse FAP.

T1.17m (seq32) is an Fc-fusion protein dimer having only a mouse IL-12 sequence containing mutations (four) of amino acids involved in heparin binding.

T1.18m (seq20, seq33, seq34) is a bispecific antibody in which a mouse anti-CD20 (18B12) sequence targeting mouse CD20 and a mouse IL-12 sequence form a knob-in-hole structure, and an effector function is removed.

T1.19m (seq21, seq33, seq34) is a bispecific antibody in which a mouse anti-CD20 (18B12) sequence targeting mouse CD20 and a mouse IL-12 sequence containing mutations (two) of amino acids involved in heparin binding form a knob-in-hole structure, and an effector function is removed.

T1.20m (seq22, seq33, seq34) is a bispecific antibody in which a mouse anti-CD20 sequence targeting mouse CD20 and a mouse IL-12 sequence containing mutations (four) of amino acids involved in heparin binding form a knob-in-hole structure, and an effector function is removed.

T1.21m (seq29, seq33, seq34) is an antibody in which an effector function of a knob-in-hole structure having only a mouse anti-CD20 sequence targeting mouse CD20 is removed.

T1.22m (seq35) is a protein having only a mouse Fc sequence in which an effector function is removed.

T1.23m (seq18, seq19, seq298) is a bispecific antibody in which a mouse anti-FAP sequence and a mouse IL-12 sequence in which a scFv of a mouse anti-FAP sequence is linked to the C-terminus form a knob-in-hole structure, and an effector function is removed.

T1.24m (seq18, seq19, seq299) is a bispecific antibody in which a mouse anti-FAP heavy chain sequence and a mouse IL-12 sequence containing mutations (four) of amino acids involved in heparin binding in which a scFv of a mouse anti-FAP sequence is linked to the C-terminus form a knob-in-hole structure, and an effector function is removed.

T1.25m (seq19, seq300) is an Fc-fusion protein dimer containing a mouse anti-FAP sequence in which a mouse IL-12 sequence is linked to the C-terminus of the heavy chain.

T1.26m (seq19, seq301) is an Fc-fusion protein dimer containing a mouse anti-FAP sequence in which a mouse IL-12 sequence containing mutations (four) of amino acids involved in heparin binding is linked to the C-terminus of the heavy chain.

T1.27m (seq302) is an Fc-fusion protein dimer consisting of a mouse IL-12 sequence in which a scFv of a mouse anti-FAP sequence is linked to the C-terminus.

T1.28m (seq303) is an Fc-fusion protein dimer composed of a mouse IL-12 sequence containing mutations (four) of amino acids involved in heparin binding in which a scFv of a mouse anti-FAP sequence is linked to the C-terminus.
[seq18] anti-mu FAP HC mu IgG2a Fc knob DANG 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
REDYGSTLRVVSALPIQHQDWMSGKEFKCKVNNKDLPAPIERTISKPKGSVRAPQVYVL
PPPEEEMTKKQVTLWCMVTDFMPEDIYVEWTNNGKTELNYKNTEPVLDSDGSYFMYS
KLRVEKKNWVERNSYSCSVVHEGLHNHHTTKSFSRTPGK
[seq19] anti-mu FAP LC 
QIVLTQSPAIMSASPGEKVTMTCSASSGVNFMHWYQQKSGTSPKRWIFDTSKLA
SGVPARFSGSGSGTSYSLTISSMEAEDAATYYCQQWSFNPPTFGGGTKLEIKRADAAPTV
SIFPPSSEQLTSGGASVVCFLNNFYPKDINVKWK
IDGSERQNGVLNSWTDQDSKDSTYSMSSTLTLTKDEYERHNSYTCEATHKTSTS
PIVKSFNRNEC
[seq20] mu scIL-12-mu IgG2a Fc hole DANG 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQRKKEKMKETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLSCAVTDFMPEDIYVEWTNNGKTE
LNYKNTEPVLDSDGSYFMVSKLRVEKKNWVERNSYSCSVVHEGLHNHHTTKSFSRTPG
K
[seq21] mu scIL-12 mut1-mu IgG2a Fc hole DANG 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQRAKEKMAETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLSCAVTDFMPEDIYVEWTNNGKTE
LNYKNTEPVLDSDGSYFMVSKLRVEKKNWVERNSYSCSVVHEGLHNHHTTKSFSRTPG
K
[seq22] mu scIL-12 mut2-mu IgG2a Fc hole DANG 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
 
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQAAAEKMAETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLSCAVTDFMPEDIYVEWTNNGKTE
LNYKNTEPVLDSDGSYFMVSKLRVEKKNWVERNSYSCSVVHEGLHNHHTTKSFSRTPG
K
[seq23] (anti-mu FAP VH)2, mu IgG2a Fc knob DANG 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAGGGGSGGGGSGGGGSQVQLQQSGAELARPGASVNLSCKASGYTFTNNGINWL
KQRTGQGLEWIGEIYPRSTNTLYNEKFKGKATLTADRSSNTAYMELRSLTSEDSAVYFC
ARTLTAPFAFWGQGTLVTVSAAKTTAPSVYPLAPVCGDTTGSSVTLGCLVKGYFPEPVT
LTWNSGSLSSGVHTFPAVLQSDLYTLSSSVTVTSSTWPSQSITCNVAHPASSTKVDKKIEP
RGPTIKPCPPCKCPAPNLLGGPSVFIFPPKIKDVLMISLSPIVTCVVVAVSEDDPDVQISWF
VNNVEVHTAQTQTHREDYGSTLRVVSALPIQHQDWMSGKEFKCKVNNKDLPAPIERTIS
KPKGSVRAPQVYVLPPPEEEMTKKQVTLWCMVTDFMPEDIYVEWTNNGKTELNYKNT
EPVLDSDGSYFMYSKLRVEKKNWVERNSYSCSVVHEGLHNHHTTKSFSRTPGK
[seq24] (anti-mu FAP VL)2 
QIVLTQSPAIMSASPGEKVTMTCSASSGVNFMHWYQQKSGTSPKRWIFDTSKLA
SGVPARFSGSGSGTSYSLTISSMEAEDAATYYCQQWSFNPPTFGGGTKLEIKGGGGSGG
GGSGGGGSQIVLTQSPAIMSASPGEKVTMTCSASSGVNFMHWYQQKSGTSPKRWIFDTS
KLASGVPARFSGSGSGTSYSLTISSMEAEDAATYYCQQWSFNPPTFGGGTKLEIKRADA
APTVSIFPPSSEQLTSGGASVVCFLNNFYPKDINVKWKIDGSERQNGVLNSWTDQDSKDS
TYSMSSTLTLTKDEYERHNSYTCEATHKTSTSPIVKSFNRNEC
[seq25] anti-mu FAP HC mu IgG2a Fc knob DANG-anti-mu FAP scFv 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
REDYGSTLRVVSALPIQHQDWMSGKEFKCKVNNKDLPAPIERTISKPKGSVRAPQVYVL
PPPEEEMTKKQVTLWCMVTDFMPEDIYVEWTNNGKTELNYKNTEPVLDSDGSYFMYS
KLRVEKKNWVERNSYSCSVVHEGLHNHHTTKSFSRTPGKGGGGSGGGGSGGGGSQVQ
 
LQQSGAELARPGASVNLSCKASGYTFTNNGINWLKQRTGQGLEWIGEIYPRSTNTLYNE
KFKGKATLTADRSSNTAYMELRSLTSEDSAVYFCARTLTAPFAFWGQGTLVTVSAGGG
GSGGGGSGGGGSGGGGSQIVLTQSPAIMSASPGEKVTMTCSASSGVNFMHWYQQKSGT
SPKRWIFDTSKLASGVPARFSGSGSGTSYSLTISSMEAEDAATYYCQQWSFNPPTFGGGT
KLEIK
[seq26] anti-mu FAP LC-anti-mu FAP scFv 
QIVLTQSPAIMSASPGEKVTMTCSASSGVNFMHWYQQKSGTSPKRWIFDTSKLA
SGVPARFSGSGSGTSYSLTISSMEAEDAATYYCQQWSFNPPTFGGGTKLEIKRADAAPTV
SIFPPSSEQLTSGGASVVCFLNNFYPKDINVKWKIDGSERQNGVLNSWTDQDSKDSTYS
MSSTLTLTKDEYERHNSYTCEATHKTSTSPIVKSFNRNECGGGGSGGGGSGGGGSQVQL
QQSGAELARPGASVNLSCKASGYTFTNNGINWLKQRTGQGLEWIGEIYPRSTNTLYNEK
FKGKATLTADRSSNTAYMELRSLTSEDSAVYFCARTLTAPFAFWGQGTLVTVSAGGGG
SGGGGSGGGGSGGGGSQIVLTQSPAIMSASPGEKVTMTCSASSGVNFMHWYQQKSGTS
PKRWIFDTSKLASGVPARFSGSGSGTSYSLTISSMEAEDAATYYCQQWSFNPPTFGGGTK
LEIK
[seq27] anti-mu FAP HC mu IgG2a Fc hole DANG-mu scIL-12 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
REDYGSTLRVVSALPIQHQDWMSGKEFKCKVNNKDLPAPIERTISKPKGSVRAPQVYVL
PPPEEEMTKKQVTLSCAVTDFMPEDIYVEWTNNGKTELNYKNTEPVLDSDGSYFMVSK
LRVEKKNWVERNSYSCSVVHEGLHNHHTTKSFSRTPGGGGGSGGGGSGGGGSMWELE
KDVYVVEVDWTPDAPGETVNLTCDTPEEDDITWTSDQRHGVIGSGKTLTITVKEFLDAG
QYTCHKGGETLSHSHLLLHKKENGIWSTEILKNFKNKTFLKCEAPNYSGRFTCSWLVQR
NMDLKFNIKSSSSSPDSRAVTCGMASLSAEKVTLDQRDYEKYSVSCQEDVTCPTAEETL
PIELALEARQQNKYENYSTSFFIRDIIKPDPPKNLQMKPLKNSQVEVSWEYPDSWSTPHS
YFSLKFFVRIQRKKEKMKETEEGCNQKGAFLVEKTSTEVQCKGGNVCVQAQDRYYNSS
CSKWACVPCRVRSGGGGSGGGGSGGGGSRVIPVSGPARCLSQSRNLLKTTDDMVKTAR
EKLKHYSCTAEDIDHEDITRDQTSTLKTCLPLELHKNESCLATRETSSTTRGSCLPPQKTS
LMMTLCLGSIYEDLKMYQTEFQAINAALQNHNHQQIILDKGMLVAIDELMQSLNHNGE
TLRQKPPVGEADPYRVKMKLCILLHAFSTRVVTINRVMGYLSSA
[seq28] anti-mu FAP HC mu IgG2a Fc hole DANG-mu scIL-12 mut2 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
 
REDYGSTLRVVSALPIQHQDWMSGKEFKCKVNNKDLPAPIERTISKPKGSVRAPQVYVL
PPPEEEMTKKQVTLSCAVTDFMPEDIYVEWTNNGKTELNYKNTEPVLDSDGSYFMVSK
LRVEKKNWVERNSYSCSVVHEGLHNHHTTKSFSRTPGGGGGSGGGGSGGGGSMWELE
KDVYVVEVDWTPDAPGETVNLTCDTPEEDDITWTSDQRHGVIGSGKTLTITVKEFLDAG
QYTCHKGGETLSHSHLLLHKKENGIWSTEILKNFKNKTFLKCEAPNYSGRFTCSWLVQR
NMDLKFNIKSSSSSPDSRAVTCGMASLSAEKVTLDQRDYEKYSVSCQEDVTCPTAEETL
PIELALEARQQNKYENYSTSFFIRDIIKPDPPKNLQMKPLKNSQVEVSWEYPDSWSTPHS
YFSLKFFVRIQAAAEKMAETEEGCNQKGAFLVEKTSTEVQCKGGNVCVQAQDRYYNSS
CSKWACVPCRVRSGGGGSGGGGSGGGGSRVIPVSGPARCLSQSRNLLKTTDDMVKTAR
EKLKHYSCTAEDIDHEDITRDQTSTLKTCLPLELHKNESCLATRETSSTTRGSCLPPQKTS
LMMTLCLGSIYEDLKMYQTEFQAINAALQNHNHQQIILDKGMLVAIDELMQSLNHNGE
TLRQKPPVGEADPYRVKMKLCILLHAFSTRVVTINRVMGYLSSA
[seq29] mu IgG2a Fc hole DANG 
EPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLSCAVTDFMPEDIYVEWTNNGKTE
LNYKNTEPVLDSDGSYFMVSKLRVEKKNWVERNSYSCSVVHEGLHNHHTTKSFSRTPG
K
[seq30] mu IgG2a Fc knob DANG 
EPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLWCMVTDFMPEDIYVEWTNNGKT
ELNYKNTEPVLDSDGSYFMYSKLRVEKKNWVERNSYSCSVVHEGLHNHHTTKSFSRTP
GK
[seq31] anti-mu FAP HC mu IgG2a Fc DANG 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
REDYGSTLRVVSALPIQHQDWMSGKEFKCKVNNKDLPAPIERTISKPKGSVRAPQVYVL
PPPEEEMTKKQVTLTCMVTDFMPEDIYVEWTNNGKTELNYKNTEPVLDSDGSYFMYSK
LRVEKKNWVERNSYSCSVVHEGLHNHHTTKSFSRTPGK
[seq32] mu scIL-12 mut2-mu IgG2a Fc DANG 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQAAAEKMAETEEGCNQKGAFLVEKTSTEVQCKGGNVCVQ
 
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLTCMVTDFMPEDIYVEWTNNGKT
ELNYKNTEPVLDSDGSYFMYSKLRVEKKNWVERNSYSCSVVHEGLHNHHTTKSFSRTP
GK
[seq33] anti-mu CD20 HC mu IgG2a Fc knob DANG 
QVQLQQPGAELVRPGTSVKLSCKASGYTFTSYWMHWIKQRPGQGLEWIGVIDP
SDNYTKYNQKFKGKATLTVDTSSSTAYMQLSSLTSEDSAVYFCAREGYYGSSPWFAYW
GQGTLVTVSSAKTTAPSVYPLAPVCGDTTGSSVTLGCLVKGYFPEPVTLTWNSGSLSSG
VHTFPAVLQSDLYTLSSSVTVTSSTWPSQSITCNVAHPASSTKVDKKIEPRGPTIKPCPPC
KCPAPNLLGGPSVFIFPPKIKDVLMISLSPIVTCVVVAVSEDDPDVQISWFVNNVEVHTA
QTQTHREDYGSTLRVVSALPIQHQDWMSGKEFKCKVNNKDLPAPIERTISKPKGSVRAP
QVYVLPPPEEEMTKKQVTLWCMVTDFMPEDIYVEWTNNGKTELNYKNTEPVLDSDGS
YFMYSKLRVEKKNWVERNSYSCSVVHEGLHNHHTTKSFSRTPGK
[seq34] anti-mu CD20 LC 
QIVMSQSPAILSASPGEKVTMTCRARSSVSYIHWYQQKPGSSPKPWIYATSNLAS
GVPGRFSGSGSGTSYSLTITRVEAEDAATYYCQQWSSKPPTFGGGTKLEIKRTDAAPTVS
IFPPSSEQLTSGGASVVCFLNNFYPKDINVKWKIDGSERQNGVLNSWTDQDSKDSTYSM
SSTLTLTKDEYERHNSYTCEATHKTSTSPIVKSFNRNEC
[seq35] mu IgG2a Fc DANG 
EPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLTCMVTDFMPEDIYVEWTNNGKT
ELNYKNTEPVLDSDGSYFMYSKLRVEKKNWVERNSYSCSVVHEGLHNHHTTKSFSRTP
GK
[seq298] mu scIL-12-mu IgG2a Fc hole DANG-anti-mu FAP scFv 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQRKKEKMKETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
 
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLSCAVTDFMPEDIYVEWTNNGKTE
LNYKNTEPVLDSDGSYFMVSKLRVEKKNWVERNSYSCSVVHEGLHNHHTTKSFSRTPG
GGGGSGGGGSGGGGSQVQLQQSGAELARPGASVNLSCKASGYTFTNNGINWLKQRTG
QGLEWIGEIYPRSTNTLYNEKFKGKATLTADRSSNTAYMELRSLTSEDSAVYFCARTLT
APFAFWGQGTLVTVSAGGGGSGGGGSGGGGSGGGGSQIVLTQSPAIMSASPGEKVTMT
CSASSGVNFMHWYQQKSGTSPKRWIFDTSKLASGVPARFSGSGSGTSYSLTISSMEAED
AATYYCQQWSFNPPTFGGGTKLEIK
[seq299] mu scIL-12 mut2-mu IgG2a Fc hole DANG-anti-mu FAP scFv 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQAAAEKMAETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLSCAVTDFMPEDIYVEWTNNGKTE
LNYKNTEPVLDSDGSYFMVSKLRVEKKNWVERNSYSCSVVHEGLHNHHTTKSFSRTPG
GGGGSGGGGSGGGGSQVQLQQSGAELARPGASVNLSCKASGYTFTNNGINWLKQRTG
QGLEWIGEIYPRSTNTLYNEKFKGKATLTADRSSNTAYMELRSLTSEDSAVYFCARTLT
APFAFWGQGTLVTVSAGGGGSGGGGSGGGGSGGGGSQIVLTQSPAIMSASPGEKVTMT
CSASSGVNFMHWYQQKSGTSPKRWIFDTSKLASGVPARFSGSGSGTSYSLTISSMEAED
AATYYCQQWSFNPPTFGGGTKLEIK
[seq300] anti-mu FAP HC mu IgG2a Fc DANG-mu scIL-12 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
REDYGSTLRVVSALPIQHQDWMSGKEFKCKVNNKDLPAPIERTISKPKGSVRAPQVYVL
PPPEEEMTKKQVTLTCMVTDFMPEDIYVEWTNNGKTELNYKNTEPVLDSDGSYFMYSK
LRVEKKNWVERNSYSCSVVHEGLHNHHTTKSFSRTPGGGGGSGGGGSGGGGSMWELE
KDVYVVEVDWTPDAPGETVNLTCDTPEEDDITWTSDQRHGVIGSGKTLTITVKEFLDAG
QYTCHKGGETLSHSHLLLHKKENGIWSTEILKNFKNKTFLKCEAPNYSGRFTCSWLVQR
 
NMDLKFNIKSSSSSPDSRAVTCGMASLSAEKVTLDQRDYEKYSVSCQEDVTCPTAEETL
PIELALEARQQNKYENYSTSFFIRDIIKPDPPKNLQMKPLKNSQVEVSWEYPDSWSTPHS
YFSLKFFVRIQRKKEKMKETEEGCNQKGAFLVEKTSTEVQCKGGNVCVQAQDRYYNSS
CSKWACVPCRVRSGGGGSGGGGSGGGGSRVIPVSGPARCLSQSRNLLKTTDDMVKTAR
EKLKHYSCTAEDIDHEDITRDQTSTLKTCLPLELHKNESCLATRETSSTTRGSCLPPQKTS
LMMTLCLGSIYEDLKMYQTEFQAINAALQNHNHQQIILDKGMLVAIDELMQSLNHNGE
TLRQKPPVGEADPYRVKMKLCILLHAFSTRVVTINRVMGYLSSA
[seq301] anti-mu FAP HC mu IgG2a Fc DANG-mu scIL-12 mut2 
QVQLQQSGAELARPGASVNLSCKASGYTFTNNGINWLKQRTGQGLEWIGEIYP
RSTNTLYNEKFKGKATLTADRSSNTAYMELRSLTSEDSAVYFCARTLTAPFAFWGQGTL
VTVSAAKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCPPCKCPAP
NLLGGPSVFIFPPKIKDVLMISLSPIVTCVVVAVSEDDPDVQISWFVNNVEVHTAQTQTH
REDYGSTLRVVSALPIQHQDWMSGKEFKCKVNNKDLPAPIERTISKPKGSVRAPQVYVL
PPPEEEMTKKQVTLTCMVTDFMPEDIYVEWTNNGKTELNYKNTEPVLDSDGSYFMYSK
LRVEKKNWVERNSYSCSVVHEGLHNHHTTKSFSRTPGGGGGSGGGGSGGGGSMWELE
KDVYVVEVDWTPDAPGETVNLTCDTPEEDDITWTSDQRHGVIGSGKTLTITVKEFLDAG
QYTCHKGGETLSHSHLLLHKKENGIWSTEILKNFKNKTFLKCEAPNYSGRFTCSWLVQR
NMDLKFNIKSSSSSPDSRAVTCGMASLSAEKVTLDQRDYEKYSVSCQEDVTCPTAEETL
PIELALEARQQNKYENYSTSFFIRDIIKPDPPKNLQMKPLKNSQVEVSWEYPDSWSTPHS
YFSLKFFVRIQAAAEKMAETEEGCNQKGAFLVEKTSTEVQCKGGNVCVQAQDRYYNSS
CSKWACVPCRVRSGGGGSGGGGSGGGGSRVIPVSGPARCLSQSRNLLKTTDDMVKTAR
EKLKHYSCTAEDIDHEDITRDQTSTLKTCLPLELHKNESCLATRETSSTTRGSCLPPQKTS
LMMTLCLGSIYEDLKMYQTEFQAINAALQNHNHQQIILDKGMLVAIDELMQSLNHNGE
TLRQKPPVGEADPYRVKMKLCILLHAFSTRVVTINRVMGYLSSA
[seq302] mu scIL-12-mu IgG2a Fc DANG-anti-mu FAP scFv 
MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQRKKEKMKETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLTCMVTDFMPEDIYVEWTNNGKT
ELNYKNTEPVLDSDGSYFMYSKLRVEKKNWVERNSYSCSVVHEGLHNHHTTKSFSRTP
 
GGGGSGGGGSGGGGSQVQLQQSGAELARPGASVNLSCKASGYTFTNNGINWLKQRTG
QGLEWIGEIYPRSTNTLYNEKFKGKATLTADRSSNTAYMELRSLTSEDSAVYFCARTLT
APFAFWGQGTLVTVSAGGGGSGGGGSGGGGSGGGGSQIVLTQSPAIMSASPGEKVTMT
CSASSGVNFMHWYQQKSGTSPKRWIFDTSKLASGVPARFSGSGSGTSYSLTISSMEAED
AATYYCQQWSFNPPTFGGGTKLEIK
[seq303] mu scIL-12 mut2-mu IgG2a Fc DANG-anti-mu FAP scFv

MWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSG
RFTCSWLVQRNMDLKFNIKSSSSSPDSRAVTCGMASLSAEKVTLDQRDYEKYSVSCQED
VTCPTAEETLPIELALEARQQNKYENYSTSFFIRDIIKPDPPKNLQMKPLKNSQVEVSWEY
PDSWSTPHSYFSLKFFVRIQAAAEKMAETEEGCNQKGAFLVEKTSTEVQCKGGNVCVQ
AQDRYYNSSCSKWACVPCRVRSGGGGSGGGGSGGGGSRVIPVSGPARCLSQSRNLLKT
TDDMVKTAREKLKHYSCTAEDIDHEDITRDQTSTLKTCLPLELHKNESCLATRETSSTTR
GSCLPPQKTSLMMTLCLGSIYEDLKMYQTEFQAINAALQNHNHQQIILDKGMLVAIDEL
MQSLNHNGETLRQKPPVGEADPYRVKMKLCILLHAFSTRVVTINRVMGYLSSAGGGGS
GGGGSEPRGPTIKPCPPCKCPAPNLLGGPSVFIFPPKIKDVLMISLSPIVTCVVVAVSEDDP
DVQISWFVNNVEVHTAQTQTHREDYGSTLRVVSALPIQHQDWMSGKEFKCKVNNKDL
PAPIERTISKPKGSVRAPQVYVLPPPEEEMTKKQVTLTCMVTDFMPEDIYVEWTNNGKT
ELNYKNTEPVLDSDGSYFMYSKLRVEKKNWVERNSYSCSVVHEGLHNHHTTKSFSRTP
GGGGSGGGGSGGGGSQVQLQQSGAELARPGASVNLSCKASGYTFTNNGINWLKQRTG
QGLEWIGEIYPRSTNTLYNEKFKGKATLTADRSSNTAYMELRSLTSEDSAVYFCARTLT
APFAFWGQGTLVTVSAGGGGSGGGGSGGGGSGGGGSQIVLTQSPAIMSASPGEKVTMT
CSASSGVNFMHWYQQKSGTSPKRWIFDTSKLASGVPARFSGSGSGTSYSLTISSMEAED
AATYYCQQWSFNPPTFGGGTKLEIK

### Manufacturing Example 1. Manufacturing of fusion proteins

Reagents and equipment are described in Tables 3 and 4 below.

**[Table 3]**

| Reagent | Manufacturer | Catalog # |
|---|---|---|
| pTT5 | chempartner | |
| In-Fusion HD cloning kit | Clontech | 639648 |
| Accuprime pfx DNA polymerase | Invitrogen | 12344-04 |
| Gel DNA fragment purification Kit | TaKaRa | D823A |
| FastDigest^{®}BamHI | Fermentas | FD0055 |
| F astDigest^{®}EcoRI | Fermentas | FD0275 |

**[Table 4]**

| Equipment and tool | Manufacturer | Model name |
|---|---|---|
| Biosafety cabinet | NUAIRE | LabGard class ± |
| Centrifuge | Eppendorf | 5424 |
| Gel Imaging System | Tanon | 2500R |

The synthesized DNA fragment was amplified through PCR, and the PCR product was purified by gel. The pTT5 vector was cleaved by the restriction enzymes EcoRI and BamHI, and then the gel was purified. Each PCR product and the linear vector were ligated using the In-Fusion kit. The produced vector was transformed into ECOS101 DH5α competent cells and the cell were cultured on 2xYT agar plates containing 100 µg/ml ampicillin. All manipulation processes were performed according to standard transformation protocols. Positive recombinants were identified by colony PCR, and sequence-verification sequencing was performed on the recombinant plasmid. A single colony was selected and the seed culture was inoculated into 5 ml of 2xYT medium containing 100 µg/ml ampicillin. It was cultured with shaking at 37°C for 8 hours.

Thereafter, the seed culture was diluted in 200 ml of selective 2xYT medium at a ratio of 1:1,000. It was cultured with shaking at 37°C for 16 hours. Bacterial cells were harvested by centrifugation at 4°C, 4,700 rpm for 10 minutes. The bacterial pellet was resuspended in 12 ml of RES-EF buffer. Thereafter, 12 ml of LYS-EF buffer was added, and the sealed tube was inverted vigorously to mix thoroughly and then incubated for 5 minutes at room temperature. 12 ml of NEU-EF buffer was added to the lysate, and inverted vigorously to mix thoroughly and rapidly.

Before injecting the lysate into the NucleoBond^{®} Xtra column filter, a homogeneous suspension of the precipitate was prepared by inverting the lysate tube 3 times to prevent clogging of the filter. Thereafter, the NucleoBond^{®} Xtra column filter and the NucleoBond^{®} Xtra column were washed with 10 ml of filter wash buffer FIL-EF. The NucleoBond^{®} Xtra column filter was removed by pulling out or inverting the column. The NucleoBond^{®} Xtra column was washed with 90 ml of wash buffer ENDO.

The NucleoBond^{®} Xtra column was washed with 45 ml of wash buffer WASH-EF. Plasmid DNA was eluted with 15 ml of elution buffer ELU. The eluate was collected in a 50 ml centrifugation tube. 10.5 ml of isopropanol at room temperature was added to precipitate the eluted plasmid DNA. After vortex, the mixture was allowed to stand for 2 minutes.

Thereafter, 5 ml of 70% ethanol was added to the pellet. Ethanol was carefully and completely removed from the tube using a pipette tip. The pellet was dried at room temperature (20°C). Thereafter, the DNA pellet was dissolved with 1,000 µl of H₂O.

### Manufacturing Example 2. Cell transfection and protein expression

### Manufacturing Example 2.1. Cell transfection

Materials and reagents used are described in Table 5 below.

**[Table 5]**

| Material and reagent | Manufacturer (Product #) |
|---|---|
| 293F cells | Invitrogen (R790-07) |
| OPM 293 | OPM (81075-001) |
| Pluronic^{®}F-68, 10% (100X) | Gibco (24040-032) |
| 1 mg/ml PEI | Polyscience (23966) |
| OPTI MEM I | Gibco (31985088) |
| Peptone (20x) | FLUKA (P0521-1KG) |
| Shaker flask | |
| ISF1-X incubator shaker | Kuhner shaker |

The 293F seed strain containing the complete medium was maintained in an incubator shaker at 130 rpm, 37°C, and 8% CO₂. It was cultured at a density of 0.3 to 0.4 × 10⁶ cell/ml, and the medium was changed every 2 to 3 days. Twenty-four hours before transfection, a new passage number of 293F cells was prepared at 2.6 × 10⁶ cell/ml. The prepared cells were cultured in an incubator shaker at 130 rpm, 37°C, and 8% CO₂. On the day of transfection, a density of cells was adjusted to a density of 5.0 × 10⁶ cells/ml using a fresh medium. It was performed at a total volume of 1 L in a 3 L shaker flask. 0.4 mg of HC and 0.6 mg of LC plasmid were diluted with 50 ml of OPTI MEM I and filtered through a 0.22 µm filter. Thereafter, 2 mg of PEI was diluted with 50 ml of OPTI MEM I to prepare a transfection reagent.

The diluted PEI was added to the DNA mixture and then mixed immediately. Thereafter, it was cultured for 15 minutes at room temperature. The DNA-PEI mixture was added to 293F cells prepared at 2.6 × 10⁶ cell/ml. Thereafter, the cells were continuously cultured for 24 hours in an incubator shaker at 130 rpm, 37°C, and 8% CO₂. Twenty-four hours after transfection, 10% peptone was added to 1/20 of the culture solution so that the final concentration was 0.5%. Thereafter, the cells were continuously cultured in an incubator shaker at 130 rpm, 37°C, and 8% CO₂. The cell density/viability was measured and recorded daily during the 2 to 5 days period after transfection. The cells were harvested for purification 7 days after transfection or when the cell viability was less than 70%.

### Manufacturing Example 2.2. Protein purification

Reagents, composition of the buffers, and equipment used for protein purification are described in Tables 6 to 8 below.

**[Table 6]**

| Reagent | Manufacturer | Catalog # |
|---|---|---|
| Mabselect SuRe | GE Healthcare | 11003493 |
| Tris | SIGMA | 77-86-1 |
| NaCl | ACROS ORGANIVS | 7647-14-5 |
| Sodium citrate | Adamas-beta | 76198B |
| Citric acid | GENERAL-Reagent | G83162B |
| Arginine | VETEC | V900343-500G |
| Succinic acid | Sigma-Aldrich | S9512-500G |
| Triton X-100 | ABCONE | X10010-1L |
| Triton X-114 | Sigma-Aldrich | X114 |
| Millex-GP Filter Unit 0.22 µm Sterile | MILLIPORE | SLGP033RS |
| NaOH | Merck | B146369740 |

**[Table 7]**

| | |
|---|---|
| Buffer A | 25 mM Tris, 150 mM NaCl, pH 8.0 |
| Buffer B | 25 mM Tris, 150 mM NaCl, 0.1% Triton X-100, 0.1% Triton X-114 pH 8.0 |
| Buffer C | 100 mM Sodium Citrate, 150 mM NaCl, pH 3.0 |
| Buffer D | 1 M Arginine, 400 mM Succinic acid, pH 9.0 |
| Buffer E | 20 mM PB pH 6.5, 1 M (NH₄)₂SO₄ |
| Buffer F | 20 mM PB pH 6.5, 25% isopropyl alcohol |
| Final buffer | 20 mM HEPES, pH 7.5, 240 mM sucrose or 20 mM his acetate pH 5.5, 240 mM sucrose |

**[Table 8]**

| Equipment | Manufacturer | Model name |
|---|---|---|
| AKTA Pure | GE Healthcare | 29-0182-24 |
| centrifuge | Beckman | J-26xp |
| Gel Imaging System | Tanon | 2500R |
| Sartopore 2 filter | Sartorius | 5445307H9-OO-A |

The proteins were purified using a Mabselect sure column. Specifically, the supernatant was harvested by centrifugation at 2,000×g, 4°C for 20 minutes. Thereafter, the supernatant was filtered with a Sartopore 2 filter. A 5 ml MabSelect Sure column equilibrated with Buffer A was loaded with the clarified supernatant. Thereafter, the column was washed with Buffer A until the A280 absorbance reached the baseline. The column was washed with 10 CV Buffer B. The column was washed with 10 CV Buffer A. The bound proteins were eluted with 6 CV Buffer C, and 1/6 volume of Buffer D was added to neutralize the eluted substance. SDS-PAGE and SEC-HPLC analysises were performed. Thereafter, the proteins were purified using a HIC column. The proteins were then dialyzed against Buffer E at 4°C overnight. A HIC column equilibrated with Buffer E was loaded with the supernatant. Thereafter, the column was washed with Buffer E until the A280 absorbance reached the baseline. The bound proteins were eluted by gradient elution (10 CV Buffer F 0%-40%). The bound proteins were eluted with 2 CV 100% Buffer F. SDS-PAGE analysis was performed.

After purifying the proteins, the proteins were collected in one place, and then the proteins were dialyzed against the final buffer at 4°C overnight. Thereafter, SDS-PAGE and SEC-HPLC analysises were performed.

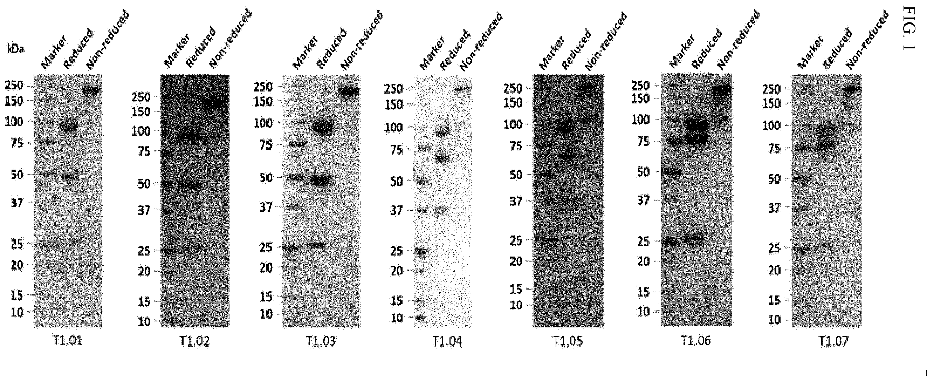

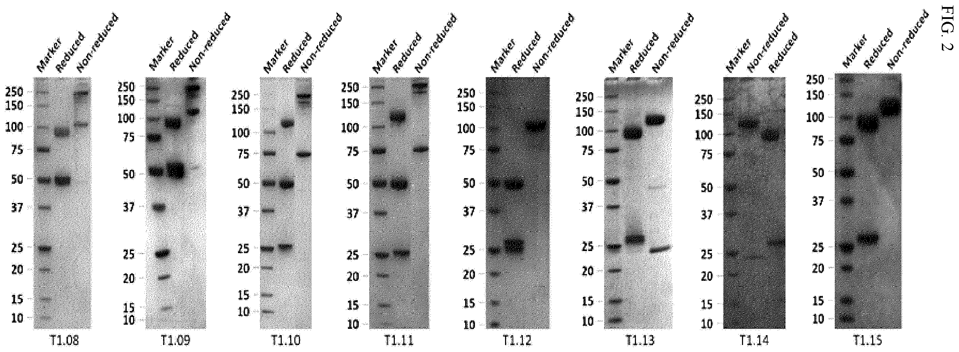

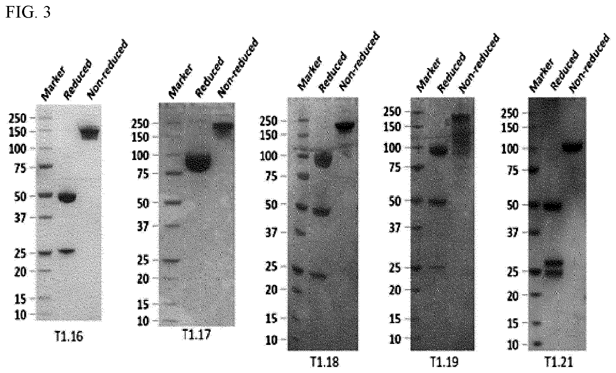

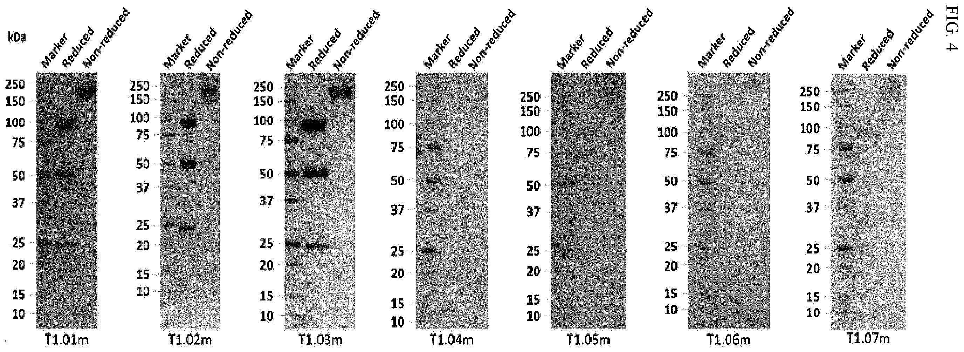

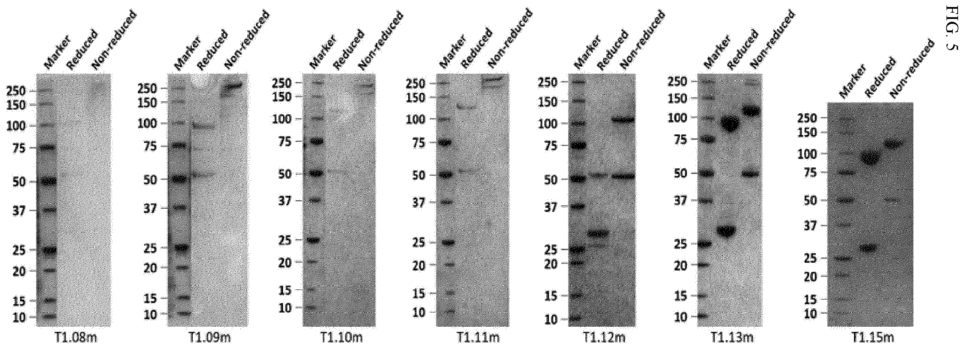

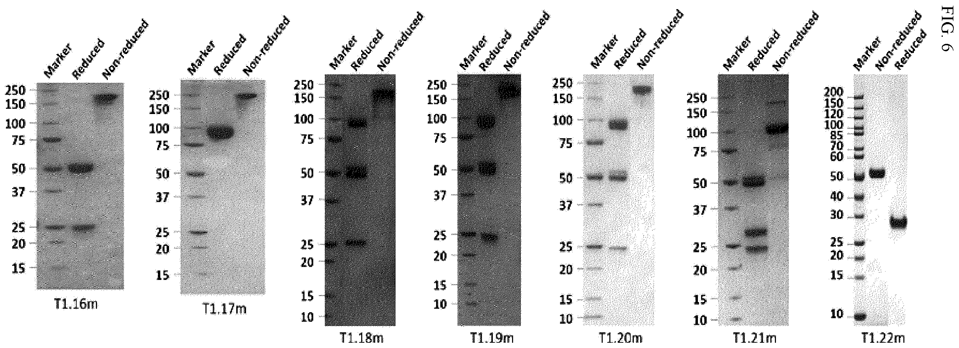

As a result, as shown in FIGS. 1 to 6, it was found that the human proteins and the mouse proteins of one embodiment were purified.

### Manufacturing Example 3. Improvement in production of bispecific antibody

### Manufacturing Example 3.1. Identification of improvement in production of fusion protein comprising human IL-12

Table 9 below shows changes in the production of a human anti-FAP/IL-12 bispecific antibody according to human IL-12 mutation. The parentheses indicate the production scale of the protein, and the unit is L. The number in front of parentheses is the production per liter obtained by dividing the amount of the proteins by the production scale.

**[Table 9]**

| PROTEIN | Description | Amount of protein after passing through Protein A column (mg) (production scale, L) |
|---|---|---|
| T1.01 | anti-hu FAP/hu IL-12, hu IgG1 DANG | 40 (1), 132 (1) |
| T1.02 | anti-hu FAP/hu IL-12 mut1, hu IgG1 DANG | 45 (1), 139.7 (2) |
| T1.03 | anti-hu FAP/hu IL-12 mut2, hu IgG1 DANG | 250 (1), 220 (5) |

As shown in Table 9 above, as the number of mutations in ammo acids involved in hepann binding in the human IL-12 p40 (beta) region increased, the amount was improved after protein purification through a Protein A column (MabSelect Sure column). It was found that the bispecific antibody T1.02 in which lysine (K) at the 280^{th} and 285^{th} amino acids was mutated to alanine (A) had improved production compared to T1.01. It was found that the bispecific antibody T1.03 in which lysine (K) at the 280^{th}, 282^{nd}, 285^{th} and 286^{th} amino acids was mutated to alanine (A) had improved production by about 2.5 times compared to T1.01.

### Manufacturing Example 3.2. Identification of improvement in production of fusion protein comprising mouse IL-12

Table 10 below shows changes in the production of a mouse anti-FAP/IL-12 bispecific antibody according to mouse IL-12 mutation.

**[Table 10]**

| PROTEIN | Description | Amount of protein after passing through Protein A column (mg) (production scale, L) |
|---|---|---|
| T1.01m | anti-mu FAP/mu IL-12, mu IgG2a DANG | 5.725 (4), 3.967 (12), 3.667 (12) |
| T1.02m | anti-mu FAP/mu IL-12 mut1, mu IgG2a DANG | 5.825 (4), 9.86 (2), 5.74 (20) |
| T1.03m | anti-mu FAP/mu IL-12 mut2, mu IgG2a DANG | 9.54 (1), 126.8 (1) |

As shown in Table 10 above, as the number of mutations in amino acids involved in heparin binding in the mouse IL-12 p40 (beta) region increased, the amount of proteins was increased after purifying the proteins through a Protein A column (MabSelect Sure column).

In each table, the amount of protein after passing through Protein A column was compared by dividing the total protein production by the production scale. It was found that the bispecific antibody T1.02m in which lysine (K) at the 277^{th} and 282^{nd} amino acids was mutated to alanine (A) and the bispecific antibody T1.03m in which arginine (R) at the 276^{th} amino acid was mutated to alanine (A), and lysine (K) at the 277^{th}, 278^{th} and 282^{nd} amino acids was mutated to alanine (A) had gradually improved production in proteins compared to T1.01m and T1.02m. In addition, it was found that the production of T1.03m using the CHO cell line was improved by about 13 times compared to the production using the HEK 293 cell line.

### Manufacturing Example 4. Cell line and culture of cell line

The HEK-Blue IL-12 cell line transformed with the IL-12 receptor gene and the STAT4 inducible SEAP reporter gene into HEK293 cells, which are human embryonic kidney fibroblasts, was obtained from Invivogen (San Diego, USA). The HEK-Blue IL-12 cells were maintained in DMEM (GIBCO) containing 10% FBS (GIBCO), 100 µg/ml Normocin and 1X HEK-Blue selection.

The human embryonic kidney fibroblast cell line HEK293, the mouse colorectal cancer cell line CT26-WT, the mouse melanoma cell line B16F10 and the mouse fibroblast cell line NIH-3T3 were obtained from ATCC (American Type Culture Collection, Manassas, VA, USA). The HEK293 cells, the B16F10 cells and the NIH-3T3 cells were maintained in DMEM (GIBCO) containing 10% FBS (GIBCO). A cell line (HEK293-hFAP) in which human FAP (fibroblast activation protein alpha) was overexpressed in the HEK293 cells was prepared using a lentivirus capable of delivering the human FAP gene. The HEK293-hFAP cells were maintained in DMEM (GIBCO) containing 10% FBS (GIBCO) and 5 µg/ml puromycin.

The cell lines (CT26-mFAP, B16F10-mFAP and NIH-3T3-mFAP) in which mouse FAP (fibroblast activation protein alpha) was overexpressed in the CT26-WT cells, B16F10 cells, and NIH-3T3 cells were prepared using a lentivirus capable of delivering the mouse FAP gene. The CT26-mFAP cells were maintained in RPMI-1640 (GIBCO) containing 10% FBS (GIBCO) and 10 µg/ml puromycin. The B16F10-mFAP cells were maintained in DMEM (GIBCO) containing 10% FBS (GIBCO) and 10 µg/ml puromycin. The NIH-3T3-mFAP cells were maintained in DMEM (GIBCO) containing 10% FBS (GIBCO) and 5 µg/ml puromycin.

### Manufacturing Example 5. Isolation and activation of human immune cells

A blood pack was obtained from the Korean Red Cross, Korea, with the approval of the Institutional Review Board (IRB), and peripheral blood mononuclear cells (PBMCs) were isolated and frozen. The thawed PBMCs were subjected to the positive selection method, and human NK cells were isolated with an Easy sep (Stem cell, Vancouver, BC, CA) kit. The isolated NK cells were activated and maintained in RPMI-1640 (GIBCO) and 10% FBS containing 50 IU/ml recombinant human IL-2 (rhIL-2, R&D systems, Minnesota, USA).

The thawed PBMCs were subjected to the negative selection method, and human T cells were isolated with an Easy sep (Stem cell) kit. The human T cells were cultured on a plate coated with 1 µg/ml anti-CD3 (OKT3, Invitrogen) and activated for 72 hours. The human T cells were maintained in RPMI-1640 (GIBCO) containing 10% FBS (GIBCO).

### Example 1. Identification of binding affinity of fusion protein

### Example 1.1. Identification of binding of fusion protein to recombinant human FAP

Binding of T1.01, T1.02 and T1.03 to recombinant human FAP was identified by surface plasmon resonance (SPR).

Specifically, the surface of the CM5 chip was activated with a 1:1 mixture of 50 nM NHS (N-hydroxysuccinimide) and 200 nM EDC (1-Ethyl-3-(3-dimethylaminopropyl)carbodiimide), and 25 µg/ml of anti-human IgG (Fc) antibody was immobilized for 400 seconds at a rate of 10 µl/min. The remaining active ester groups were blocked with 1 M ethanolamine. T1.01, T1.02 and T1.03 were diluted to 2 µg/ml and reacted on the CM5 chip immobilized with the anti-human IgG (Fc) antibody. The recombinant human FAP was diluted to 200 nM in 1xHBS-EP + buffer solution and diluted by serial dilution. The diluted recombinant human FAP was reacted at a rate of 30 µl/min. The association and dissociation times were 180 seconds and 400 seconds, respectively. After the dissociation, stabilization was performed for 60 seconds, and then a regeneration was performed for 30 seconds with a 10 mM glycine pH 1.5 solution at a rate of 30 µl/min.

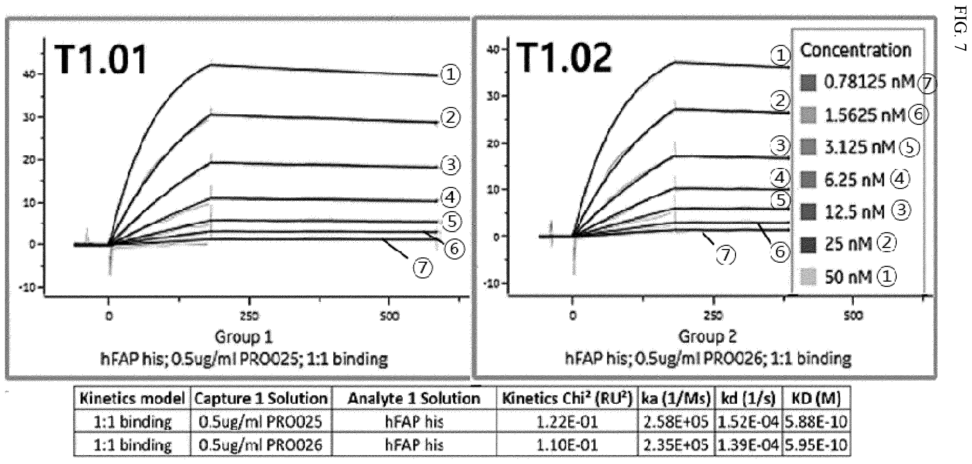

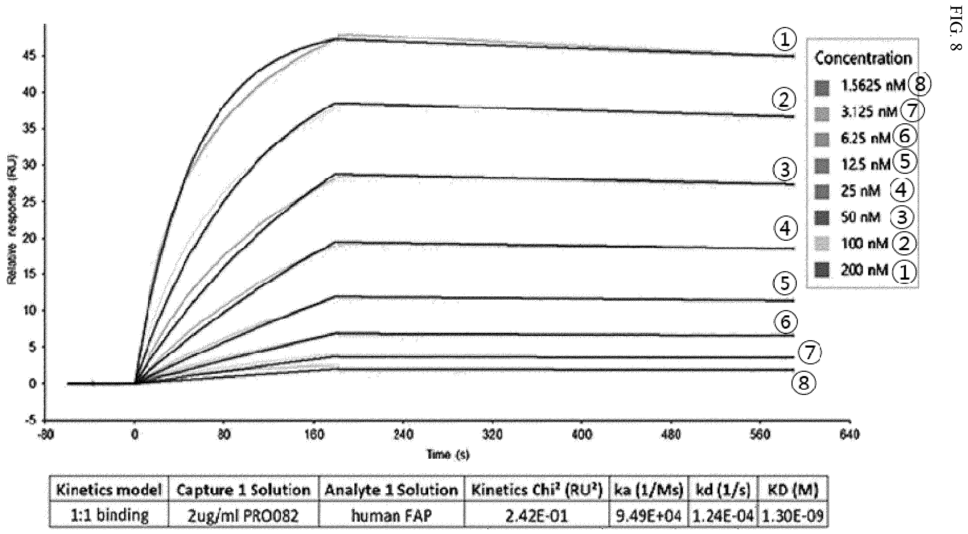

As a result, as shown in FIGS. 7 and 8, it was found that T1.01, T1.02 and T1.03 may specifically bind to recombinant human FAP.

Bindings of T1.04, T1.05, T1.06, T1.07, T1.08, T1.09, T1.10 and T1.11 to recombinant human CD20 were identified by surface plasmon resonance (SPR). The surface of the CM5 chip was activated with a 1:1 mixture of 50 nM NHS (N-hydroxysuccinimide) and 200 nM EDC (1-Ethyl-3-(3-dimethylaminopropyl)carbodiimide), and 25 µg/ml of anti-human IgG (Fc) antibody was immobilized for 400 seconds at a rate of 10 µl/min. The remaining active ester groups were blocked with 1 M ethanolamine.

Specifically, human proteins T1.04, T1.05, T1.06, T1.07, T1.08, T1.09, T1.10 and T1.11 were diluted to 1 µg/ml, 1 µg/ml, 1 µg/ml, 1 µg/ml, 0.5 µg/ml, 0.5 µg/ml, 0.5 µg/ml, and 1 µg/ml, respectively, and reacted on a CM5 chip immobilized with an anti-human IgG (Fc) antibody. The recombinant human FAP was diluted to 25 nM or 50 nM in 1xHBS-EP+ buffer solution and diluted by serial dilution. The diluted recombinant human FAP was reacted at a rate of 30 µl/min. The association and dissociation times were 180 seconds and 400 seconds, respectively. After the dissociation, stabilization was performed for 60 seconds, and then a regeneration was performed for 30 seconds with a 10 mM glycine pH 1.5 solution at a rate of 30 µl/min.

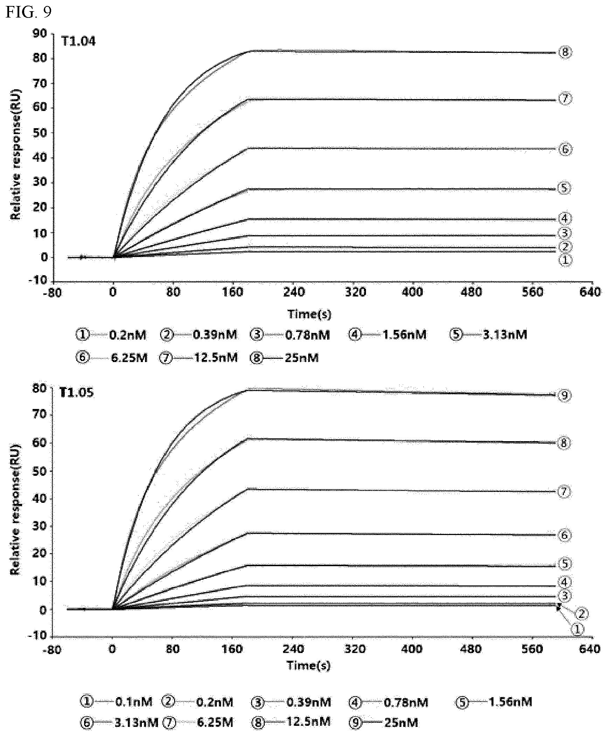

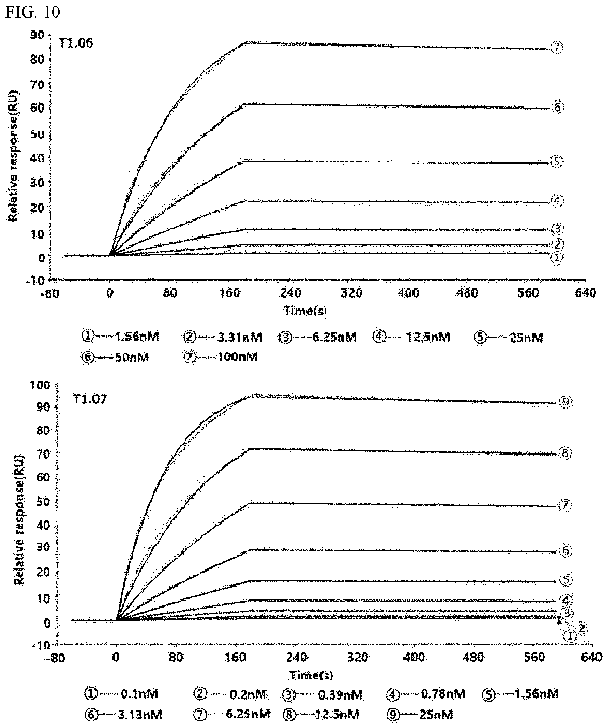

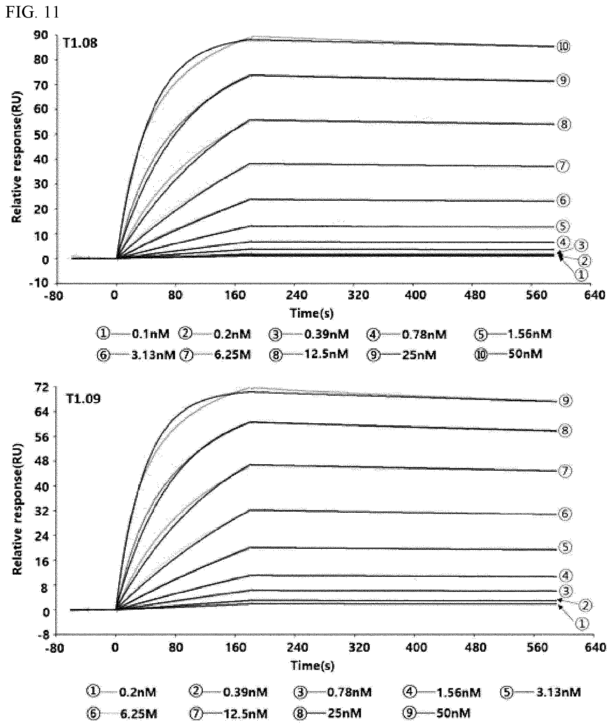

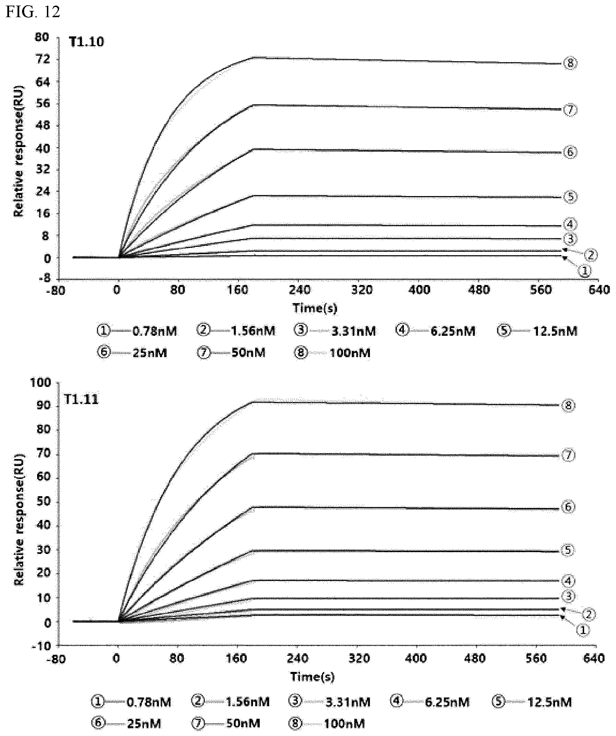

As a result, as shown in Table 11 and FIGS. 9 and 10, it was found that T1.04, T1.05, T1.06 and T1.07 may bind to recombinant human FAP to specifically target FAP. In addition, as shown in Table 12 and FIGS. 11 and 12, it was found that T1.08, T1.09, T1.10 and T1.11 may bind to recombinant human FAP to specifically target FAP.

**[Table 11]**

| **Kinetics model** | **Capture 1 Solution** | **Analyte 1 Solution** | **Kinetics Chi² (RU²)** | **ka (1/Ms)** | **kd (1/s)** | **KD (M)** |
|---|---|---|---|---|---|---|
| 1:1 binding | 1 µg/ml T1.04 | human FAP | 2.70E-01 | 5.83E+05 | 1.50E-05 | 2.57E-11 |
| 1:1 binding | 1 µg/ml T1.05 | human FAP | 2.80E-01 | 6.25E+05 | 5.83E-05 | 9.32E-11 |
| 1:1 binding | 1 µg/ml T1.06 | human FAP | 1.32E-01 | 1.04E+05 | 6.22E-05 | 5.96E-10 |
| 1:1 binding | 1 µg/ml T1.07 | human FAP | 2.33E-01 | 5.97E+05 | 7.64E-05 | 1.28E-10 |

**[Table 12]**

| **Kinetics model** | **Capture 1 Solution** | **Analyte 1 Solution** | **Kinetics Chi² (RU²)** | **ka (1/Ms)** | **kd (1/s)** | **KD (M)** |
|---|---|---|---|---|---|---|
| 1:1 binding | 0.5 µg/ml T1.08 | human FAP | 4.70E-01 | 4.46E+05 | 7.87E-05 | 1.77E-10 |
| 1:1 binding | 0.5 µg/ml T1.09 | human FAP | 3.31E-01 | 5.17E+05 | 1.11E-04 | 2.14E-10 |
| 1:1 binding | 0.5 µg/ml T1.10 | human FAP | 1.84E-01 | 1.56E+05 | 7.48E-05 | 4.79E-10 |
| 1:1 binding | 1 µg/ml T1.11 | human FAP | 2.85E-01 | 1.20E+05 | 3.19E-05 | 2.67E-10 |

### Example 1.2. Identification of binding of mouse protein to recombinant mouse FAP

Bindings of T1.01m, T1.02m and T1.03m to recombinant mouse FAP were identified by surface plasmon resonance (SPR).

The surface of the CM5 chip was activated with a 1:1 mixture of 50 nM NHS (N-hydroxysuccinimide) and 200 nM EDC (1-Ethyl-3-(3-dimethylaminopropyl)carbodiimide), and 25 µg/ml of anti-mouse IgG (Fc) antibody was immobilized for 400 seconds at a rate of 10 µl/min. The remaining active ester groups were blocked with 1 M ethanolamine. T1.01m, T1.02m and T1.03m were diluted to 2 µg/ml and reacted on the CM5 chip immobilized with the anti-mouse IgG (Fc) antibody. The recombinant mouse FAP was diluted to 200 nM in 1xHBS-EP+ buffer solution and diluted by serial dilution. The diluted recombinant mouse FAP was reacted at a rate of 30 µl/min. The association and dissociation times were 180 seconds and 400 seconds, respectively. After the dissociation, stabilization was performed for 60 seconds, and then a regeneration was performed for 30 seconds with a 10 mM glycine pH 1.5 solution at a rate of 30 µl/min.

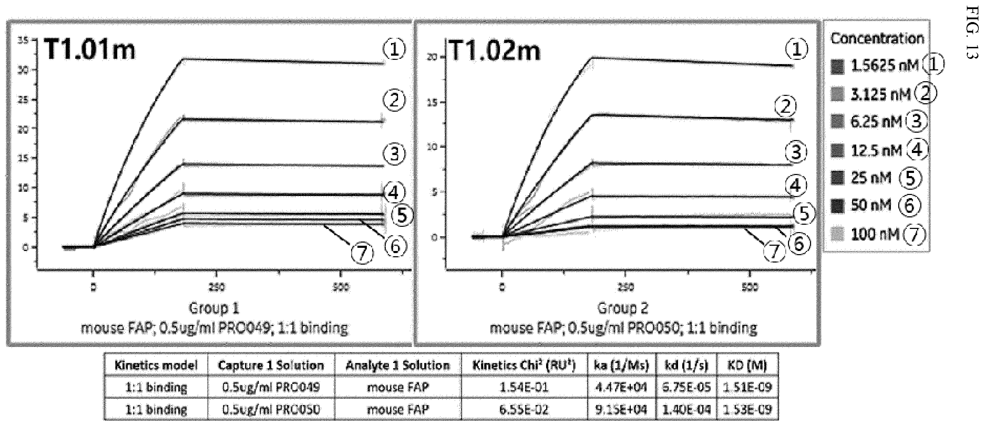

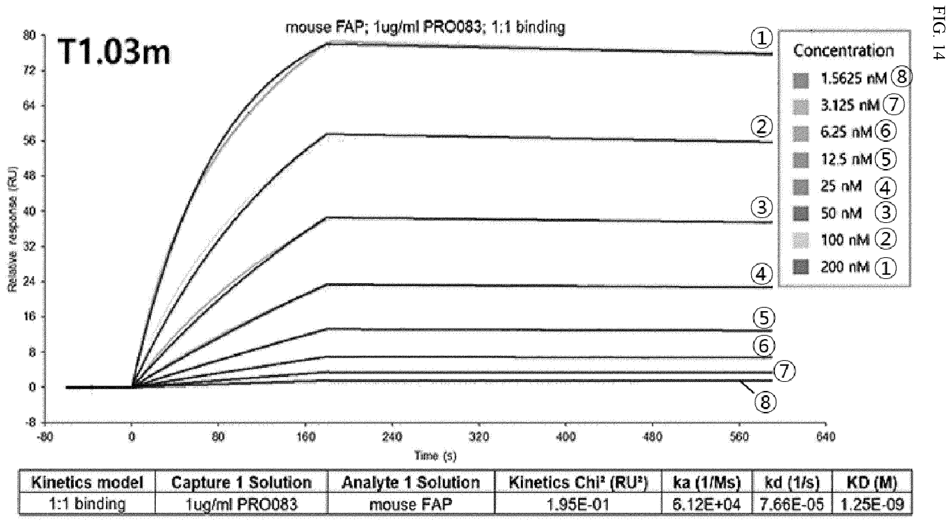

As a result, as shown in FIGS. 13 and 14, it was found that T1.01m, T1.02m and T1.03m may bind to recombinant mouse FAP to specifically target FAP.

### Example 1.3. Identification of binding to FAP-expressing cells

The degrees of binding of T1.12, T1.01, T1.02 and T1.03 to HEK293 and HEK293-hFAP cells were identified.

Specifically, the cell line was prepared by suspending in a FACS buffer solution at 1 × 10⁵ cells/100 µl, and treated with 1 µg each of T1.12, T1.01, T1.02 and T1.03. The cells were washed twice with a FACS buffer solution. The cells were stained with an anti-human IgG antibody (Biolegend). The negative control was stained only with an anti-human IgG antibody (Biolegend). The expression rates of the stained cells were measured using BD LSR and analyzed using a FlowJo software.

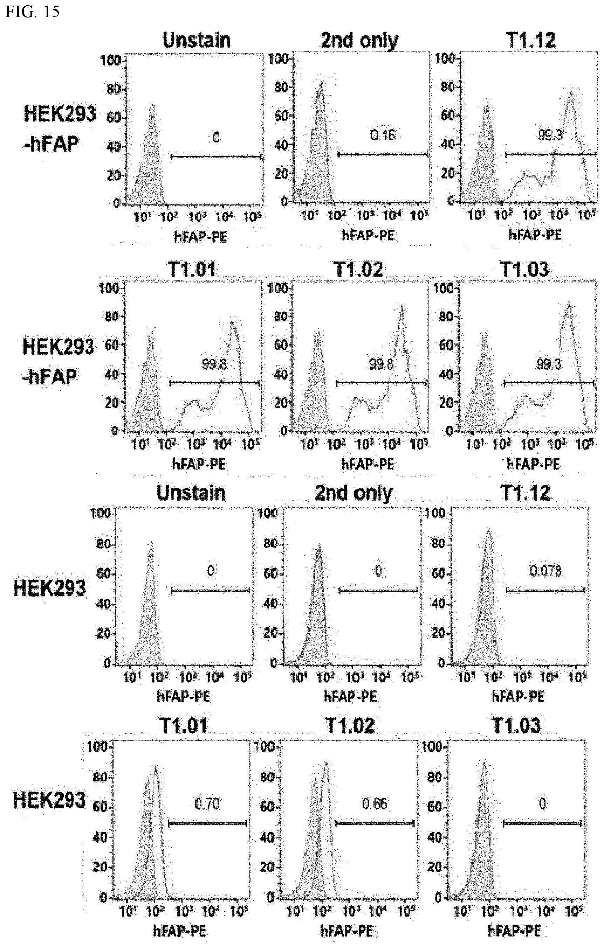

As a result, as shown in FIG. 15, it was found that T1.12, T1.01, T1.02 and T1.03 bind to the human FAP-expressing cell line by 99% or more. These results indicate that the fusion protein of one embodiment may bind to the human FAP-expressing cell line to specifically target FAP.

In addition, the degrees of binding of T1.12m, T1.01m, T1.02m and T1.03m to B16F10 and B16F10-mFAP cells were identified.

Specifically, the cell line was prepared by suspending in a FACS buffer solution at 1 × 10⁵ cells/100 µl, and treated with 1 µg of each T1.12m, T1.01m, T1.02m and T1.03m. The cells were washed twice with a FACS buffer solution. The cells were stained with an anti-mouse IgG2a antibody (Biolegend). The negative control was stained only with an anti-mouse IgG2a antibody (Biolegend). The expression rate of the stained cells was measured using BD LSR and analyzed using a FlowJo software.

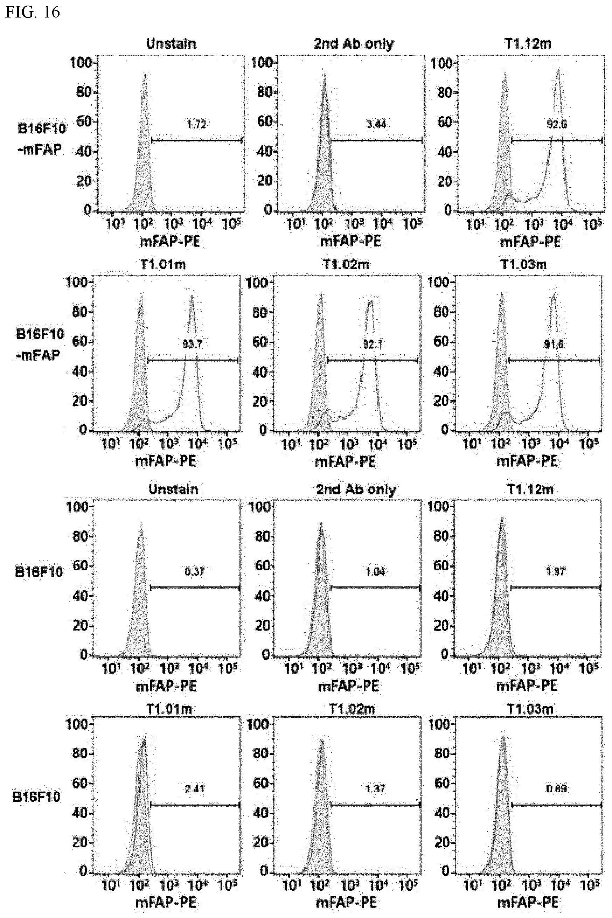

As a result, as shown in FIG. 16, it was found that T1.12m, T1.01m, T1.02m and T1.03m bind to the FAP-expressing cell line by 99% or more. These results indicate that the fusion protein of one embodiment may bind to the mouse FAP-expressing cell line to specifically target FAP.

### Example 1.4. Identification of binding of fusion protein to recombinant human IL-12 receptor

Considering that IL-12 of the fusion protein must bind to the IL-12 receptor for the action, it was found that T1.01, T1.02, T1.03 and T1.12 bind to the recombinant human IL-12 receptor.

T1.01, T1.02 and T1.03 were immobilized on a plate, and a recombinant human IL-12 receptor protein conjugated with horseradish peroxidase (HRP) was bound. Specifically, the following steps were performed:
(i) T1.01, T1.02, T1.03 and T1.12 were suspended at 5 µg/ml and diluted by serial dilution. The diluted T1.01, T1.02, T1.03 and T1.12 were aliquoted in a 96-well immune plate and treated for 24 hours.
(ii) The wells were washed with a wash solution, and then blocking was performed for 1 hour with 1% BSA (Bovine serum albumin, Sigma) solution.
(iii) The wells were washed with a wash solution, and then treated with 1 nM IL-12 receptor beta 1-biotin (IL-12Rβ1-Biotin; Acrobiosystems, Newark, USA) for 2 hours.
(iv) The wells were washed with a wash solution, and then treated with streptavidin-HRP for 20 minutes.
(v) The wells were washed with a wash solution, and then treated with a substrate solution for 20 minutes.
(vi) The wells were treated with a stop solution, and the absorbance was measured at 450 nm.

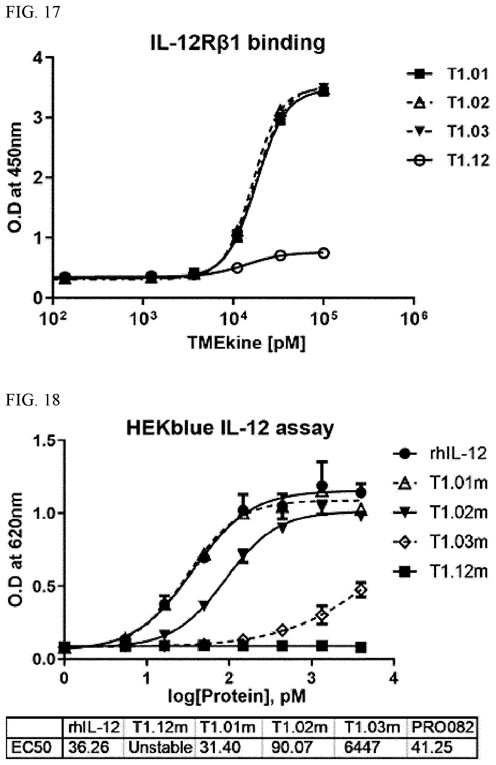

As a result, as shown in FIG. 17, it was found that T1.01, T1.02 and T1.03 bind to the recombinant human IL-12 receptor protein in a concentration dependent manner. T1.12 was used as a control.

### Example 2. Identification of ability of fusion protein to induce signaling

### Example 2.1. Identification of signaling ability in IL-12 recognizing cells

Considering that IL-12 is known to initiate signaling by inducing phosphorylation of STAT4 (Signal transducer and activator or transcription 4), a signaling mediator, when IL-12 binds to the IL-12 receptor, the signaling ability of the fusion protein of one embodiment in IL-12 recognizing cells expressing the IL-12 receptor was evaluated.

Specifically, the HEK-Blue IL-12 cell line, which is an IL-12 recognizing cell, was diluted to 2.8 × 10⁵ cells/ml and dispensed at 180 µl/well. Recombinant human IL-12, T1.01m, T1.02m, T1.03m and T1.12m were suspended at 4 nM and diluted by serial dilution. HEK-Blue IL-12 cells were treated with the diluted recombinant human IL-12, T1.01m, T1.02m, T1.03m and T1.12m. After 24 hours of treatment, the cell supernatant was collected and mixed with QUANTI-Blue Solution (Invivogen) in a 96-well plate. The reporter expression level was measured at 620 nm using a spectrophotometer (Thermo Fisher).

As a result, as shown in FIG. 18, it was identified by 620 nm absorbance that in the case of IL-12, T1.01m, T1.02m and T1.03m treatment groups, IL-12 was recognized, and STAT4 expressed a reporter gene by inducing phosphorylation. In contrast, it was identified that the control, T1.12m, did not recognize IL-12 when IL-12 recognizing cells were treated with T1.12m. These results indicate that the fusion protein of one embodiment specifically binds to the IL-12 receptor to induce signaling.

### Example 2.2. Identification of signaling ability in human T cells

In order to identify whether when IL-12 binds to the IL-12 receptor of human T cells, IL-12 initiate signaling by inducing phosphorylation of STAT4, a signaling mediator, the human T cells activated with anti-CD3 (OKT3, Invitrogen) were analyzed using the AlphaLISA SureFire Ultra p-STAT4 (Tyr693) Assay Kit (PerkinElmer, Massachusetts, USA).

Specifically, human proteins IL-12, T1.01, T1.02, T1.03 and T1.12 were suspended at 100 ng/ml and diluted by serial dilution. The human T cells activated with anti-CD3 (OKT3, Invitrogen) were treated with the diluted recombinant human IL-12, T1.01, T1.02, T1.03 and T1.12. After 2 hours of treatment, the cells were lysed and treated with a reagent (PerkinElmer) that detects p-STAT4 (phosphorylated STAT4). pSTAT4 was detected at an excitation wavelength of 680 nm and an emission wavelength of 615 nm with a spectrophotometer.

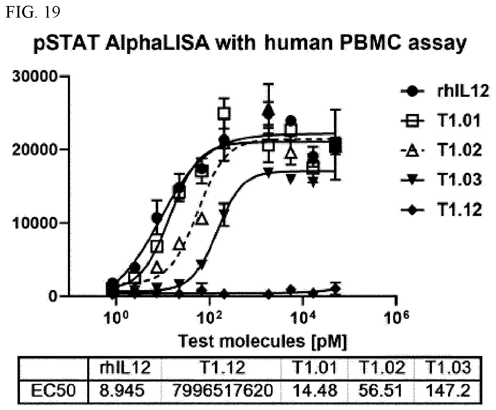

As a result, as shown in FIG. 19, it was identified that the half maximal effective concentration (EC50) of the group treated with recombinant human IL-12 was 8.945 pM; the EC50 of the group treated with T1.01 was 14.48 pM; the EC50 of the group treated with T1.02 was 56.51 pM; and the EC50 of the group treated with T1.03 was 147.2 pM. In contrast, phosphorylated STAT4 was not detected in the group treated with T1.12, which is control.

These results indicate that the IL-12 mutation attenuated T cell signaling ability.

### Example 2.3. Identification of change in signaling ability of fusion protein by low molecular weight heparin in human T cells

Next, enhancement of signaling ability of the IL-12 mutation (IL-12 mut1) fusion protein by low molecular weight heparin was identified using the human T cells activated with anti-CD3 (OKT3, Invitrogen) and the AlphaLISA SureFire Ultra p-STAT4 (Tyr693) Assay Kit.

In particular, by observing whether the induction of phosphorylation of STAT4, a signaling mediator, was attenuated when attenuated IL-12 mut2 was bound to the IL-12 receptor of human T cells, the signaling ability according to the presence or absence of low molecular weight heparin (LMWH) in human T cells was identified.

Specifically, recombinant human IL-12, T1.01, T1.02 and T1.03 were suspended at 100 ng/ml and diluted by serial dilution. The activated human T cells were treated with the diluted recombinant human IL-12, T1.01, T1.02 and T1.03 together with low molecular weight heparin. After 2 hours of treatment, the cells were lysed and treated with a reagent for detecting p-STAT4 (PerkinElmer). pSTAT4 was detected at an excitation wavelength of 680 nm and an emission wavelength of 615 nm with a spectrophotometer.

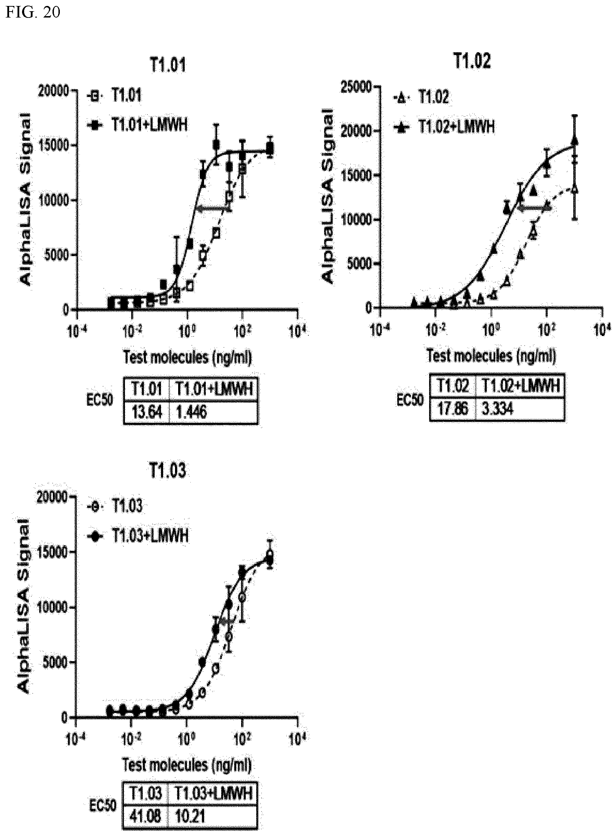

As a result, as shown in FIG. 20, the half maximal effective concentration (EC50) in the group treated with T1.01 and low molecular weight heparin was increased by 9.4 times compared to the group treated with T1.01, the half maximal effective concentration (EC50) in the group treated with T1.02 and low molecular weight heparin was increased by 5.5 times compared to the group treated with T1.02, and the half maximal effective concentration (EC50) in the group treated with T1.03 and low molecular weight heparin was increased by 4 times compared to the group treated with T1.03.

These results indicate that when the IL-12 mutation (IL-12 mut2) was added to the existing IL-12 mutation (IL-12 mut1), the degree of enhancement of signaling ability by the addition of low molecular weight heparin was reduced, so IL-12 was effectively attenuated.

### Example 3. Identification of cytokine secretion ability of fusion protein

### Example 3.1. Enzyme-linked immunosorbent assay for cytokine measurement

For cytokine measurement, enzyme-linked immunosorbent assay (ELISA, R&D systems) was performed on the supernatant stored at -20°C in the following steps:
(i) The capture antibody was diluted according to the Certificate of Analysis (CoA) and coated on a 96-well plate for 24 hours.
(ii) The wells were washed with a wash solution, and then blocking was performed for 1 hour with 1% BSA (Bovine serum albumin, Sigma) solution.
(iii) The wells were washed with a wash solution, and then the samples were treated for 2 hours.
(iv) The wells were washed with a wash solution, and then the detection antibody was diluted according to the Certificate of Analysis and aliquoted into the wells, and then the wells were treated therewith for 2 hours.
(v) The wells were washed with a wash solution, and then treated with streptavidin-HRP for 20 minutes.
(vi) The wells were washed with a wash solution, and then treated with a substrate solution for 20 minutes.
(vii) The wells were treated with a stop solution, and the absorbance was measured at 450 nm.

### Example 3.2. Identification of cytokine secretion ability in human T cells

Considering that IFN-y (interferon-gamma) secretion, which is a major immune response, occurs when human T cells are treated with IL-12, IFN-y secretion ability was evaluated when human T cells were treated with recombinant human IL-12, T1.12, T1.01, T1.02 and T1.03.

Specifically, the human T cells activated with anti-CD3 (OKT3, Invitrogen) were diluted to 5 × 10⁵ cells/ml and dispensed in a 96-well plate at 200 µl/well. Recombinant human IL-12, T1.12, T1.01, T1.02 and T1.03 were diluted to 10 nM and dispensed at 20 µl/well. After 48 hours of treatment, the cell supernatant was collected, and the samples were stored at -80°C.

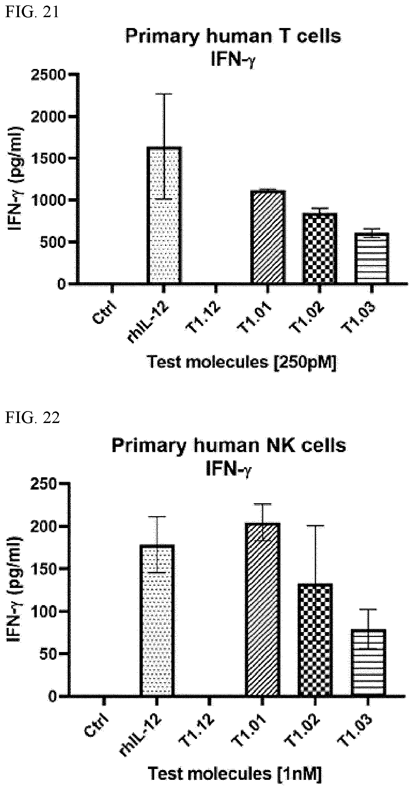

As a result, as shown in FIG. 21, it was found that the sample treated with recombinant human IL-12 had an average of 1,640 pg/ml of IFN-y; the sample treated with T1.01 had an average of 1,115 pg/ml of IFN-y; the sample treated with T1.02 had an average of 845 pg/ml of IFN-y; and the sample treated with T1.03 had an average of 607 pg/ml of IFN-y. In contrast, it was found that the sample treated with T1.12 as a control had no IFN-y.

These results indicate that T1.01, T1.02 and T1.03 act on human T cells to induce IFN-y secretion.

### Example 3.3. Identification of cytokine secretion ability in human NK cells

Considering that IFN-y (interferon-gamma) secretion, which is a major immune response, occurs when human NK cells are treated with IL-12, the present inventors evaluated IFN-y secretion ability when the human NK cells activated with recombinant human IL-2 (R&D systems) were treated with recombinant human IL-12, T1.12, T1.01, T1.02 and T1.03.

Specifically, the human NK cells activated with recombinant human IL-2 (R&D Systems) were diluted to 5 × 10⁵ cells/ml and dispensed in a 96-well plate at 200 µl/well. Recombinant human IL-12, T1.12, T1.01, T1.02 and T1.03 were diluted to 10 nM and dispensed at 20 µl/well. After 24 hours of treatment, the cell supernatant was collected, and the samples were stored at - 80°C.

As a result, as shown in FIG. 22, it was found that the sample treated with recombinant human IL-12 had an average of 180 pg/ml of IFN-y; the sample treated with T1.01 had an average of 205 pg/ml of IFN-y; the sample treated with T1.02 had an average of 133 pg/ml of IFN-y; and the sample treated with T1.03 had an average of 80 pg/ml of IFN-y. In contrast, it was found that the sample treated with T1.12 as a control had no IFN-y. These results indicate that T1.01, T1.02 and T1.03 act on human NK cells to induce IFN-y secretion.

### Example 4. Evaluation of anticancer efficacy of fusion protein in colorectal cancer animal model

### Example 4.1. Evaluation of anticancer efficacy in colorectal cancer animal model through co-injection of FAP-expressing fibroblasts

Considering that the fusion protein is in the form of a dual antibody having an anti-FAP sequence structure and an IL-12 sequence structure, and FAP is overexpressed in fibroblasts present in many tumor microenvironments, after overexpressing mouse FAP in NIH3T3 cells, a mouse fibroblast cell line, the tumor growth inhibitory ability was evaluated in a tumor animal model co-injected 1:1 with CT26, a mouse colorectal cancer cell line.

CT26, a mouse colorectal cancer cell line, and NIH-3 T3, a mouse fibroblast cell line, were used to prepare an FAP-expressing tumor animal model. Then, NIH-3T3 was prepared as an FAP-expressing cell line.

Specifically, the mouse FAP overexpressing NIH-3T3 cells and the CT26 cells in culture were resuspended in Hanks' Balanced salt solution (HBSS, Gibco) at a concentration of 1 × 10⁶ cells/50 µl, respectively, and the cells were mixed in a 1:1 ratio, and then 100 µl per each mouse was transplanted into the subcutaneous region of the right flank of 6-week-old BALB/c mice using a 1 cc syringe (25G) to obtain an FAP-expressing fibroblast co-injection animal model. The tumor size was measured using a digital vernier caliper by measuring the short axis and long axis of the tumor, and the tumor size was measured twice a week using the calculation formula of (short axis, mm)² × (long axis, mm) × 0.5.

Next, for drug efficacy evaluation, when the size of the tumor reached 100 mm³, the mice were randomly divided into groups. Each experimental group consisted of 10 animals, and as a control, each 100 µl of 50 µg of T1.24m was intraperitoneally administered three times at an interval of 3 days. In the test group, each 100 µl of 10 µg and 50 µg of T1.02m and 50 µg of T1.01m, respectively, were intraperitoneally administered three times at an interval of 3 days to evaluate the tumor growth inhibitory ability.

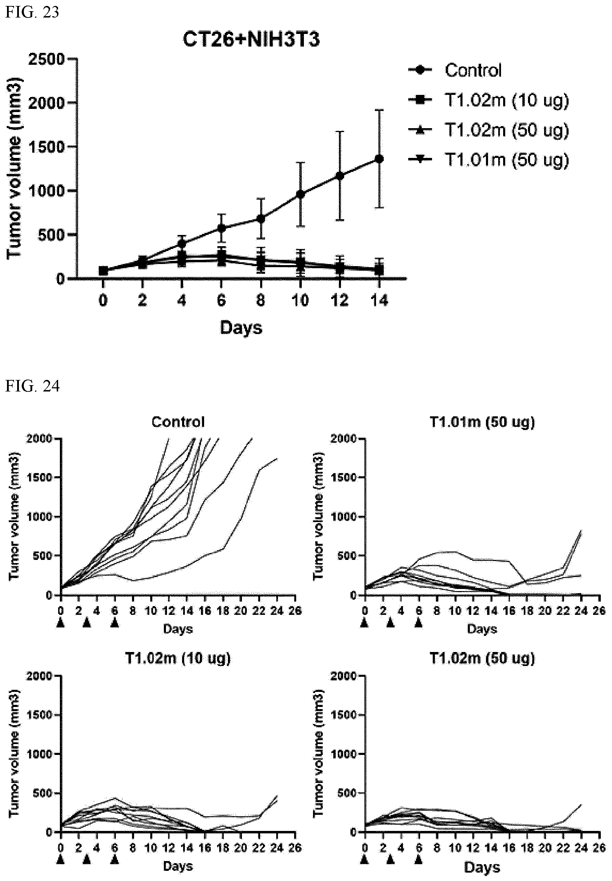

As a result, as shown in FIG. 23, when comparing the tumor size on the 14^{th} day, it was found that the groups administered with 10 µg and 50 µg of T1.02m had an excellent tumor growth inhibitory ability of 93.2% and 92.7%, respectively, compared to the group administered with 50 µg of T1.24m as a control. In addition, it was observed that the group administered with 50 µg of T1.01m had tumor growth inhibition of 91.9% compared to the group administered with 50 µg of T1.24m as a control.

In addition, as shown in FIG. 24, on the 24^{th} day after administration, subjects in complete response were observed in the group administered with T1.01m and T1.02m compared to the control.

These results indicate that the fusion protein of one embodiment has an excellent anticancer efficacy in a colorectal cancer animal model.

### Example 4.2. Evaluation of anticancer efficacy in FAP-expressing colorectal cancer animal model

In the case of colorectal cancer, considering the high expression of FAP in the tumor cells themselves, in order to evaluate the anticancer effect, the tumor growth inhibitory ability was evaluated in a tumor model in which mouse FAP overexpression was induced in CT26 cells, a mouse colorectal cancer cell line.

In order to prepare an mouse FAP-expressing tumor animal model, FAP overexpression was induced in CT26, a mouse colorectal cancer cell line. Specifically, the FAP-overexpressing CT26 cells in culture were resuspended in Hanks' Balanced salt solution (HBSS, Gibco), and then each 100 µl of 1 × 10⁶ tumor cells per each mouse was transplanted into the subcutaneous region of the left dorsal side of 6-week-old BALB/c mice using a 1 cc syringe (25G) to obtain a mouse FAP-expressing colorectal cancer animal model. The tumor size was measured using a digital vernier caliper by measuring the short axis and long axis of the tumor, and the tumor size was measured twice a week using the calculation formula of (short axis, mm)² × (long axis, mm) × 0.5.

Next, for drug efficacy evaluation, when the size of the tumor reached 100 mm³, the mice were randomly divided into groups. Each experimental group consisted of 8 animals, and as a control, each 100 µl of 50 µg of T1,24m was intraperitoneally administered four times at an interval of 3 days. In the test group, each 100 µl of 10 µg and 100 µg of T1.01m, and 2 µg, 10 µg and 100 µg of T1.02m, respectively, were intraperitoneally administered four times at an interval of 3 days to evaluate the tumor growth inhibitory ability.

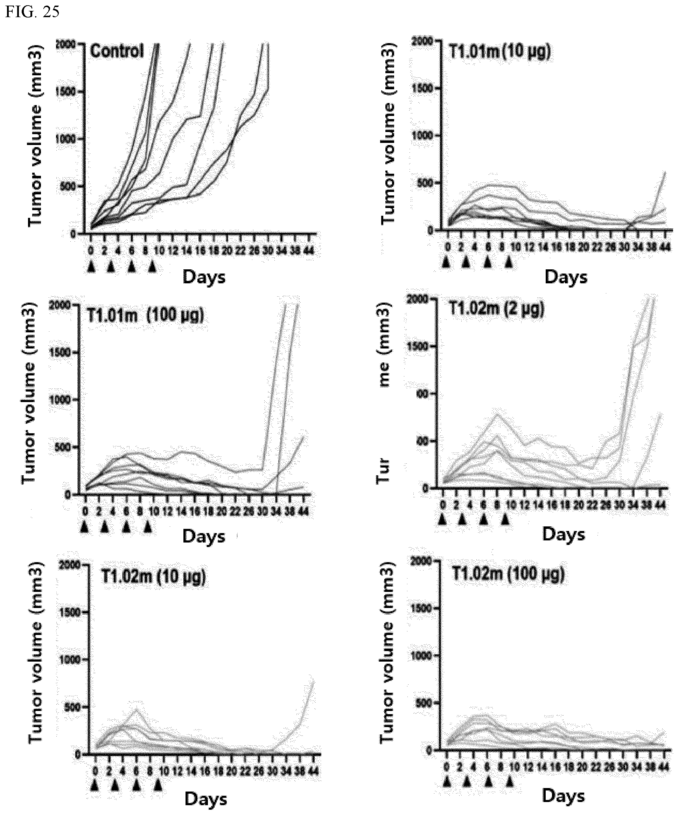

As a result, as shown in FIG. 25, at 18 days after administration, it was found that the group administered with 10 µg and 100 µg of T1.01m had a high tumor growth inhibitory ability of 84.9% and 85.6%, respectively, compared to the group administered with 50 µg of T1.24m as a control. In addition, it was observed that the group administered with T1.02m had a high tumor growth inhibitory ability of 80.1%, 90.5% and 89.8%, respectively, by dose compared to the group administered with T1.24m as a control.

In addition, it was identified that subjects in complete response (CR) existed in each administration group on the 30^{th} day after administration together with tumor growth inhibition, and no change in body weight was observed during the test according to the administration. These results indicate that the fusion protein of one embodiment has excellent anticancer efficacy in a colorectal cancer animal model.

### Example 4.3. Evaluation of growth inhibition of recurrent solid cancer in mice with complete response by fusion protein

In order to evaluate whether tumor recurrence may be inhibited by inducing long-term immune response after fusion protein treatment, tumor re-challenge was performed in subjects in which complete response was achieved by administration of T1.01m in a tumor model transplanted with CT26 cells overexpressing FAP. BALB/c mice without a treatment history were used as control mice.

4T1 cells were subcutaneously injected into the left flank and CT26 cells were subcutaneously injected into the right flank of control mice and mice in which complete response was achieved by administration of T1.01m, to obtain a tumor recurrence model.

Specifically, in a model transplanted with CT26 cells, a tumor recurrence model was induced in mice in which complete response was exhibited by administration of T1.01m. CT26 cells and 4T1 cells were suspended in Hanks' Balanced salt solution (HBSS, Gibco) at a concentration of 1 × 10⁶ cells/100 µl, respectively, and CT26 cells were transplanted into the subcutaneous region of the right flank of the mice using a 1 cc syringe (25G). Tumor formation and growth were evaluated by subcutaneous transplantation of 4T1 cells into the left flank of the same mice. Nine animals were used for the control group, and seven animals were used for the test group.

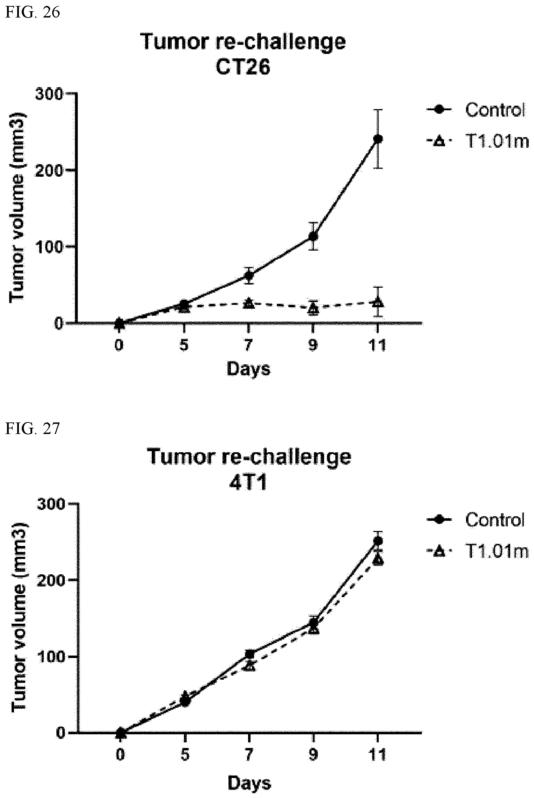

As a result, as shown in FIGS. 26 and 27, it was found that both the transplanted 4T1 tumor and CT26 tumor grew in the control mice without a treatment history, whereas in the mice in which complete response was achieved after administration of T1.01m, the 4T1 tumor grew, but the growth of the CT26 tumor was inhibited. These results indicate that the fusion protein of one embodiment may inhibit tumor recurrence.

### Example 4.4. Evaluation of decrease in activity according to IL-12 amino acid mutation of fusion protein

In order to reduce the toxicity *in vivo* due to excessive activation of the immune system by IL-12, the present inventors used an IL-12 sequence structure containing a mutation in the amino acid sequence binding to heparin to induce the decrease in activity by blocking of binding to the IL-12 receptor, and this was identified through a tumor animal model.

Among the anti-FAP antibodies, T1.02m and T1.03m in which amino acids involved in heparin binding were mutated and T1.01m was used as a control. Then, T1.02m has a mouse IL-12 sequence structure containing mutations (two) of amino acids involved in heparin binding together with the anti-FAP sequence structure. T1.03m is a fusion protein having an IL-12 sequence structure containing mutations (four) of amino acids involved in heparin binding together with the anti-FAP sequence structure.

In order to evaluate the change in IL-12 activity according to the presence and extent of amino acid mutation, FAP-overexpressing mouse colorectal cancer CT26 was prepared by resuspending in PBS at a concentration of 5 × 10⁵ cells/100 µl, and the tumor growth inhibitory ability was evaluated in a tumor animal model prepared by transplantation of the cells into 5-week-old female BALB/c mice.

Specifically, when the size of the tumor in the model reached 100 mm³, the mice were randomly divided into groups. 100 µl of phosphate buffered saline (PBS) was administered once via tail vein injection of the control, and each of T1.01m, T1.02m and T1.03m was intravenously administered once at a dose of 10 µg.

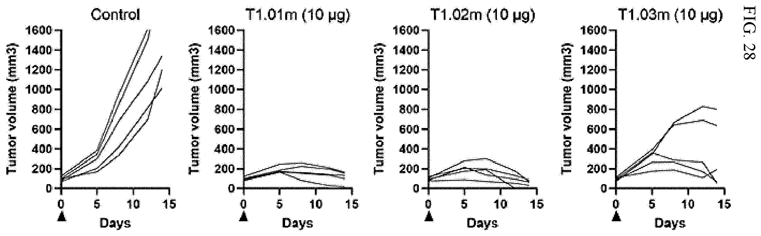

As a result, as shown in FIG. 28, it was found that all of the T1.01m, T1.02m and T1.03m administration groups had an excellent tumor growth inhibitory ability compared to the control at 15 days after administration.

In addition, it was found that the tumor growth inhibitory ability was reduced in the group administered with T1.03m having an IL-12 containing mutations (four) of amino acids involved in heparin binding together with the mouse anti-FAP sequence compared to the group administered with T1.01m. These results indicate that the IL-12 activity was reduced in the group administered with T1.03m having a mouse IL-12 sequence structure containing mutations (four) of amino acids involved in heparin binding compared to other experimental groups and the control.

### Example 4.5. Evaluation of anticancer efficacy upon single administration of IL-12 amino acid mutation fusion protein

In order to evaluate the anticancer effect of a fusion protein having reduced IL-12 activity due to a sequence structure containing mutations (four) of amino acids involved in heparin binding, the tumor growth inhibitory ability was evaluated in a tumor model transplanted with FAP-overexpressing mouse colorectal cancer cell line CT26 cells.

Specifically, the tumor cells were transplanted into the right dorsal flank side of 6-week-old BALB/c mice, and when the size of the tumor of the animal model reached 70 to 100 mm³, the mice were randomly divided into groups and the administration was performed. As the control, PBS was administered, and as the test group, 100 µl of T1.03m was intravenously administered once at a dose of 2 µg, 10 µg and 100 µg. The size of the tumor was measured in the same manner as in Example 4.1.

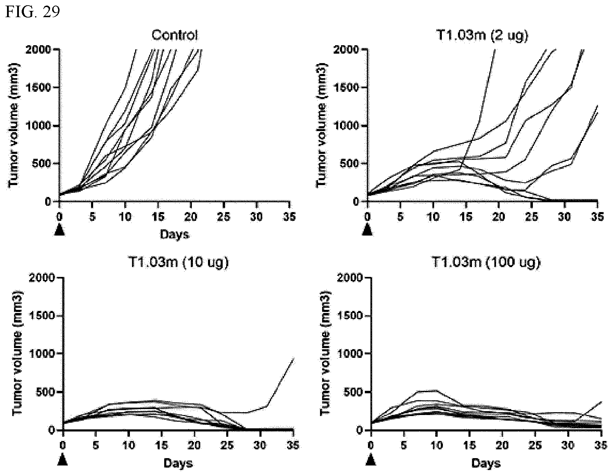

As a result, as shown in FIG. 29, it was observed that the group administered with 2 µg, 10 µg and 100 µg of T1.03m had a high tumor growth inhibitory ability compared to the control. No change in body weight was observed during the test period. These results indicate that T1.03m, which has reduced IL-12 activity due to a sequence structure containing mutations (four) of amino acids involved in heparin binding, has an excellent anticancer efficacy at a certain concentration or higher.

### Example 5. Evaluation of anticancer efficacy of fusion protein in melanoma animal model

In order to identify the anticancer effect through immune activation of the fusion protein, mouse FAP was overexpressed in the mouse melanoma cell line B16F10, which is known to have little immune cell infiltration, and then transplanted into C57BL/6J mice, and the tumor growth inhibitory ability was evaluated.

Specifically, the cells in culture were resuspended in Hanks' Balanced salt solution (HBSS, Gibco), and then 100 µl of 1 × 10⁶ tumor cells per each mouse were transplanted into the subcutaneous region of the left the dorsal flank side of 6-week-old C57BL/6J mice.

Next, for drug efficacy evaluation, when the size of the tumor reached 70 to 100 mm³, the mice were randomly divided into groups. As the control, PBS was administered, and as the test group, T1.03m was intravenously administered once at a dose of 10 µg and 100 µg.

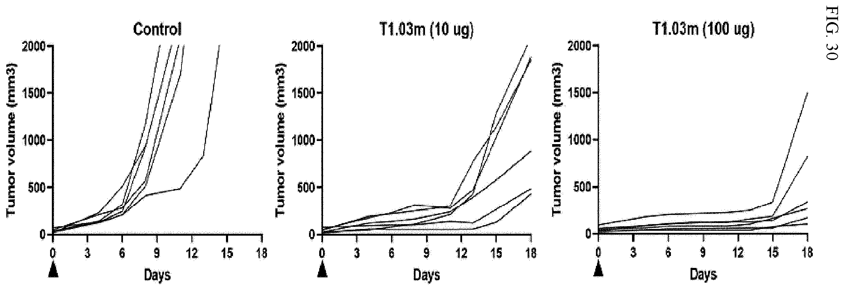

As a result, as shown in FIG. 30, it was observed that both groups administered with 10 µg and 100 µg of T1.01m had a high tumor growth inhibitory ability compared to the control. No change in body weight was observed during the test period. These results indicate that T1.03m, which has reduced IL-12 activity due to a sequence structure containing mutations (four) of amino acids involved in heparin binding, has an excellent anticancer efficacy even in a melanoma animal model.

### Example 6. Evaluation of anticancer efficacy of fusion protein in lung cancer animal model

### Example 6.1. Evaluation of anticancer efficacy of IL-12 amino acid mutation fusion protein in lung cancer animal model

Considering that FAP is overexpressed in fibroblasts present in the microenvironment around the tumor, FAP was overexpressed in NIH-3T3 cell, a mouse fibroblast cell line, and then co-injected with LLC1 cell, a mouse lung cancer cell line, at a 1:1 ratio to prepare a tumor animal model, and the tumor growth inhibitory ability was evaluated.

Specifically, the NIH-3T3 cell line overexpressing FAP and the LLC1 cells were resuspended in Hanks' Balanced salt solution (HBSS, Gibco) at a concentration of 1 × 10⁶ cells/50 µl, respectively, and the cells were mixed in a 1:1 ratio, and then 100 µl per each mouse was transplanted into the subcutaneous region of the right flank of 6-week-old C57BL/6J mice using a 1 cc syringe (25G).

Next, for drug efficacy evaluation, when the size of the tumor reached 70 to 100 mm³, the mice were randomly divided into groups. As the control, PBS was administered, and as the test group, T1.03m was intravenously administered once at a dose of 10 µg and 100 µg.

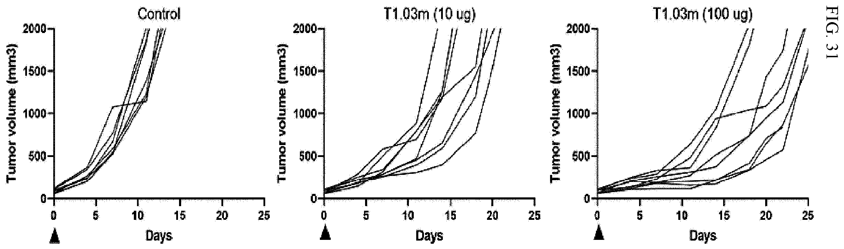

As a result, as shown in FIG. 31, it was observed that both groups administered with 10 µg or 100 µg of T1.03m had an excellent tumor growth inhibitory ability compared to the control. No change in body weight was observed during the test period. These results indicate that T1.03m, which has reduced IL-12 activity due to a sequence structure containing mutations (four) of amino acids involved in heparin binding, recognizes a target factor of the tumor microenvironment, thereby having an excellent anticancer efficacy even in a lung cancer animal model.

### Example 6.2. Evaluation of cancer targeting efficacy in lung cancer animal model through co-injection of fusion protein

In order to compare the anticancer efficacy according to the cancer targeting of the dual antibody-type fusion protein in which the anti-FAP sequence structure and the IL-12 sequence structure form a Knob-in-Hole structure, the tumor growth inhibitory ability was identified in an animal model co-injected with NIH-3T3 cell overexpressing FAP, a mouse fibroblast cell line, and LLC1, a mouse lung cancer cell line, in a 1:1 ratio.

Specifically, for drug efficacy evaluation, when the size of the tumor reached 70 to 100 mm³, the mice were randomly divided into groups. As the control, PBS was intraperitoneally administered twice a week. The group administered with T1.16m or T1.17m alone, the group administered with T1.16m and T1.17m in combination (T1.16m + T1.17m), and the group administered with T1.03m were used as test groups to evaluate the tumor growth inhibitory ability. T1.16m and T1.17m, which are dimers of anti-FAP and IL-12, respectively, were intraperitoneally administered twice a week at a dose of 0.01 µg, and T1.03m, which is a monomer fusion protein, was intraperitoneally administered twice a week at a dose of 0.02 µg to observe the tumor growth inhibitory ability.

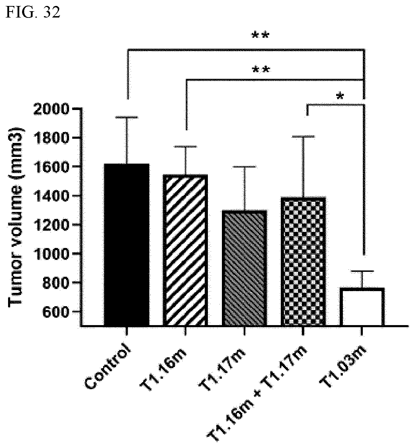

As a result, as shown in FIG. 32, it was observed that the group administered with T1.03m had an excellent tumor growth inhibitory ability at the time point 10 days after administration compared to the control. The group administered with T1.16m or T1.17m alone, and each combination administration group were compared, and it was found that the group administered with T1.03m alone had an excellent tumor growth inhibitory ability. These results indicate that the synergistic effect of the fusion protein of one embodiment is significant compared to the case where only FAP is targeted or when compared with IL-12 without a cancer target.

### Example 7. Confirmation of anticancer effect when fusion protein and anticancer agent are co-administered

### Example 7.1. Confirmation of anticancer effect by co-administration of immune checkpoint inhibitor in lung cancer mouse model

To compare the anticancer efficacy when a fusion protein, in which anti-FAP sequence structure and IL-12 sequence structure form a knob-in-hole structure, and an immune checkpoint inhibitor, the inhibitory ability for tumor growth was confirmed in a mouse animal model. Specifically, a mouse animal model was used in which NIH-3T3 cells, a mouse fibroblast cell line overexpressing FAP, and LLC1, a mouse lung cancer cell line, were co-injected at a 1:1 ratio.

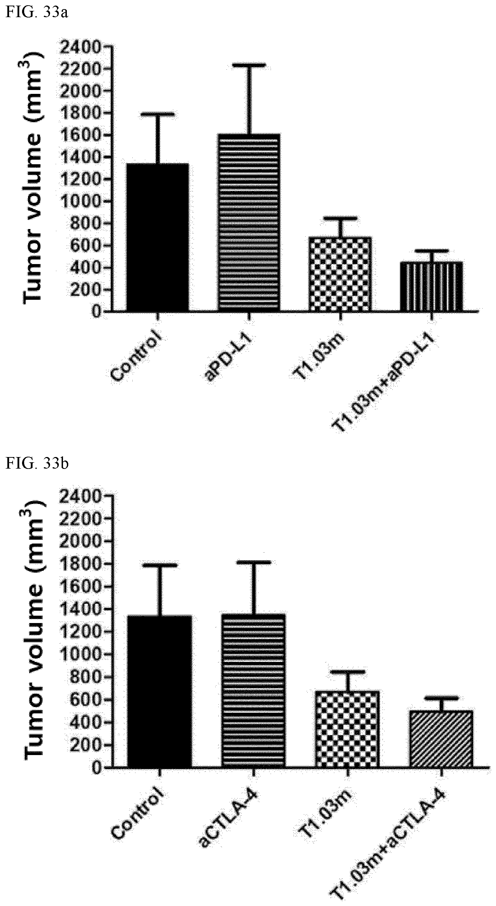

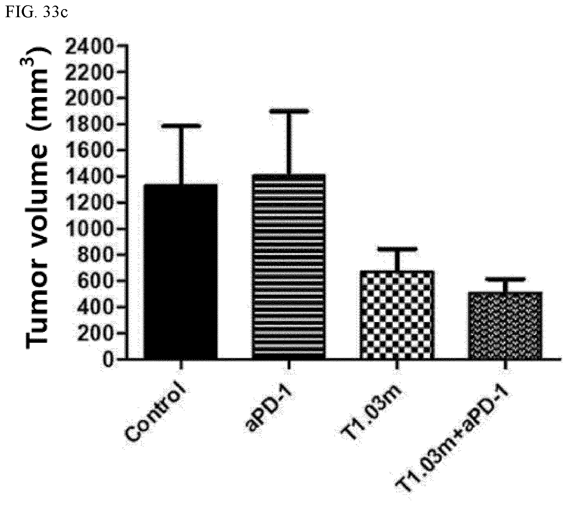

For drug efficacy evaluation, when the tumor size reached an average of 110 to 120 mm³, they were randomly divided into groups and PBS was intraperitoneally administered once every 3 days, total of three times, as a control group. T1.03m single administration group, immune checkpoint inhibitors aPD-L1(clone 10F.9G2, BioXCell), aCTLA-4(clone 9D9, BioXCell), or aPD-1(Clone RMP1-14, BioXCell) single administration group, T1.03m and aPD-L1 co-administration (FIG. 33a), T1.03m and aCTLA-4 co-administration (FIG. 33b), and T1.03m and aPD-1 co-administration group (FIG. 33c) were used as experimental groups to evaluate tumor growth inhibition ability. T1.03m was injected intravenously once at a dose of 10 µg, and the immune checkpoint inhibitors were administered at a dose of 200 µg once every 3 days, and observed the tumor growth inhibition ability.

As a result, as shown in FIG. 33a to FIG. 33c, at 9 days after administration, a superior tumor growth inhibition ability was observed in the immune checkpoint inhibitor co-administration group, compared to the control group and the T1.03m administration group. These results indicate that T1.03m, an anti-FAP and IL-12 monomer fusion protein, has a significant synergistic effect in a lung cancer mouse model when co-administered with an immune checkpoint inhibitor.

### Example 7.2. Confirmation of anticancer effect by co-administration of immune checkpoint inhibitor in colon cancer mouse model

In order to compare the anti-cancer efficacy of a bispecific antibody form fusion protein in which anti-FAP sequence structure and IL-12 sequence structure form a knob-in-hole structure and co-administered with an immune checkpoint inhibitor, the tumor growth inhibition ability was confirmed in a mouse animal model. Specifically, a mouse animal model was used in which NIH-3T3 cells, a mouse fibroblast cell line overexpressing FAP, and MC38, a mouse colon cancer cell line, were co-injected at a 1:1 ratio.

Specifically, for drug efficacy evaluation, when the tumor size reached an average of 90 to 160 mm³, they were randomly divided into groups and PBS was intraperitoneally administered once every 3 days, as a control group. T1.03m single administration group, an immune checkpoint inhibitor of aPD-L1(clone 10F.9G2, BioXCell) single administration group, and T1.03m and aPD-L1 co-administration group were used as experimental groups to evaluate tumor growth inhibition ability. T1.03m was injected intravenously once at a dose of 2 µg, and the immune checkpoint inhibitor was administered at a dose of 200 µg once every 3 days, and observed the tumor growth inhibition ability.

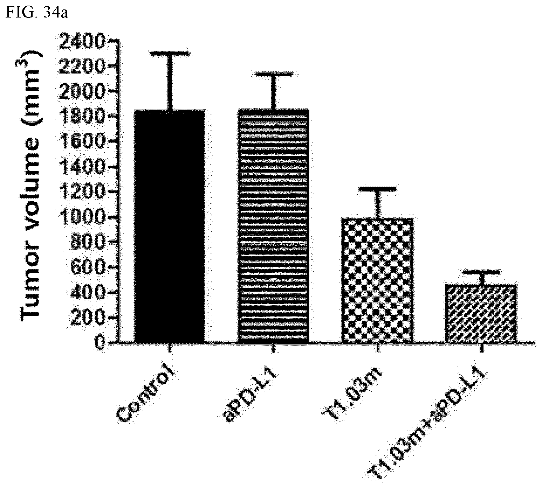

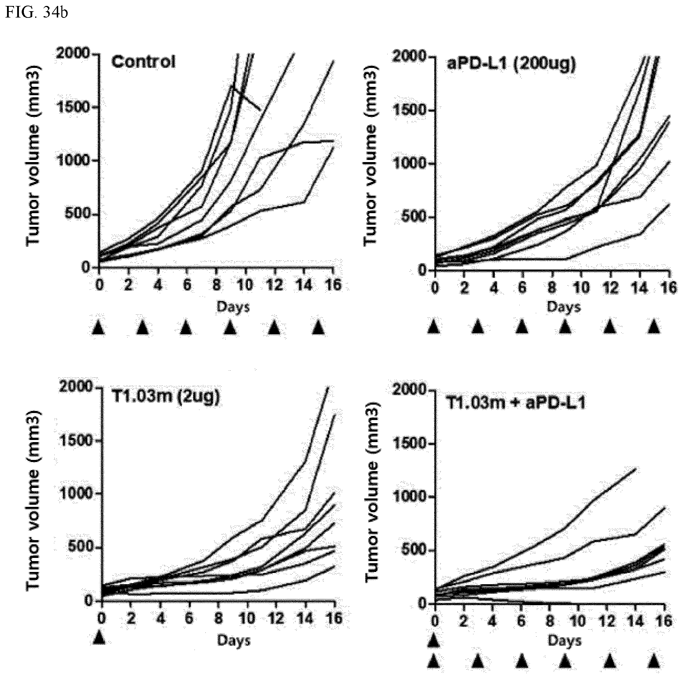

As a result, as shown in FIG. 34a and FIG. 34b, at 16 days after administration, a superior tumor growth inhibition ability was observed in the immune checkpoint inhibitor co-administration group compared to the control group and the T1.03m administration group. These results indicate that T1.03m, an anti-FAP and IL-12 monomer fusion protein, has a significant synergistic effect in a colon cancer mouse model when co-administered with an immune checkpoint inhibitor.

In addition, in order to further evaluate the tumor growth inhibition ability with T1.03m single administration group, an immune checkpoint inhibitor of aPD-L1(clone 10F.9G2, BioXCell) single administration group, and T1.03m and aPD-L1 co-administration group as experimental groups, T1.03m was injected intravenously once at a dose of 10 µg, and the immune checkpoint inhibitor was administered at a dose of 200 µg once every 3 days, and observed the tumor growth inhibition ability.

As a result, as shown in FIG. 35a and FIG. 35b, at 11 days after administration, a superior tumor growth inhibition ability was observed in the immune checkpoint inhibitor of aPD-L1 co-administration group, compared to the control group and the T1.03m administration group.

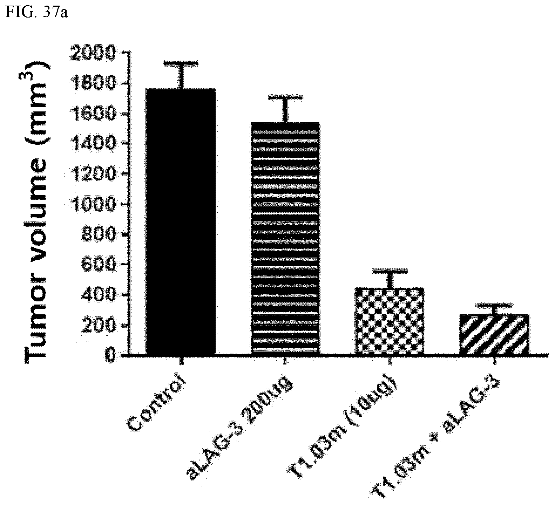

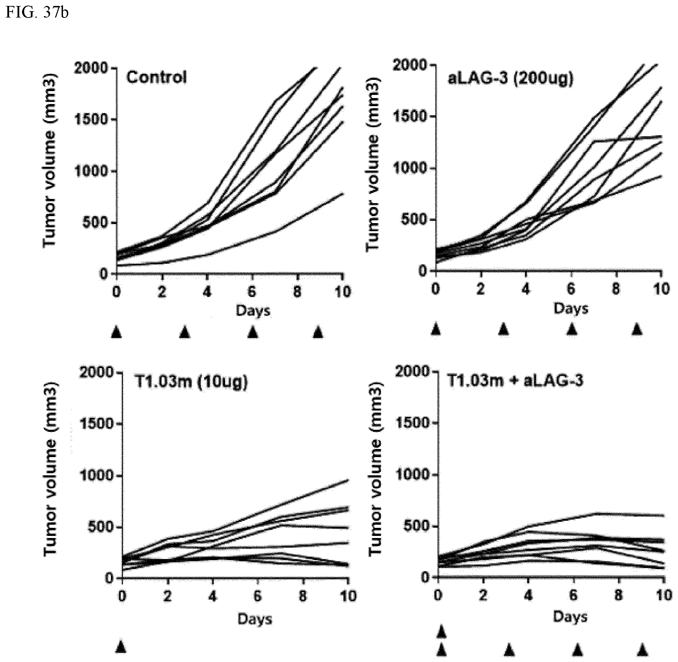

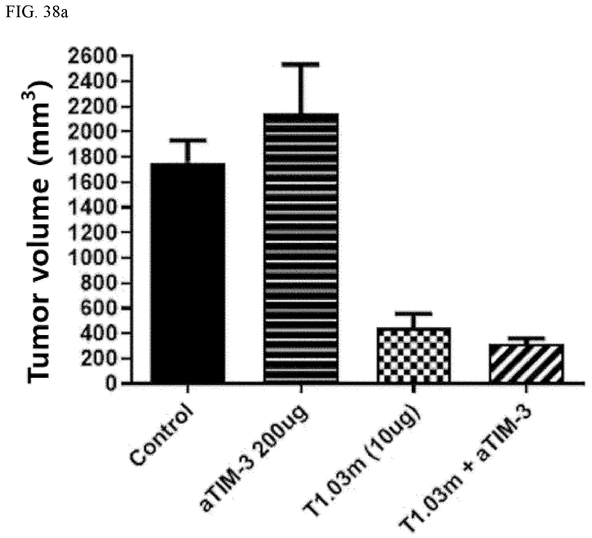

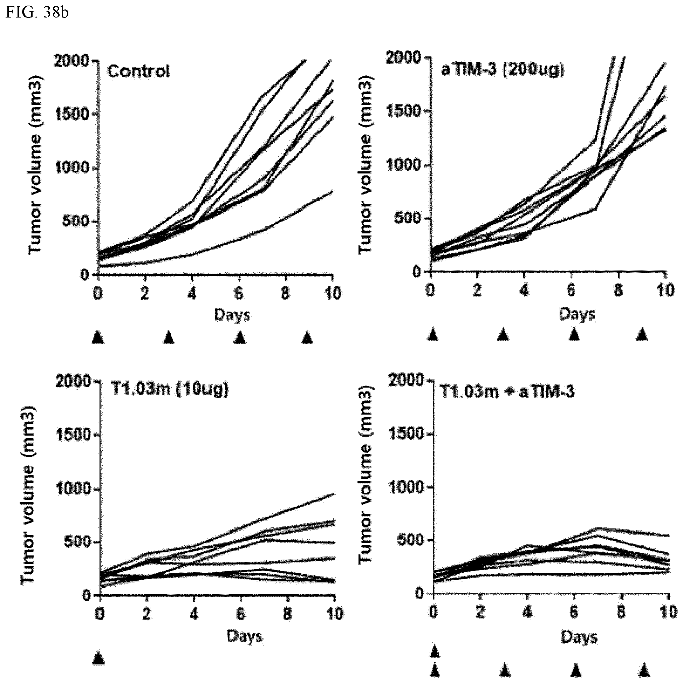

In addition, the tumor growth inhibition ability was evaluated using T1.03m single administration group, an immune checkpoint inhibitor of aTIGIT(clone 1G9, BioXCell), aLAG-3(clone C9B7W, BioXCell), or aTIM-3(Clone RMT3-23, BioXCell) single administration group, T1.03m and aTIGIT co-administration (FIG. 36), T1.03m and aLAG-3 co-administration (FIG. 37), and T1.03m and aTIM-3 co-administration group (FIG. 38) as the experimental groups. T1.03m was injected intravenously once at a dose of 10 µg, and the immune checkpoint inhibitor was administered at a dose of 200 µg once every 3 days, and observed the tumor growth inhibition ability.

As a result, as shown in FIG. 36a and FIG. 36b, at 10 days after administration, a superior tumor growth inhibition ability was observed in the T1.03m and aTIGIT co-administration group, compared to the control group and the T1.03m single administration group.

In addition, as shown in FIG. 37a and FIG. 37b, at 10 days after administration, a superior tumor growth inhibition ability was observed in the T1.03m and aLAG-3 co-administration group, compared to the control group and the T1.03m single administration group.

Further, as shown in FIG. 38a and FIG. 38b, at 10 days after administration, a superior tumor growth inhibition ability was observed in the T1.03m and aTIM-3 co-administration group, compared to the control group and the T1.03m single administration group.

These results indicate that T1.03m, an anti-FAP and IL-12 monomer fusion protein, has a significant synergistic effect in a colon cancer mouse model when co-administered with an immune checkpoint inhibitor.

### Example 7.3. Confirmation of anticancer effect by co-administration of chemical anticancer agent in colon cancer mouse model

In order to compare the anticancer efficacy when bispecific antibody form fusion protein, in which anti-FAP sequence structure and IL-12 sequence structure form a Knob-in-Hole structure, and a chemical anticancer agent are co-administered, the tumor growth inhibition ability was confirmed in a mouse animal model. Specifically, a mouse animal model was used in which NIH-3T3 cells, a mouse fibroblast cell line that overexpressing FAP, and MC38, a mouse colon cancer cell line, were co-injected at a 1:1 ratio.

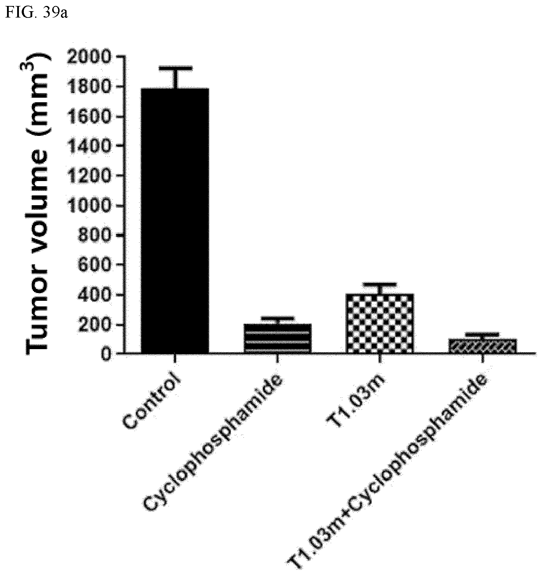

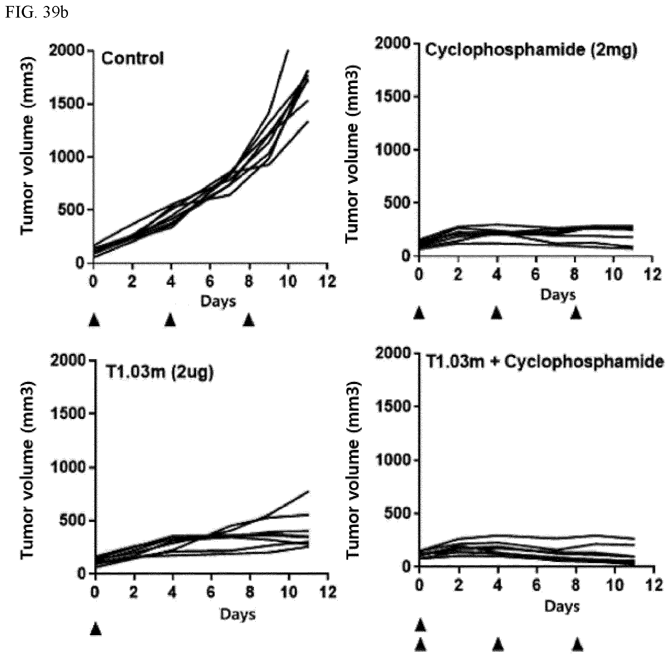

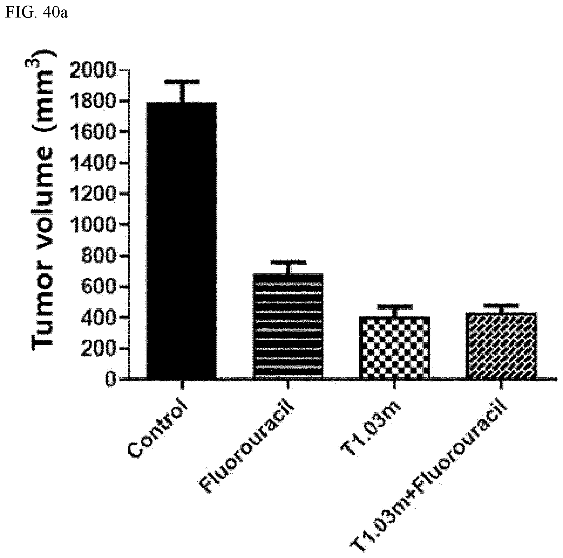

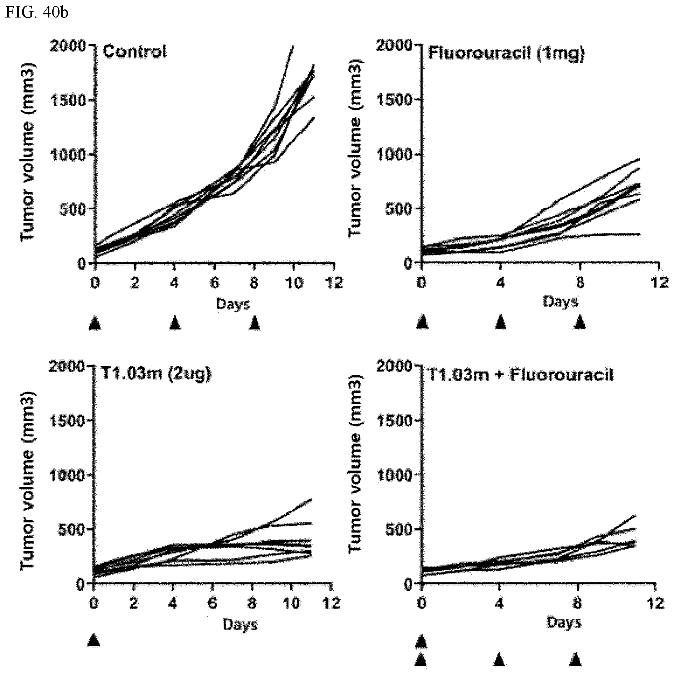

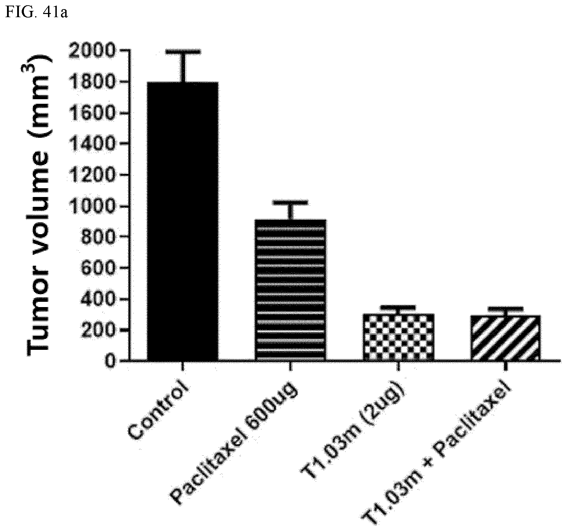

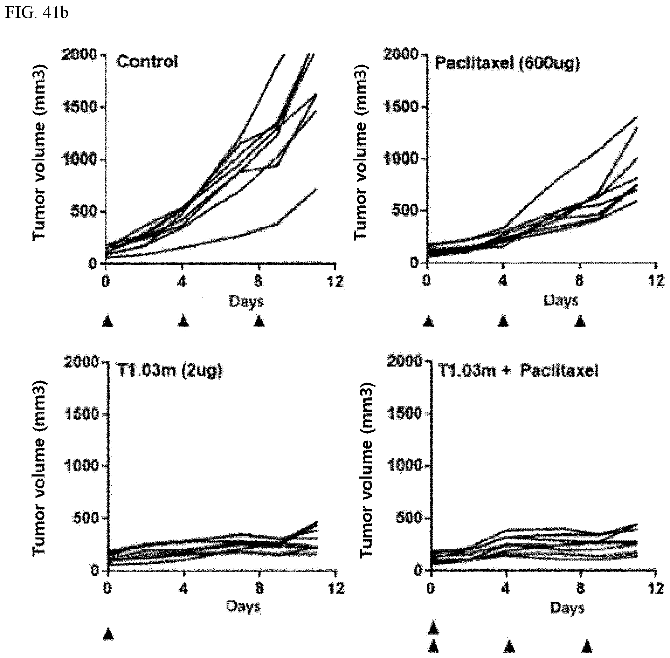

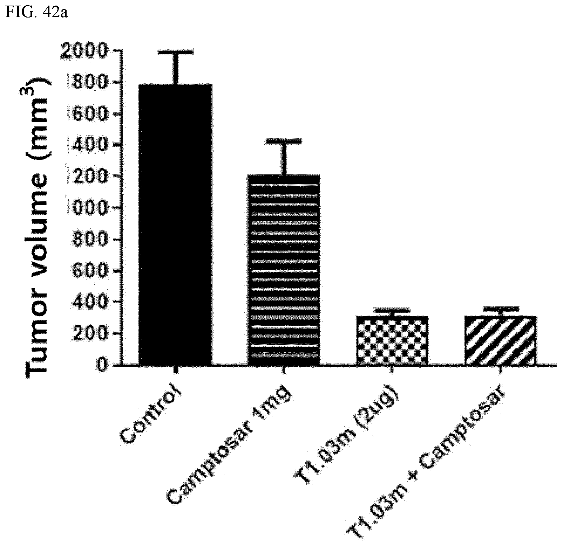

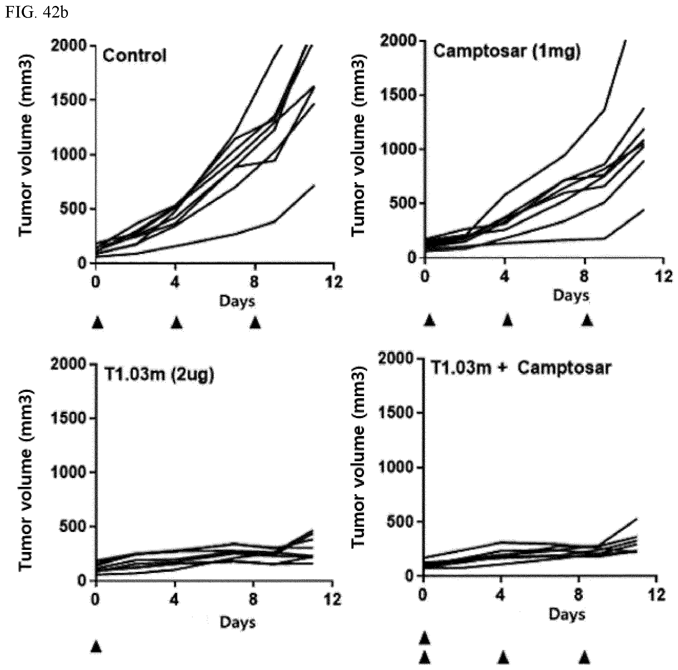

Specifically, for drug efficacy evaluation, when the tumor size reached an average of 110 to 120 mm³, they were randomly divided into groups and PBS was intraperitoneally administered once every 4 days, as a control group. T1.03m single administration group; a chemical anticancer agent of Cyclophosphamide(Endoxan Injection, Bukwang Pharm. Co., Ltd.), Fluorouracil(5-FU Injection, JW Pharmaceutical), Paclitaxel(Neotax Injection, JW Pharmaceutical), or Camptosar(Irinotecan Injection, Pfizer) single administration; T1.03m and Cyclophosphamide co-administration group (FIG. 39), T1.03m and Fluorouracil co-administration group (FIG. 40), T1.03m and Pacliatxel co-administration group (FIG. 41), and T1.03m and Camptosar co-administration group (FIG. 42) were used as experimental groups, and evaluated tumor growth inhibition ability.

As a result, as shown in FIG. 39a and FIG. 39b, at 11 days after administration, a superior tumor growth inhibition ability was observed in the T1.03m and Cyclophosphamide co-administration group, compared to the control group and the T1.03m single administration group.

In addition, as shown in FIG. 40a and FIG. 40b, at 11 days after administration, a superior tumor growth inhibition ability was observed in the T1.03m and Fluorouracil co-administration group, compared to the control group and T1.03m single administration group.

Furthermore, as shown in FIG. 41a and FIG. 41b, at 11 days after administration, a superior tumor growth inhibition ability was observed in the T1.03m and Pacliatxel co-administration group, compared to the control group and the T1.03m single administration group.

In addition, as shown in FIG. 42a and FIG. 42b, at 11 days after administration, a superior tumor growth inhibition ability was observed in the T1.03m and Camptosar co-administration group, compared to the control and the T1.03m administration group.

These results mean that T1.03m, an anti-FAP and IL-12 monomer fusion protein, has a significant synergistic effect in a colon cancer mouse model of when co-administered with a chemical anticancer agent.

## Claims

1. A pharmaceutical composition for preventing or treating cancer, comprising
a fusion protein comprising a first monomer comprising IL-12 or a variant thereof; and a second monomer comprising an antigen binding site that specifically binds to FAP (fibroblast activation protein alpha); and
an anticancer agent.

2. The pharmaceutical composition for preventing or treating cancer according to claim 1, wherein the IL-12 or the variant thereof comprises IL-12A (p35) or a variant thereof; and IL-12B (p40) or a variant thereof.

3. The pharmaceutical composition for preventing or treating cancer according to claim 2, wherein the variant of the IL-12B (p40) comprises an amino acid sequence obtained by one or more substitutions selected from the group consisting of K258A, K260A, K263A and K264A in the amino acid sequence of SEQ ID NO: 74.

4. The pharmaceutical composition for preventing or treating cancer according to claim 1, wherein the first monomer and the second monomer form a knob-into-hole structure.

5. The pharmaceutical composition for preventing or treating cancer according to claim 1, wherein the anticancer agent is any one selected from the group consisting of chemical anticancer agent, an anticancer virus and an immune checkpoint inhibitor.

6. The pharmaceutical composition for preventing or treating cancer according to claim 5, wherein the chemical anticancer agent is any one selected from the group consisting of alkylating agent, microtubule inhibitor, anti metabolite and topoisomerase inhibitor.

7. The pharmaceutical composition for preventing or treating cancer according to claim 5, wherein the chemical anticancer agent is any one selected from the group consisting of cyclophosphamide, cisplatin, fluorouracil, gemcitabine, camptosar, paclitaxel and adriamycin.

8. The pharmaceutical composition for preventing or treating cancer according to claim 5, wherein the anticancer virus is talimogene laherparepvec(T-VEC).

9. The pharmaceutical composition for preventing or treating cancer according to claim 5, wherein the immune checkpoint inhibitor is any one selected from the group consisting of anti-CTLA-4 antibody, anti-PD-1 antibody, anti-PD-L1 antibody, anti-TIM3 antibody, anti-TIGIT antibody and anti-LAG3 antibody.

10. The pharmaceutical composition for preventing or treating cancer according to claim 5, wherein the immune checkpoint inhibitor is any one selected from the group consisting of ipilimumab, pembrolizumab, nivolumab, cemiplimab, atezolizumab, avelumab and durvalumab.

11. The pharmaceutical composition for preventing or treating cancer according to claim 1, wherein the cancer is any one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, melanoma, bladder cancer, esophageal cancer, head and neck cancer, skin cancer, colorectal cancer, and lymphoma.

12. A kit for preventing or treating cancer, comprising
a fusion protein comprising a first monomer comprising IL-12 or a variant thereof; and a second monomer comprising an antigen binding site that specifically binds to FAP; and
an anticancer agent as active ingredients.

13. A method for preventing or treating cancer, comprising
a step of administering a fusion protein comprising a first monomer comprising IL-12 or a variant thereof; and a second monomer comprising an antigen binding site that specifically binds to FAP or a dimer thereof; and an anticancer agent to a subject.
